(19) Europäisches Patentamt
European Patent Office
Office européen des brevets

(11) EP 2 143 797 A1

# (12) EUROPÄISCHE PATENTANMELDUNG

(43) Veröffentlichungstag:
**13.01.2010 Patentblatt 2010/02**

(51) Int Cl.:
***C12N 15/82*** *(2006.01)* ***A01H 5/00*** *(2006.01)*

(21) Anmeldenummer: **08075631.5**

(22) Anmeldetag: **10.07.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **Bayer CropScience AG**
**40789 Monheim (DE)**

(72) Erfinder:
- **Frohberg, Claus**
  **14532 Kleinmachnow (DE)**
- **Schmidt, Ralf-Christian**
  **14532 Stahnsdorf (DE)**

(74) Vertreter: **Quanz, Martin et al**
**Bayer BioScience GmbH**
**Hermannswerder 20a**
**14473 Potsdam (DE)**

## (54) Weizenstärke sowie Weizenmehle und Lebensmittel enthaltend diese Weizenstärke/ Weizenmehle

(57) Die vorliegende Erfindung betrifft Weizenmehle, deren Stärkekomponente einen Amylosegehalt zwischen 15.0 Gew.-% bis 30.0 Gew.-% und die einen Gehalt an resistenter Stärke von mehr als 5.0 Gew.-% aufweisen sowie Lebensmittel enthaltend diese Weizenmehle. Ferner betrifft die vorliegende Erfindung Verfahren zur Herstellung besagter Weizenmehle und deren Verwendung als resistente Stärke, als Präbiotikum oder zur Herstellung von Lebensmitteln mit verringertem glykämischen Index. Die vorliegende Erfindung betrifft auch Nucleinsäuremoleküle, die eine lösliche Stärkesynthase II kodieren, sowie Vektoren enthaltend solche Nucleinsäuremoleküle. Ferner betrifft die vorliegende Erfindung auch Wirtszellen und Pflanzen, die solche Nucleinsäuremoleküle oder Vektoren umfassen.

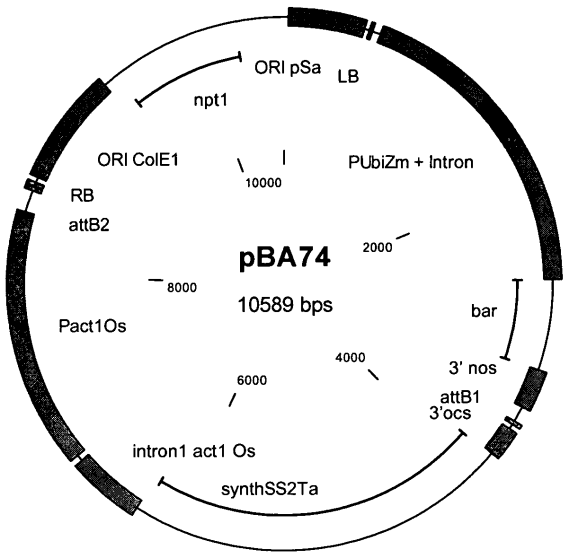


Figur 2

EP 2 143 797 A1

SEQUENCE LISTING

<110> Bayer CropScience AG

<120> Weizenstärke sowie Weizenmehle und Lebensmittel enthaltend diese Weizenstärke/Weizenmehle

<130> BCS 08-5010 EP

<160> 18

<170> PatentIn version 3.3

<210> 1
<211> 2433
<212> DNA
<213> Oryza sativa

<400> 1
atgtcgtcgg ccgtcgtcgc gtcatccacc acgttcctcg tcgcgctcgc ctcttcggcg 60

tcacggggag ggcccaggag ggggagggtg gtgggcgtgg ccgcgccgcc ggccctgctt 120

tacgacggcc gcgccggaag gctagcccta cgggcgccgc ctccgccccg gcctcggcct 180

cggcgtcggg atgcgggcgt ggtgcgccgc gcggatgatg gggagaacga ggcggcggtg 240

gagcgggcgg gtgaggacga cgacgaggag gaggagttct cttcgggcgc gtggcagccg 300

ccccgctcgc gtcgcggcgg cgtcggcaag gtcttgaagc ggaggggcac cgtcccgccc 360

gtcggccggt acggctccgg cggtgatgcg gcgagagtgc gcggggcggc cgcgccggcg 420

ccggcgccga cacaggacgc cgcctcaagt aagaacggag cgcttctcag cggccgcgac 480

gacgacacac ctgcctcacg gaacggatcg gtcgttaccg gcgccgacaa gcctgccgcc 540

gccacgccgc cggtgaccat aacgaagctc ccagcgccgg actcccccgt gatccttcca 600

tccgtagaca agccgcagcc ggagttcgtc atcccagacg cgacggcgcc ggcgccgcca 660

ccgcccggtt caaatcccag gtcgtccgct cctctcccca agcctgacaa ttcggaattt 720

gcagaggata agagcgcaaa agttgttgag agtgctccga agccaaaggc gactagatct 780

tcccctattc ctgcggtaga agaggagacg tgggatttca agaaatattt tgatctgaac 840

gaaccggacg ccgcggagga tggcgatgac gatgatgact gggctgattc agatgcgtca 900

gattctgaga tcgaccagga tgacgattcg ggccctttgg ctggggagaa tgtcatgaac 960

gtgatcgtgg tggctgctga atgttctccc tggtgcaaaa caggtgggct tggagatgtt 1020

gcaggtgctt tacccaaagc tttggcgagg agaggacatc gtgttatggt tgtggtacca 1080

aggtacggtg attacgcgga agcccaggat gtaggaatca ggaaatacta caaggctgct 1140

ggacaggatc tggaagtgaa atatttccat gcatttatcg atggagttga ttttgtgttc 1200

attgacgctc ctctcttccg tcaccgtcag gatgacatct atgggggaa cagacaggaa 1260

atcatgaagc gcatgattct gttttgtaag gctgctgttg aggttccttg gcacgttcca 1320

tgcggtggtg tgccctatgg ggatggcaac ttggtgttcc ttgcaaacga ttggcacact 1380

gcactcctgc ctgtttatct gaaggcatat tacagagaca atggcatgat gcagtacact 1440

cgctctgtcc ttgtgataca taatatcgct taccagggcc gtggcccagt agatgaattc 1500

```
ccctacatgg aattgccgga gcactacctg gatcacttca agctgtacga ccccgtcggc    1560
ggcgagcacg ccaacatctt cggcgcgggc ctgaagatgg cggaccgggt ggtgaccgtg    1620
agccccggct acctctggga gctgaagacg acggagggcg gctggggcct ccacgacatc    1680
atacgggaga acgactggaa gatgaacggc atcgtgaacg gcatcgacta ccgggagtgg    1740
aacccggagg tggacgtgca cctgcagtcc gacggctacg ccaactacac cgtggcctcg    1800
ctggactcca gcaagccgcg gtgcaaggcg gcgctgcagc gcgagctggg gctggaggtg    1860
cgcgacgacg tgccgctgat cgggttcatc gggcggctcg acgggcagaa aggtgtggac    1920
atcatcggcg acgcgatgcc gtggatcgcc gggcaggacg tgcagctggt gctgctgggc    1980
tccggccgcc gcgacctgga ggtgatgctg cagcggttcg aggcgcagca caacagcaag    2040
gtgcgcgggt gggtggggtt ctcggtgaag atggcgcacc ggatcacggc gggcgccgac    2100
gtgctggtca tgccgtcgcg gttcgagccg tgcggcctca accagctcta cgccatggcg    2160
tacggcaccg tccccgtcgt gcacgccgtc ggcgggctga gggacaccat gtcggcgttc    2220
gacccgttcg aggacaccgg cctcgggtgg acgttcgacc gcgccgagcc gcacaagctc    2280
atcgaggcgc tcggccactg cctcgagacg taccgcaagt acaaggagag ctggaggggg    2340
ctccaggtgc gcggcatgtc gcaggacctc agctgggacc acgccgccga gctctacgag    2400
gaggtccttg tcaaggccaa gtaccaatgg tga                                  2433
```

```
<210>  2
<211>  810
<212>  PRT
<213>  Oryza sativa

<400>  2

Met Ser Ser Ala Val Val Ala Ser Ser Thr Thr Phe Leu Val Ala Leu
1               5                   10                  15

Ala Ser Ser Ala Ser Arg Gly Gly Pro Arg Arg Gly Arg Val Val Gly
            20                  25                  30

Val Ala Ala Pro Pro Ala Leu Leu Tyr Asp Gly Arg Ala Gly Arg Leu
        35                  40                  45

Ala Leu Arg Ala Pro Pro Pro Pro Arg Pro Arg Pro Arg Arg Arg Asp
    50                  55                  60

Ala Gly Val Val Arg Arg Ala Asp Asp Gly Glu Asn Glu Ala Ala Val
65                  70                  75                  80

Glu Arg Ala Gly Glu Asp Asp Asp Glu Glu Glu Glu Phe Ser Ser Gly
                85                  90                  95

Ala Trp Gln Pro Pro Arg Ser Arg Arg Gly Gly Val Gly Lys Val Leu
            100                 105                 110
```

Lys Arg Arg Gly Thr Val Pro Pro Val Gly Arg Tyr Gly Ser Gly Gly
115 120 125

Asp Ala Ala Arg Val Arg Gly Ala Ala Ala Pro Ala Pro Ala Pro Thr
130 135 140

Gln Asp Ala Ala Ser Ser Lys Asn Gly Ala Leu Leu Ser Gly Arg Asp
145 150 155 160

Asp Asp Thr Pro Ala Ser Arg Asn Gly Ser Val Val Thr Gly Ala Asp
165 170 175

Lys Pro Ala Ala Ala Thr Pro Pro Val Thr Ile Thr Lys Leu Pro Ala
180 185 190

Pro Asp Ser Pro Val Ile Leu Pro Ser Val Asp Lys Pro Gln Pro Glu
195 200 205

Phe Val Ile Pro Asp Ala Thr Ala Pro Ala Pro Pro Pro Pro Gly Ser
210 215 220

Asn Pro Arg Ser Ser Ala Pro Leu Pro Lys Pro Asp Asn Ser Glu Phe
225 230 235 240

Ala Glu Asp Lys Ser Ala Lys Val Val Glu Ser Ala Pro Lys Pro Lys
245 250 255

Ala Thr Arg Ser Ser Pro Ile Pro Ala Val Glu Glu Glu Thr Trp Asp
260 265 270

Phe Lys Lys Tyr Phe Asp Leu Asn Glu Pro Asp Ala Ala Glu Asp Gly
275 280 285

Asp Asp Asp Asp Asp Trp Ala Asp Ser Asp Ala Ser Asp Ser Glu Ile
290 295 300

Asp Gln Asp Asp Asp Ser Gly Pro Leu Ala Gly Glu Asn Val Met Asn
305 310 315 320

Val Ile Val Val Ala Ala Glu Cys Ser Pro Trp Cys Lys Thr Gly Gly
325 330 335

Leu Gly Asp Val Ala Gly Ala Leu Pro Lys Ala Leu Ala Arg Arg Gly
340 345 350

His Arg Val Met Val Val Val Pro Arg Tyr Gly Asp Tyr Ala Glu Ala
355 360 365

Gln Asp Val Gly Ile Arg Lys Tyr Tyr Lys Ala Ala Gly Gln Asp Leu
370 375 380

```
Glu Val Lys Tyr Phe His Ala Phe Ile Asp Gly Val Asp Phe Val Phe
385                 390                 395                 400

Ile Asp Ala Pro Leu Phe Arg His Arg Gln Asp Asp Ile Tyr Gly Gly
                405                 410                 415

Asn Arg Gln Glu Ile Met Lys Arg Met Ile Leu Phe Cys Lys Ala Ala
            420                 425                 430

Val Glu Val Pro Trp His Val Pro Cys Gly Gly Val Pro Tyr Gly Asp
        435                 440                 445

Gly Asn Leu Val Phe Leu Ala Asn Asp Trp His Thr Ala Leu Leu Pro
    450                 455                 460

Val Tyr Leu Lys Ala Tyr Tyr Arg Asp Asn Gly Met Met Gln Tyr Thr
465                 470                 475                 480

Arg Ser Val Leu Val Ile His Asn Ile Ala Tyr Gln Gly Arg Gly Pro
                485                 490                 495

Val Asp Glu Phe Pro Tyr Met Glu Leu Pro Glu His Tyr Leu Asp His
            500                 505                 510

Phe Lys Leu Tyr Asp Pro Val Gly Gly Glu His Ala Asn Ile Phe Gly
        515                 520                 525

Ala Gly Leu Lys Met Ala Asp Arg Val Val Thr Val Ser Pro Gly Tyr
    530                 535                 540

Leu Trp Glu Leu Lys Thr Thr Glu Gly Gly Trp Gly Leu His Asp Ile
545                 550                 555                 560

Ile Arg Glu Asn Asp Trp Lys Met Asn Gly Ile Val Asn Gly Ile Asp
                565                 570                 575

Tyr Arg Glu Trp Asn Pro Glu Val Asp Val His Leu Gln Ser Asp Gly
            580                 585                 590

Tyr Ala Asn Tyr Thr Val Ala Ser Leu Asp Ser Ser Lys Pro Arg Cys
        595                 600                 605

Lys Ala Ala Leu Gln Arg Glu Leu Gly Leu Glu Val Arg Asp Asp Val
    610                 615                 620

Pro Leu Ile Gly Phe Ile Gly Arg Leu Asp Gly Gln Lys Gly Val Asp
625                 630                 635                 640

Ile Ile Gly Asp Ala Met Pro Trp Ile Ala Gly Gln Asp Val Gln Leu
                645                 650                 655
```

Val Leu Leu Gly Ser Gly Arg Arg Asp Leu Glu Val Met Leu Gln Arg
660 665 670

Phe Glu Ala Gln His Asn Ser Lys Val Arg Gly Trp Val Gly Phe Ser
675 680 685

Val Lys Met Ala His Arg Ile Thr Ala Gly Ala Asp Val Leu Val Met
690 695 700

Pro Ser Arg Phe Glu Pro Cys Gly Leu Asn Gln Leu Tyr Ala Met Ala
705 710 715 720

Tyr Gly Thr Val Pro Val Val His Ala Val Gly Gly Leu Arg Asp Thr
725 730 735

Met Ser Ala Phe Asp Pro Phe Glu Asp Thr Gly Leu Gly Trp Thr Phe
740 745 750

Asp Arg Ala Glu Pro His Lys Leu Ile Glu Ala Leu Gly His Cys Leu
755 760 765

Glu Thr Tyr Arg Lys Tyr Lys Glu Ser Trp Arg Gly Leu Gln Val Arg
770 775 780

Gly Met Ser Gln Asp Leu Ser Trp Asp His Ala Ala Glu Leu Tyr Glu
785 790 795 800

Glu Val Leu Val Lys Ala Lys Tyr Gln Trp
805 810

<210> 3
<211> 2433
<212> DNA
<213> Artificial sequence

<220>
<223> Synthetic DNA, mutation at position 2209

<400> 3
atgtctagcg cggtggttgc gtccagcaca acttttctcg tcgcacttgc ctctagcgcg 60

agccggggcg ggccacgtag ggggcgcgtc gtgggcgtgg ccgctccccc agccctcctg 120

tatgacggga gagctggcag gctagccctg cgcgcccctc cgccaccccg ccctagacct 180

aggcgcaggg atgcgggtgt tgtcaggcgg gctgatgacg gggagaacga ggccgcagtg 240

gagcgggccg gcgaggacga tgacgaggag gaggagttct cgtccggggc ctggcagcca 300

ccgcgttcaa ggcgcggtgg agttggcaag gtcctcaaac gtcgcggtac cgtgccgcca 360

gtcggaaggt acggctccgg tggagacgcc gctcgggtga gaggagccgc ggcacccgct 420

ccagcaccga cgcaagacgc agcgtcgtct aagaatggcg cgcttttgtc aggcagggat 480

gacgacacac ctgcctcacg gaacggatcg gtcgttaccg gcgccgacaa gcctgccgcc 540

gccacgccgc cggtgaccat aacgaagctc ccagcgccgg actcccccgt gatccttcca 600
tccgtagaca agccgcagcc ggagttcgtc atcccagacg cgacggcgcc ggcgccgcca 660
ccgcccggtt caaatcccag gtcgtccgct cctctcccca agcctgacaa ttcggaattt 720
gcagaggata agagcgcaaa agttgttgag agtgctccga agccaaaggc gactagatct 780
tcccctattc ctgcggtaga agaggagacg tgggatttca agaaatattt tgatctgaac 840
gaaccggacg ccgcggagga tggcgatgac gatgatgact gggctgattc agatgcgtca 900
gattctgaga tcgaccagga tgacgattcg ggtcctttgg ctggggagaa tgtcatgaac 960
gtgatcgtgg tggctgctga atgttctccc tggtgcaaaa caggtgggct tggagatgtt 1020
gcaggtgctt tacccaaggc tttggcgagg agaggacatc gtgttatggt tgtcgtacca 1080
aggtacggtg attacgcgga agcccaggat gtaggaatca ggaaatacta caaggctgct 1140
ggacaggatc tggaagtgaa atatttccat gcatttatcg acggagttga ttttgtgttc 1200
attgacgctc ctctcttccg tcaccgtcag gatgacatct atggggggaa cagacaggaa 1260
atcatgaagc gcatgattct gttttgtaag gctgctgttg aggttccttg gcacgttcca 1320
tgcggtggtg tgccctatgg ggatggcaac ttggtgttcc ttgcaaacga ttggcacact 1380
gcactcctgc ctgtttatct gaaggcatat tacagagaca atggcatgat gcagtacact 1440
cgctctgtcc ttgtgataca taatatcgct taccagggcc gtggcccagt agatgaattc 1500
ccctacatgg aattgccgga gcactacctg gatcacttca agctgtacga ccccgtcggc 1560
ggcgagcacg ccaacatctt cggcgcgggc ctgaagatgg cggaccgggt ggtgaccgtg 1620
agccccggct acctctggga gctgaagacg acggagggcg gctggggcct ccacgacatc 1680
atacgggaga acgactggaa gatgaacggc atcgtgaacg gcatcgacta ccgggagtgg 1740
aacccggagg tggacgtgca cctgcagtcc gacggctacg ccaactacac cgtggcctcg 1800
ctggactcca gcaagccgcg gtgcaaggcg gcgctgcagc gcgagctggg gctggaggtg 1860
cgcgacgacg tgccgctgat cgggttcatc gggcggctcg acgggcagaa aggtgtggac 1920
atcatcggcg acgcgatgcc gtggatcgcc gggcaggacg tgcagctggt gctgctgggc 1980
tccggccgcc gcgacctgga ggtgatgctg cagcggttcg aggcgcagca caacagcaag 2040
gtgcgcgggt gggtggggtt ctcggtgaag atggcgcacc ggatcacggc gggcgccgac 2100
gtgctggtca tgccgtcgcg gttcgagccg tgcggcctca accagctcta cgccatggcg 2160
tacggcaccg tccccgtcgt gcacgccgtc ggcgggctga gggacaccgt gtcggcgttc 2220
gacccgttcg aggacaccgg cctcgggtgg acgttcgacc gcgccgagcc gcacaagctc 2280
atcgaggcgc tcggccactg cctggagacg taccgcaagt acaaggagag ctggaggggg 2340
ctccaggtgc gcggcatgtc gcaggacctc agctgggacc acgccgccga gctctacgag 2400
gaggtccttg tcaaggccaa gtaccaatgg tga 2433

<210> 4
<211> 810
<212> PRT

<213> Artificial

<220>
<223> Point mutation of amino acid 737 (valin instead of methionin)

<400> 4

```
Met Ser Ser Ala Val Val Ala Ser Ser Thr Thr Phe Leu Val Ala Leu
1               5                   10                  15

Ala Ser Ser Ala Ser Arg Gly Gly Pro Arg Arg Gly Arg Val Val Gly
            20                  25                  30

Val Ala Ala Pro Pro Ala Leu Leu Tyr Asp Gly Arg Ala Gly Arg Leu
        35                  40                  45

Ala Leu Arg Ala Pro Pro Pro Pro Arg Pro Arg Pro Arg Arg Arg Asp
    50                  55                  60

Ala Gly Val Val Arg Arg Ala Asp Asp Gly Glu Asn Glu Ala Ala Val
65                  70                  75                  80

Glu Arg Ala Gly Glu Asp Asp Asp Glu Glu Glu Glu Phe Ser Ser Gly
                85                  90                  95

Ala Trp Gln Pro Pro Arg Ser Arg Arg Gly Gly Val Gly Lys Val Leu
            100                 105                 110

Lys Arg Arg Gly Thr Val Pro Pro Val Gly Arg Tyr Gly Ser Gly Gly
        115                 120                 125

Asp Ala Ala Arg Val Arg Gly Ala Ala Ala Pro Ala Pro Ala Pro Thr
    130                 135                 140

Gln Asp Ala Ala Ser Ser Lys Asn Gly Ala Leu Leu Ser Gly Arg Asp
145                 150                 155                 160

Asp Asp Thr Pro Ala Ser Arg Asn Gly Ser Val Val Thr Gly Ala Asp
                165                 170                 175

Lys Pro Ala Ala Ala Thr Pro Pro Val Thr Ile Thr Lys Leu Pro Ala
            180                 185                 190

Pro Asp Ser Pro Val Ile Leu Pro Ser Val Asp Lys Pro Gln Pro Glu
        195                 200                 205

Phe Val Ile Pro Asp Ala Thr Ala Pro Ala Pro Pro Pro Pro Gly Ser
    210                 215                 220

Asn Pro Arg Ser Ser Ala Pro Leu Pro Lys Pro Asp Asn Ser Glu Phe
225                 230                 235                 240

Ala Glu Asp Lys Ser Ala Lys Val Val Glu Ser Ala Pro Lys Pro Lys
```

```
            245                 250                 255
Ala Thr Arg Ser Ser Pro Ile Pro Ala Val Glu Glu Glu Thr Trp Asp
        260                 265                 270
Phe Lys Lys Tyr Phe Asp Leu Asn Glu Pro Asp Ala Ala Glu Asp Gly
    275                 280                 285
Asp Asp Asp Asp Asp Trp Ala Asp Ser Asp Ala Ser Asp Ser Glu Ile
    290                 295                 300
Asp Gln Asp Asp Asp Ser Gly Pro Leu Ala Gly Glu Asn Val Met Asn
305                 310                 315                 320
Val Ile Val Val Ala Ala Glu Cys Ser Pro Trp Cys Lys Thr Gly Gly
            325                 330                 335
Leu Gly Asp Val Ala Gly Ala Leu Pro Lys Ala Leu Ala Arg Arg Gly
        340                 345                 350
His Arg Val Met Val Val Val Pro Arg Tyr Gly Asp Tyr Ala Glu Ala
    355                 360                 365
Gln Asp Val Gly Ile Arg Lys Tyr Tyr Lys Ala Ala Gly Gln Asp Leu
    370                 375                 380
Glu Val Lys Tyr Phe His Ala Phe Ile Asp Gly Val Asp Phe Val Phe
385                 390                 395                 400
Ile Asp Ala Pro Leu Phe Arg His Arg Gln Asp Asp Ile Tyr Gly Gly
            405                 410                 415
Asn Arg Gln Glu Ile Met Lys Arg Met Ile Leu Phe Cys Lys Ala Ala
        420                 425                 430
Val Glu Val Pro Trp His Val Pro Cys Gly Gly Val Pro Tyr Gly Asp
    435                 440                 445
Gly Asn Leu Val Phe Leu Ala Asn Asp Trp His Thr Ala Leu Leu Pro
    450                 455                 460
Val Tyr Leu Lys Ala Tyr Tyr Arg Asp Asn Gly Met Met Gln Tyr Thr
465                 470                 475                 480
Arg Ser Val Leu Val Ile His Asn Ile Ala Tyr Gln Gly Arg Gly Pro
            485                 490                 495
Val Asp Glu Phe Pro Tyr Met Glu Leu Pro Glu His Tyr Leu Asp His
        500                 505                 510
Phe Lys Leu Tyr Asp Pro Val Gly Gly Glu His Ala Asn Ile Phe Gly
```

515 520 525

Ala Gly Leu Lys Met Ala Asp Arg Val Val Thr Val Ser Pro Gly Tyr
530 535 540

Leu Trp Glu Leu Lys Thr Thr Glu Gly Gly Trp Gly Leu His Asp Ile
545 550 555 560

Ile Arg Glu Asn Asp Trp Lys Met Asn Gly Ile Val Asn Gly Ile Asp
565 570 575

Tyr Arg Glu Trp Asn Pro Glu Val Asp Val His Leu Gln Ser Asp Gly
580 585 590

Tyr Ala Asn Tyr Thr Val Ala Ser Leu Asp Ser Ser Lys Pro Arg Cys
595 600 605

Lys Ala Ala Leu Gln Arg Glu Leu Gly Leu Glu Val Arg Asp Asp Val
610 615 620

Pro Leu Ile Gly Phe Ile Gly Arg Leu Asp Gly Gln Lys Gly Val Asp
625 630 635 640

Ile Ile Gly Asp Ala Met Pro Trp Ile Ala Gly Gln Asp Val Gln Leu
645 650 655

Val Leu Leu Gly Ser Gly Arg Arg Asp Leu Glu Val Met Leu Gln Arg
660 665 670

Phe Glu Ala Gln His Asn Ser Lys Val Arg Gly Trp Val Gly Phe Ser
675 680 685

Val Lys Met Ala His Arg Ile Thr Ala Gly Ala Asp Val Leu Val Met
690 695 700

Pro Ser Arg Phe Glu Pro Cys Gly Leu Asn Gln Leu Tyr Ala Met Ala
705 710 715 720

Tyr Gly Thr Val Pro Val Val His Ala Val Gly Gly Leu Arg Asp Thr
725 730 735

Val Ser Ala Phe Asp Pro Phe Glu Asp Thr Gly Leu Gly Trp Thr Phe
740 745 750

Asp Arg Ala Glu Pro His Lys Leu Ile Glu Ala Leu Gly His Cys Leu
755 760 765

Glu Thr Tyr Arg Lys Tyr Lys Glu Ser Trp Arg Gly Leu Gln Val Arg
770 775 780

Gly Met Ser Gln Asp Leu Ser Trp Asp His Ala Ala Glu Leu Tyr Glu

785 790 795 800

Glu Val Leu Val Lys Ala Lys Tyr Gln Trp
805 810

<210> 5
<211> 2400
<212> DNA
<213> Artificial

<220>
<223> Synthetic sequence

<400> 5
atgtcgagcg cagtcgcttc tgcagcgagt ttcctggcgc tggcttcggc ctccccgggg 60

cgttcccgga ggcgggccag ggttagcgcc cagccgccac acgcaggcgc cggtaggctg 120

cattggccgc catggccgcc acaaagaact gcgcgcgacg gcgccgttgc agccctcgca 180

gctggaaaga aggacgctgg gatcgatgac gcggcagcca gtgtgaggca accgcgcgca 240

ttgcgggggg gagccgctac aaaggtggcg gagagacggg atccagtcaa gaccctggac 300

cgtgacgcgg ccgagggcgg gggcccatca cctccggccg cgcggcaaga cgcggcaagg 360

ccaccttcta tgaacgggat gcccgtcaac ggggagaata agtccacggg tggcgggggc 420

gcgacgaagg attctgggct accaacccct gcgcgtgccc cccatccgag tactcagaac 480

cgcgcccccg tgaatggtga gaacaaggcg aacgtcgcgt ccccaccgac ctctatcgca 540

gaagcggcag ccagcgattc agctgcgacg atctcaatta gcgacaaggc tccggagtct 600

gtggtccccg cagagaagac acctccatca tctggcagca acttcgagtc aagcgcatcg 660

gcgccaggct ccgacacggt gagcgacgtc gagcaggagc tcaagaaggg agccgtcgtg 720

gttgaggaag cccctaagcc gaaggctctc tccccacccg ctgcacctgc tgtgcaggaa 780

gatctgtggg acttcaagaa gtatatcggc tttgaggaac cagttgaagc caaggacgat 840

ggcagggctg tggcggacga tgccgggagc ttcgaacacc atcagaacca cgattccggg 900

ccactcgcgg gggagaacgt gatgaacgtg gttgtggttg ccgctgagtg ctcgccctgg 960

tgtaagacag gtggactggg cgatgtcgcg ggagcactcc caaaggccct tgctaagaga 1020

gggcatcgtg tgatggtcgt ggtccccagg tatggcgact acgaggaagc atatgacgtt 1080

ggcgtcagga agtactataa ggctgccggg caggacatgg aggtcaacta ctttcatgcc 1140

tacattgacg gagtggattt cgtctttatt gatgctccac tcttcaggca ccgccaggaa 1200

gatatttacg gtggctccag gcaagagatc atgaagagaa tgattctatt ttgcaaggcg 1260

gccgtcgagg tcccctggca cgtgccatgc gggggtgtgc catacggcga tggcaacttg 1320

gtctttatcg ccaatgactg gcacaccgct ctgctccctg tctatttgaa ggcctattac 1380

cgcgatcacg gactgatgca atacacccgt tccatcatgg tgatccataa tattgcacac 1440

cagggacgcg gccccgtgga tgagtttccg ttcacagagc ttcccgaaca ctacctggag 1500

cacttccggc tatacgatcc ggtgggcggt gagcacgcaa actacttcgc tgcgggtctc 1560

```
aagatggccg accaagttgt cgttgtgtcc ccagggtacc tctgggagtt gaagactgtg    1620
gaaggtggct ggggactgca cgacatcatt cgccaaaacg actggaagac gcgcggcatc    1680
gttaatggca ttgacaatat ggagtggaat ccagaggtcg acgcgcattt gaagagtgac    1740
gggtacacta acttctcact aaggacgctt gacagcggaa agcggcaatg taaggaggcc    1800
ctccaaaggg aactcggcct ccaagtgagg gccgacgtgc ctctcctggg cttcatcgga    1860
agactcgacg gccaaaaggg ggtggaaatc atcgccgatg ccatgccatg gatcgtctct    1920
caggacgtcc agcttgtgat gttgggtacc ggccgccatg acttggagtc gatgctccaa    1980
cattttgaga gggagcatca cgacaaggtt cggggatggg tgggattcag cgtgcgccta    2040
gctcatagga tcacagccgg cgccgacgcc ctccttatgc cctcgagatt cgaaccgtgc    2100
ggactcaatc agctctatgc catggcgtac gggaccgtcc ctgtggttca cgcggtggga    2160
ggtttgcgcg acaccgttcc tccctttgat ccatttaatc actccggtct gggctggacc    2220
ttcgatcgtg ccgaggccca caagctcatc gaggccctcg gtcactgcct gaggacttac    2280
cgcgacttta aggaaagctg gagagccctc caagagcgcg ggatgtccca ggatttctca    2340
tgggagcatg ccgcgaagct ttacgaggac gttctcgtga aggccaagta ccaatggtga    2400

<210>  6
<211>  799
<212>  PRT
<213>  Triticum aestivum

<400>  6

Met Ser Ser Ala Val Ala Ser Ala Ala Ser Phe Leu Ala Leu Ala Ser
1               5                   10                  15

Ala Ser Pro Gly Arg Ser Arg Arg Arg Ala Arg Val Ser Ala Gln Pro
            20                  25                  30

Pro His Ala Gly Ala Gly Arg Leu His Trp Pro Pro Trp Pro Pro Gln
        35                  40                  45

Arg Thr Ala Arg Asp Gly Ala Val Ala Ala Leu Ala Ala Gly Lys Lys
    50                  55                  60

Asp Ala Gly Ile Asp Asp Ala Ala Ala Ser Val Arg Gln Pro Arg Ala
65                  70                  75                  80

Leu Arg Gly Gly Ala Ala Thr Lys Val Ala Glu Arg Arg Asp Pro Val
                85                  90                  95

Lys Thr Leu Asp Arg Asp Ala Ala Glu Gly Gly Gly Pro Ser Pro Pro
            100                 105                 110

Ala Ala Arg Gln Asp Ala Ala Arg Pro Pro Ser Met Asn Gly Met Pro
        115                 120                 125
```

Val Asn Gly Glu Asn Lys Ser Thr Gly Gly Gly Gly Ala Thr Lys Asp
    130                 135                 140

Ser Gly Leu Pro Thr Pro Ala Arg Ala Pro His Pro Ser Thr Gln Asn
145                 150                 155                 160

Arg Ala Pro Val Asn Gly Glu Asn Lys Ala Asn Val Ala Ser Pro Pro
                165                 170                 175

Thr Ser Ile Ala Glu Ala Ala Ala Ser Asp Ser Ala Ala Thr Ile Ser
            180                 185                 190

Ile Ser Asp Lys Ala Pro Glu Ser Val Val Pro Ala Glu Lys Thr Pro
        195                 200                 205

Pro Ser Ser Gly Ser Asn Phe Glu Ser Ser Ala Ser Ala Pro Gly Ser
    210                 215                 220

Asp Thr Val Ser Asp Val Glu Gln Glu Leu Lys Lys Gly Ala Val Val
225                 230                 235                 240

Val Glu Glu Ala Pro Lys Pro Lys Ala Leu Ser Pro Pro Ala Ala Pro
                245                 250                 255

Ala Val Gln Glu Asp Leu Trp Asp Phe Lys Lys Tyr Ile Gly Phe Glu
            260                 265                 270

Glu Pro Val Glu Ala Lys Asp Asp Gly Arg Ala Val Ala Asp Asp Ala
        275                 280                 285

Gly Ser Phe Glu His His Gln Asn His Asp Ser Gly Pro Leu Ala Gly
    290                 295                 300

Glu Asn Val Met Asn Val Val Val Val Ala Ala Glu Cys Ser Pro Trp
305                 310                 315                 320

Cys Lys Thr Gly Gly Leu Gly Asp Val Ala Gly Ala Leu Pro Lys Ala
                325                 330                 335

Leu Ala Lys Arg Gly His Arg Val Met Val Val Val Pro Arg Tyr Gly
            340                 345                 350

Asp Tyr Glu Glu Ala Tyr Asp Val Gly Val Arg Lys Tyr Tyr Lys Ala
        355                 360                 365

Ala Gly Gln Asp Met Glu Val Asn Tyr Phe His Ala Tyr Ile Asp Gly
    370                 375                 380

Val Asp Phe Val Phe Ile Asp Ala Pro Leu Phe Arg His Arg Gln Glu
385                 390                 395                 400

```
Asp Ile Tyr Gly Gly Ser Arg Gln Glu Ile Met Lys Arg Met Ile Leu
                405                 410                 415

Phe Cys Lys Ala Ala Val Glu Val Pro Trp His Val Pro Cys Gly Gly
            420                 425                 430

Val Pro Tyr Gly Asp Gly Asn Leu Val Phe Ile Ala Asn Asp Trp His
        435                 440                 445

Thr Ala Leu Leu Pro Val Tyr Leu Lys Ala Tyr Tyr Arg Asp His Gly
    450                 455                 460

Leu Met Gln Tyr Thr Arg Ser Ile Met Val Ile His Asn Ile Ala His
465                 470                 475                 480

Gln Gly Arg Gly Pro Val Asp Glu Phe Pro Phe Thr Glu Leu Pro Glu
                485                 490                 495

His Tyr Leu Glu His Phe Arg Leu Tyr Asp Pro Val Gly Gly Glu His
            500                 505                 510

Ala Asn Tyr Phe Ala Ala Gly Leu Lys Met Ala Asp Gln Val Val Val
        515                 520                 525

Val Ser Pro Gly Tyr Leu Trp Glu Leu Lys Thr Val Glu Gly Gly Trp
    530                 535                 540

Gly Leu His Asp Ile Ile Arg Gln Asn Asp Trp Lys Thr Arg Gly Ile
545                 550                 555                 560

Val Asn Gly Ile Asp Asn Met Glu Trp Asn Pro Glu Val Asp Ala His
                565                 570                 575

Leu Lys Ser Asp Gly Tyr Thr Asn Phe Ser Leu Arg Thr Leu Asp Ser
            580                 585                 590

Gly Lys Arg Gln Cys Lys Glu Ala Leu Gln Arg Glu Leu Gly Leu Gln
        595                 600                 605

Val Arg Ala Asp Val Pro Leu Leu Gly Phe Ile Gly Arg Leu Asp Gly
    610                 615                 620

Gln Lys Gly Val Glu Ile Ile Ala Asp Ala Met Pro Trp Ile Val Ser
625                 630                 635                 640

Gln Asp Val Gln Leu Val Met Leu Gly Thr Gly Arg His Asp Leu Glu
                645                 650                 655

Ser Met Leu Gln His Phe Glu Arg Glu His His Asp Lys Val Arg Gly
            660                 665                 670
```

Trp Val Gly Phe Ser Val Arg Leu Ala His Arg Ile Thr Ala Gly Ala
675 680 685

Asp Ala Leu Leu Met Pro Ser Arg Phe Glu Pro Cys Gly Leu Asn Gln
690 695 700

Leu Tyr Ala Met Ala Tyr Gly Thr Val Pro Val Val His Ala Val Gly
705 710 715 720

Gly Leu Arg Asp Thr Val Pro Pro Phe Asp Pro Phe Asn His Ser Gly
725 730 735

Leu Gly Trp Thr Phe Asp Arg Ala Glu Ala His Lys Leu Ile Glu Ala
740 745 750

Leu Gly His Cys Leu Arg Thr Tyr Arg Asp Phe Lys Glu Ser Trp Arg
755 760 765

Ala Leu Gln Glu Arg Gly Met Ser Gln Asp Phe Ser Trp Glu His Ala
770 775 780

Ala Lys Leu Tyr Glu Asp Val Leu Val Lys Ala Lys Tyr Gln Trp
785 790 795

<210> 7
<211> 13
<212> PRT
<213> Artificial

<220>
<223> Peptide

<400> 7

Pro Val Val His Ala Val Gly Gly Leu Arg Asp Thr Val
1 5 10

<210> 8
<211> 5
<212> PRT
<213> Artificial

<220>
<223> Peptide

<220>
<221> misc_feature
<222> (3)..(4)
<223> Xaa can be any naturally occurring amino acid

<400> 8

Ser Trp Xaa Xaa Ile
1 5

<210> 9
<211> 5

<212> PRT
<213> Artificial

<220>
<223> Peptide

<220>
<221> misc_feature
<222> (3)..(4)
<223> Xaa can be any naturally occurring amino acid

<400> 9

Ser Trp Xaa Xaa Leu
1               5

<210> 10
<211> 6
<212> PRT
<213> Artificial

<220>
<223> Peptide 5

<400> 10

Met Asn Val Ile Val Val
1               5

<210> 11
<211> 5
<212> PRT
<213> Artificial

<220>
<223> Peptide

<400> 11

Gly Gly Asn Arg Gln
1               5

<210> 12
<211> 6
<212> PRT
<213> Artificial

<220>
<223> Peptide

<400> 12

Met Ala Asp Arg Val Val
1               5

<210> 13
<211> 5
<212> PRT
<213> Artificial

<220>
<223> Peptide

<400> 13

Glu Leu Lys Thr Thr
1 5

<210> 14
<211> 6
<212> PRT
<213> Artificial sequence

<220>
<223> Peptide

<400> 14

Arg Ala Glu Pro His Leu
1 5

<210> 15
<211> 5
<212> PRT
<213> Artificial sequence

<220>
<223> Peptide

<400> 15

Leu Asp Ser Ser Lys
1 5

<210> 16
<211> 26
<212> DNA
<213> Artificial sequence

<220>
<223> PCR Primer

<400> 16
ctgagggaca ccgtgtcggc gttcga 26

<210> 17
<211> 26
<212> DNA
<213> Artificial sequence

<220>
<223> PCR Primer

<400> 17
tcgaacgccg acacggtgtc cctcag 26

<210> 18
<211> 2400
<212> DNA
<213> Triticum aestivum

<400> 18
atgtcgtcgg cggtcgcgtc cgccgcatcc ttcctcgcgc tcgcgtcagc ctcccccggg 60

agatcacgca ggcgggcgag ggtgagcgcg cagccacccc acgccggggc cggcaggttg 120

cactggccgc cgtggccgcc gcagcgcacg gctcgcgacg gagctgtggc ggcgctcgcc 180
gccgggaaga aggacgcggg gatcgacgac gccgccgcgt ccgtgaggca gccccgcgca 240
ctccgcggtg gcgccgccac caaggtcgcg gagcgaaggg atcccgtcaa gacgctcgac 300
cgcgacgccg cggaaggcgg cgggccgtcc ccgccggcag cgaggcagga cgccgcccgt 360
ccgccgagta tgaacggcat gccggtgaac ggcgagaaca aatctaccgg cggcggcggc 420
gcgactaaag acagcgggct gcccacgccc gcacgcgcgc cccatccgtc gacccagaac 480
agagcaccgg tgaacggtga aaacaaagct aacgtcgcct cgccgccgac gagcatagcc 540
gaggccgcgg cttcggattc cgcagctacc atttccatca gcgacaaggc gccggagtcc 600
gttgtcccag ctgagaagac gccgccgtcg tccggctcaa atttcgagtc ctcggcctct 660
gctcccgggt ctgacactgt cagcgacgtg gaacaagaac tgaagaaggg tgcggtcgtt 720
gtcgaagaag ctccaaagcc aaaggctctt tcgccgcctg cagcccccgc tgtacaagaa 780
gacctttggg atttcaagaa atacattggt ttcgaggagc ccgtggaggc caaggatgat 840
ggccgggctg tcgcagatga tgcgggctcc tttgaacacc accagaatca cgactccgga 900
cctttggcag gggagaatgt catgaacgtg gtcgtcgtgg ctgctgagtg ttctccctgg 960
tgcaaaacag gtggtctggg agatgttgcg ggtgctctgc ccaaggcttt ggcaaagaga 1020
ggacatcgtg ttatggttgt ggtaccaagg tatggggact atgaagaagc ctacgatgtc 1080
ggagtccgaa aatactacaa ggctgctgga caggatatgg aagtgaatta tttccatgct 1140
tatatcgatg gagttgattt tgtgttcatt gacgctcctc tcttccgaca ccgtcaggaa 1200
gacatttatg gggcagcag acaggaaatt atgaagcgca tgattttgtt ctgcaaggcc 1260
gctgttgagg ttccatggca cgttccatgc ggcggtgtcc cttatgggga tggaaatctg 1320
gtgtttattg caaatgattg gcacacggca ctcctgcctg tctatctgaa agcatattac 1380
agggaccatg gtttgatgca gtacactcgg tccattatgg tgatacataa catcgctcac 1440
cagggccgtg gccctgtaga tgaattcccg ttcaccgagt tgcctgagca ctacctggaa 1500
cacttcagac tgtacgaccc cgtgggtggt gaacacgcca actacttcgc cgccggcctg 1560
aagatggcgg accaggttgt cgtggtgagc cccgggtacc tgtgggagct gaagacggtg 1620
gagggcggct gggggcttca cgacatcata cggcagaacg actggaagac ccgcggcatc 1680
gtcaacggca tcgacaacat ggagtggaac cccgaggtgg acgcccacct caagtcggac 1740
ggctacacca acttctccct gaggacgctg gactccggca agcggcagtg caaggaggcc 1800
ctgcagcgcg agctgggcct gcaggtccgc gccgacgtgc cgctgctcgg cttcatcggc 1860
cgcctggacg ggcagaaggg cgtggagatc atcgcggacg ccatgccctg gatcgtgagc 1920
caggacgtgc agctggtgat gctgggcacc gggcgccacg acctggagag catgctgcag 1980
cacttcgagc gggagcacca cgacaaggtg cgcgggtggg tggggttctc cgtgcgcctg 2040
gcgcaccgga tcacggcggg ggcggacgcg ctcctcatgc cctcccggtt cgagccgtgc 2100
gggctgaacc agctctacgc catggcctac ggcaccgtcc ccgtcgtgca cgccgtcggc 2160

ggcctcaggg acaccgtgcc gccgttcgac cccttcaacc actccgggct cgggtggacg 2220
ttcgaccgcg ccgaggcgca caagctgatc gaggcgctcg ggcactgcct ccgcacctac 2280
cgagacttca aggagagctg gagggccctc caggagcgcg gcatgtcgca ggacttcagc 2340
tgggagcacg ccgccaagct ctacgaggac gtcctcgtca aggccaagta ccagtggtga 2400

**Beschreibung**

Die vorliegende Erfindung betrifft Weizenmehle, deren Stärkekomponente einen Amylosegehalt zwischen 15.0 Gew.-% bis 30.0 Gew.-% und die einen Gehalt an resistenter Stärke von mehr als 5.0 Gew.-% aufweisen sowie Lebensmittel enthaltend diese Weizenmehle. Ferner betrifft die vorliegende Erfindung Verfahren zur Herstellung besagter Weizenmehle und deren Verwendung als Präbiotikum oder zur Herstellung von Lebensmitteln mit verringertem glykämischen Index. Die vorliegende Erfindung betrifft auch Nucleinsäuremoleküle, die eine lösliche Stärkesynthase II kodieren, sowie Vektoren enthaltend solche Nucleinsäuremoleküle. Ferner betrifft die vorliegende Erfindung auch Wirtszellen und Pflanzen, die solche Nucleinsäuremoleküle oder Vektoren umfassen.

Der Einsatz resistenter Stärke (RS) gewinnt in der Lebensmittelindustrie zunehmend an Bedeutung. Stärke wird hauptsächlich im Dünndarm durch das Enzym alpha-Amylase verdaut, das die alpha-1,4-glucosidischen Bindungen der Stärke zu Zuckern hydrolysiert. Im Gegensatz dazu wird die resistente Stärke im Dünndarm nicht durch alpha-Amylasen verdaut, sondern geht in den Dickdarm über, wo sie sich ähnlich wie Ballaststoffe verhält. Aus dem Abbau von RS-haltigen Produkten bezieht der Organismus nur in geringem Umfang Energie. Diese Energiezufuhr betrifft ausschließlich den oxidativen Abbau resorbierter kurzkettiger Fettsäuren aus dem Dickdarm. Diese kurzkettigen Fettsäuren sind Endprodukte des Kohlenhydratstoffwechsels der intestinalen Mikroflora. Mit der Aufnahme RS-haltiger Lebensmittel werden Substrate für den Energiestoffinrechsel der intestinalen Mikroflora und der Dickdarmepithelzellen bereitgestellt. Letztere sind zur Aufrechterhaltung ihrer Struktur und Funktion auf die luminale Zufuhr der kurzkettigen Fettsäuren und insbesondere von Butyrat angewiesen. Resistente Stärke ist wahrscheinlich ein Faktor zur Verhinderung von Divertikulose und Dickdarmkrebs.

Es wird zwischen den folgenden Typen resistenter Stärke unterschieden:

- $RS_1$ Physikalisch der Verdauung unzugängliche Stärke, z.B. in ein Protein oder eine Fasermatrix eingebettete Stärke. Wenn diese physikalisch (z.B. durch Kauen) oder chemisch (z.B. durch Abbau der sie umgebenden Matrix) aufgeschlossen wird, kann sie durch die Verdauungssäfte in normaler Weise bearbeitet werden.
- $RS_2$ Unverdauliche intakte (granuläre) native Stärkekörner, z.B. ungekochte Kartoffel- oder Bananenstärke insbesondere von unreifen Bananen)
- $RS_3$ Unverdauliche retrogradierte Stärke, die nicht granulär ist
- $RS_4$ Unverdauliche chemisch modifizierte Stärke, z.B. durch Quervernetzung oder Veresterung (Acetylierung etc.)

Im Gegensatz zu RS 4 können die RS-Formen 1 bis 3 durch Lösung in NaOH oder Dimethylsulfoxid dem alpha-Amylase-Abbau zugänglich gemacht werden.

Zur Herstellung von resistenter Stärke wurden verschiedene Verfahren beschrieben. Die meisten dieser Verfahren betreffen die Herstellung von RS3-Stärken (EP 564893 A1; EP 688872 A1; EP 846704 A1; US5051271). Alle diese Verfahren zur Herstellung resistenter Stärke beinhalten das Dispergieren und Gelatinisieren von Stärke in großen Überschussmengen an Wasser, gefolgt von einer Retrogradation unter Verwendung von Enzymen oder Säuren. Sie beruhen auf der Auffassung, dass resistente Stärke gebildet wird, wenn die Amylosefraktion von Stärke nach der Gelatinisierung von Stärke retrogradiert. Man geht davon aus, dass sich die linearen Amylosemoleküle nach Gelatinisierung zu dichten, durch Wasserstoffbrückenbindungen gebundenen Doppelhelixkonfigurationen anordnen, so dass die alpha-1,4-Glucosidbindungen für alpha-Amylasen nicht mehr zugänglich sind. Diese Verfahren sind arbeitsintensiv, zeitaufwendig und können zu geringen Ausbeuten führen. Darüber hinaus kann der große Wassergehalt der Produkte teure Trocknungsschritte erforderlich machen.

Granuläre Stärken des Typs RS2 mit einem hohen Gehalt an resistenter Stärke findet man vor allem bei nativen, ungekochten Wildtyp-Kartoffelstärken, die je nach Bestimmungsmethode einen RS-Gehalt zwischen 74-85 Gew.-% aufweisen (Faisant et al., Sciences des Aliments 15, (1995), 83-89; Evans and Thompson, Cereal Chemistry 81(1), (2004), 31-37).

Bisher bekannte granuläre Maisstärken mit hohem RS-Anteil zeichnen sich immer durch einen hohen Amylosegehalt (>40 Gew.-%) aus. Für native, d.h. granuläre Maisstärken mit hohem Amylosegehalt, die in verschiedenen Maispflanzen des Genotyps amylose extender ("ae") synthetisiert werden, wurden mit Hilfe der RS-Bestimmungsmethode von Englyst et al. (Europ. J. of Clinical Nutrition 46 (Suppl. 2), (1992), S 33-50) RS-Werte zwischen etwa 40-70 Gew.-% ermittelt (Evans and Thompson, Cereal Chemistry 81(1), (2004), 31-37). Auch der von Faisant et al. mit Hilfe zweier anderer RS-Bestimmungsmethoden ermittelten RS-Gehalte für native, d.h. granuläre Amylomaisstärke des Typs Hylon VII (identisch zu ae VII, die von Evans and Thompson untersucht wurde) liegen mit ca. 54 Gew.-% bzw. 67 Gew-% in diesem Bereich, der auch durch eine laborübergreifende Studie bestätigt wird, die mit Hilfe unterschiedlicher RS-Bestimmungsmethoden RS-Werte für native Amylomaisstärke zwischen etwa 50 und 72 Gew.-% ermittelt (McCleary und Monaghan, J. AOAC Int. 85, (2002), 665-675). Derartige granuläre Amylomaisstärken von amylose extender (ae) Mutanten weisen bei bestimmten Produktgruppen den Nachteil schlechter Verarbeitungseigenschaften auf, weil diese Stärken kaum verkleistern, eine geringe Löslichkeit und geringes Quellvermögen aufweisen. Für Anwendungen, in denen nur verkleisterte Stärken einsetzbar sind oder die lösliche Stärken oder Stärken mit Quellvermögen erfordern, sind die Amylomaisstärken daher entweder überhaupt nicht geeignet, oder sie müssen zusätzlich chemisch modifiziert werden, um diese Anforderungen zu erfüllen, was zeit- und kostenintensiv ist (Senti und Russell, Tappi Vol. 43, No.4, (April 1960), 343-349; Z. Luo et al., Starch/Stärke 58, (2006), 468-474).

Weizen-Stärken mit einem gegenüber Weizen-Wildtyp-Pflanzen erhöhten RS-Anteil sind erst seit kurzem bekannt und bisher nur in sehr begrenztem Umfang verfügbar. Der erhöhte RS-Anteil der bisher bekannten RS-Weizenstärken beruht - wie bei den Amylomaisstärken - auf einer Erhöhung des Amylosegehaltes. Im Unterschied zu den ae-Mutanten in Mais, die auf einer Mutation des BEIIb-Gens aus Mais beruhen und einen Amylosegehalt zwischen 50 bis 90 Gew.-% aufweisen, zeigt sich die für die Steigerung des RS-Gehaltes erforderliche Erhöhung des Amylosegehaltes bei Weizen nach Inhibierung der Genexpression der Verzweigungsenzyme IIa und IIb (Regina et al., PNAS Vol. 103 No. 10, (2006), 3546-3551). Ein alternativer Ansatz, der in Weizen auch zu einem erhöhten Amylosegehalt und erhöhtem RS-Gehalt der Weizenstärke gegenüber der Stärke von Weizen-Wildtyp-Pflanzen führt, beruht auf der Inhibierung des Gens der löslichen Stärkesynthase IIa (SSIIa) (Yamamori et al., Australian Journal of Agricultural Research 57, (2006), 531-535). Diese SSIIa inhibierten Weizen-Pflanzen weisen eine Stärke mit einem erhöhten apparenten Amylosegehalt auf, für den Werte von 37 Gew.-% (Yamamori et al., Australian Journal of Agricultural Research 57, (2006), 531-535) und 44 Gew.-% (Konik-Rose et al., Theor. Appl. Genet. 115, (2007), 1053-1065) ermittelt wurden. Die Erhöhung des apparenten Amylosegehaltes führt zu einem RS-Gehalt der nativen Weizenstärke von bis zu 3,6 Gew.-%, wohingegen native (granuläre) Weizen-Stärken von Wildtyp-Pflanzen keine oder kaum resistente Stärke enthalten (Yamamori et al., Australian Journal of Agricultural Research 57, (2006), 531-535). Das Weizenmehl dieser SSIIa inhibierten Weizenpflanzen führt beim Backen zu einer unerwünschten Verringerung des Brotvolumens (Morita et al., Cereal Chemistry 79, (2002), 491-495) und der aus dem Weizenmehl hergestellte Teig zeigt eine verringerte Teigstabilität (Morita et al., Cereal Chemistry 79, (2002), 491-495; Hung et al., Cereal Chemsitry 82, (2005), 690-694; Hung et al., Trends in Food Science & Technology 17, (2006), 448-456). Die Fachwelt geht davon aus, dass eine Steigerung des RS-Gehaltes von Weizenstärken oder -mehlen über eine Steigerung des apparenten Amylosegehaltes erzielt werden kann (Morell et al., Journal of AOAC International Vol. 87 No. 3, (2004), 740-748; Yamamori et al., Australian Journal of Agricultural Research 57, (2006), 531-535).

Neben resistenten Stärken (RS) sind bei der Lebensmittelzubereitung zunehmend auch Stärken oder Mehle mit geringem Anteil an schnell verdaulicher Stärke (rapidly digestible starch = RDS) gefragt. Denn es besteht der Verdacht, dass der fortwährende Konsum von Lebensmitteln mit einer hohen glykämischen Ladung, wie z.B. bei herkömmlichen stärkehaltigen Lebensmitteln mit relativ hohem RDS-Anteil, und die damit verbundene Insulinausschüttung ein Risikofaktor bei der Entstehung von Krankheiten wie Bluthochdruck, Übergewicht, Herzkrankheiten und Diabetes Typ II ist. Nahrungsmittel mit hohem RDS-Anteil haben in der Regel einen hohen Glykämischen Index (=GI) (Englyst et al., British Journal of Nutrition, 75, 327-337). Die bei der Verdauung von herkömmlichen Stärken/Mehlen oder von Verarbeitungsprodukten dieser Stärken/Mehle (z.B. Backwaren, Nudeln) zu beobachtende schnelle Freisetzung größerer Mengen an Glukose und deren Absorption über das Dünndarmepithel führt zu einer abrupten Zunahme des Blutzuckerspiegels und zu einer Ausschüttung von Insulin (Insulinantwort). Verringert man den Gehalt an RDS einer Stärke oder eines Mehles so führt dies zu einer verlangsamten Freisetzung von Glukose aus der Stärke, zu einer veränderten Insulinantwort und somit letztlich zu einer Verringerung des Risikos für die oben genannten Krankheiten.

Der Einsatz von Weizenstärken und -mehlen mit geringem Anteil an RDS scheint vor allem in solchen Nahrungsmitteln wünschenswert, bei denen eine kontinuierliche Freisetzung von Glukose angestrebt wird, wie z.B. bei Sportlernahrung für Ausdauersport oder bei Diätnahrung zur Reduktion des Hungergefühls.

Aufgabe der vorliegenden Erfindung ist es daher, Weizenmehle/Weizenstärken zur Verfügung zu stellen, welche im Vergleich zu Weizenmehlen/Weizenstärken von Weizen-Wildtyppflanzen veränderte Verdauungseigenschaften, insbesondere einen erhöhten Anteil an resistenter Stärke (RS) und/oder einen verringerten Anteil an schnell verdaulicher Stärke (RDS) aufweisen.

Eine weitere Aufgabe der vorliegenden Erfindung ist es, Weizenmehle oder Weizenstärken zur Verfügung zu stellen, die neben den veränderten Verdauungseigenschaften solche Verarbeitungseigenschaften aufweisen, die gegenüber den Verarbeitungseigenschaften der im Stand der Technik beschriebenen Weizenmehlen/Weizenstärken mit erhöhtem Amylosegehalt verbessert sind. Unter verbesserten Verarbeitungseigenschaften sind in diesem Zusammenhang beispielsweise ein vergrößertes Brotvolumen der Brote und/oder eine erhöhte Teigstabilität und/oder eine erhöhte thermische Stabilität zu nennen.

Diese Aufgaben werden durch die in den Ansprüchen bezeichneten Ausführungsformen gelöst.

Die vorliegende Erfindung betrifft somit eine Weizenstärke, die einen Amylosegehalt zwischen 15.0 Gew.-% bis 30.0 Gew.-%, vorzugsweise zwischen 18.0.0 Gew.-% bis 30.0 Gew.-% und besonders bevorzugt zwischen 20.0 Gew.-% bis 30.0 Gew.-% und die einen Gehalt an resistenter Stärke (RS Stärke) (englisch: resistant starch, abgekürzt RS) von mehr als 5.0 Gew.-%, vorzugsweise zwischen 5.0 Gew.-% bis 35.0 Gew.-%, besonders bevorzugt zwischen 7.0 Gew.-% bis 30 Gew.-% aufweist.

In diesem Zusammenhang und im Zusammenhang mit der vorliegenden Erfindung soll der Begriff "zwischen" die jeweils angegebenen Zahlengrenzen nicht mit einschließen.

Da der Stand der Technik davon ausgeht, dass eine Steigerung des RS-Gehaltes von Weizenstärken oder -mehlen über eine Steigerung des apparenten Amylosegehaltes erzielt werden kann (Morell et al., Journal of AOAC International Vol. 87 No. 3, (2004), 740-748; Yamamori et al., Australian Journal of Agricultural Research 57, (2006), 531-535), ist es für den Fachmann sehr überraschend, dass die erfindungsgemäßen Weizen-Stärken/-Mehle einen gegenüber Stärken/Mehlen von Weizenwildtyp-Pflanzen deutlich erhöhten RS-Gehalt bei nahezu unverändertem oder sogar leicht verringertem apparentem Amylosegehalt aufweisen.

In einer weiteren Ausführungsform betrifft die vorliegende Erfindung eine Weizenstärke, die einen Amylosegehalt zwischen 15.0 Gew.-% bis 30.0 Gew.-%, vorzugsweise zwischen 18.0 Gew.-% bis 29.5 Gew.-% und besonders bevorzugt zwischen 20.0 Gew.-% bis 29.5 Gew.-% und einen Gehalt an resistenter Stärke (RS Stärke) (englisch: resistant starch, abgekürzt RS) von mehr als 5.0 Gew.-%, vorzugsweise zwischen 5.0 Gew.-% bis 15.0 Gew.-% oder zwischen 16.0 Gew.-% bis 29.0 Gew.-% aufweist.

Die vorliegende Erfindung betrifft auch eine Weizenstärke, die einen Amylosegehalt zwischen 15.0 Gew.-% bis 30.0 Gew.-%, vorzugsweise zwischen 18.0 Gew.-% bis 30.0 Gew.-% und besonders bevorzugt zwischen 20.0 Gew.-% bis 30.0 Gew.-% und die einen Gehalt an schnell verdaulicher Stärke (englisch: rapidly digestible starch = RDS Stärke) zwischen 10.0 Gew.-% bis 38 Gew.-%, vorzugsweise zwischen 15.0 Gew.-% bis 35.0 Gew.-%, besonders bevorzugt zwischen 20.0 Gew.-% bis 33 Gew.-% aufweist.

In einer weiteren Ausführungsform betrifft die vorliegende Erfindung eine Weizenstärke, die einen Gehalt an schnell verdaulicher Stärke (englisch: rapidly digestible starch = RDS) zwischen 10.0 Gew.-% bis 38 Gew.-%, vorzugsweise zwischen 15.0 Gew.-% bis 35.0 Gew.-%, besonders bevorzugt zwischen 20.0 Gew.-% bis 33 Gew.-% aufweist.

In einer weiteren Ausführungsform weist die erfindungsgemäße Weizenstärke einen RS-Gehalt (RS Stärke) zwischen 5.0 Gew.-% bis 35.0 Gew.-%, vorzugsweise zwischen 7.0 Gew.-% bis 32 Gew.-%, besonders bevorzugt zwischen 10 Gew.-% bis 30.0 Gew.-% auf.

Verfahren zur Bestimmung des Amylosegehaltes sind dem Fachmann bekannt. Einige dieser Methoden basieren auf dem Jodbindevermögen der Amylose, das potentiometrisch (Banks & Greenwood, in W. Banks & C.T. Greenwood, Starch and its components (pp. 51-66), Edinburgh, Edinburgh University Press), amperometrisch (Larson et al., Analytical Chemistry 25(5), (1953), 802-804) oder spektrophotometrisch (Morrison & Laignelet, J. Cereal Sc. 1, (1983), 9-20) bestimmt werden kann. Die Bestimmung des Amylosegehaltes kann auch kalorimetrisch mittels DSC-(Differential Scanning Calorimetry)-Messungen erfolgen (Kugimiya & Donovan, Journal of Food Science 46, (1981), 765-770; Sievert & Holm, Starch/Stärke 45 (4), (1993), 136-139). Ferner besteht die Möglichkeit, den Amylosegehalt über den Einsatz von SEC-(size exclusion chromatography)-Chromatographie von nativer oder entzweigter Stärke zu bestimmen.

Im Zusammenhang mit der vorliegenden Erfindung wird der Amylosegehalt der Stärkekomponente des erfindungsgemäßen Weizenmehles mit Hilfe der weiter unten beschriebenen DSC-Methode "Bestimmung des Gehaltes an apparenter Amlyose" ermittelt. Die DSC-Methode kann dabei wahlweise an einer Weizenstärkeprobe (eine Methode zur Isolierung von Weizenstärke aus Weizenmehl ist weiter unten beschrieben unter "Herstellung von Weizenmehlen und nachfolgende Extraktion von Weizenstärke") oder an einer Weizenmehlprobe durchgeführt werden.

Vorzugsweise wird die DSC-Methode an einer Weizenmehlprobe (zur Herstellung des Weizenmehls siehe "Herstellung von Weizenmehlen und nachfolgende Extraktion von Weizenstärke") durchgeführt. Der Amylosegehalt der Stärke ergibt sich dann - unter Annahme eines x-%-igen Gewichtsanteils der Stärke an einer Weizenmehlprobe (Hung et al., Trends in Food Science & Technology 17, (2006), 448-456) - rechnerisch gemäß folgender Formel:

$$\text{Amylosegehalt der Stärke} = \frac{\text{Amylosegehalt (Mehl)} \times 100}{x}$$

Der Gewichtsanteil der Stärke an der Mehlprobe wird vorzugsweise nach der in Methode 8 (Bestimmung des Anteiles an schnell verdaubarer und resistenter Stärke in Weizenmehlen/-stärken) beschriebenen Weise bestimmt.

Im Zusammenhang mit der vorliegenden Erfindung erfolgt die Bestimmung des RS-Gehaltes der Stärke (RS Stärke) vorzugsweise über die Methode von Englyst et al. (Europ. J. of Clinical Nutrition 46 (Suppl. 2), (1992), S 33-50, siehe insbesondere folgende Abschnitte aus Englyst et al., Seite S35-S36: "Reagents, Apparatus, Spectrophotometer"; Seite S36-S37, Absatz ,,Measurement of free glucose (FG)"; Seite S38, Absatz ,,Measurement of RDS and SDS"). Als resistenten Stärkeanteil der Stärke (RS Stärke) bezeichnet man den Anteil der eingewogenen Mehlprobe (Frischgewicht), der in der beschriebenen Methode nach 2 Stunden nicht als Glukose freigesetzt wird. Er ergibt sich demnach gemäß folgender Formel:

$$\text{RS Stärke in \% Gesamtstärke} = 100\% - 100\% \times (\text{freigesetzte Glukose nach 2 h in mg / Gesamtstärke in mg})$$

Der Gesamtstärkegehalt wird vorzugsweise nach der in Methode 8 (Bestimmung des Anteiles an schnell verdaubarer und resistenter Stärke in Weizenmehlen/-stärken) beschriebenen Weise bestimmt.

Im Zusammenhang mit der vorliegenden Erfindung soll unter "dem Gehalt an schnell verdaulicher Stärke (RDS)" (= Rapidly Digestible Starch = RDS Stärke) der Anteil einer Weizenstärke verstanden werden, der in der oben genannten Methode von Englyst et al. zur Bestimmung des RS-Gehaltes nach 20 Minuten als Glukose freigesetzt wird. Die Angabe des RDS Stärke-Gehaltes erfolgt in Gewichtsprozent der Gesamtstärke. Demnach gilt im Zusammenhang mit der vorliegenden Erfindung:

$$\text{RDS Stärke in \% Gesamtstärke} = 100\% \times \text{freigesetzte Glukose nach 20 Minuten in mg/ Gesamtstärke in mg}$$

Der Gesamtstärkegehalt wird vorzugsweise nach der in Methode 8 (Bestimmung des Anteiles an schnell verdaubarer und resistenter Stärke in Weizenmehlen/-stärken) beschriebenen Weise bestimmt.

Die Wärmeeigenschaften der erfindungsgemäßen Weizenstärke sowie des erfindungsgemäßen Weizenmehls lassen sich durch das Wärmefluss-Kalorimetrieverfahren (Differential Scanning Calorimetry = DSC) analysieren. Diese werden als Gelatinisierungstemperatur mit den Werten für die DSC T-onset (=Gelatinisierungstiefsttemperatur) sowie für DSC T-peak (=Gelatinisierungshöchsttemperatur) dargestellt.

Unter dem Begriff "DSC T-onset Temperatur" ist im Zusammenhang mit der vorliegenden Erfindung diejenige Temperatur zu verstehen, welche den Beginn der Phasenumwandlung der Stärke- bzw. der Mehlprobe darstellt. Sie wird charakterisiert als der Schnittpunkt zwischen der Verlängerung der Basislinie und der an die ansteigende Flanke des Peaks angelegten Tangente durch den Wendepunkt.

Unter dem Begriff "DSC T-peak Temperatur" wird im Zusammenhang mit der vorliegenden Erfindung die Temperatur bezeichnet, bei der die DSC-Kurve der Stärke- bzw. der Mehlprobe ein Maximum erreicht hat und die erste Ableitung der Kurve Null ist.

Die Bestimmung der "DSC T-onset"- sowie der "DSC T-peak"- Temperatur erfolgt im Zusammenhang mit der vorliegenden Erfindung nach der unten beschriebenen Methode ("Thermische Analyse von Weizenmehl/-stärke mittels Wärmefluss-Kalorimetrieverfahren (Differential Scanning Calorimetry").

Die vorliegende Erfindung betrifft in einer weiteren Ausführungsform eine Weizenstärke, die einen Amylosegehalt zwischen 15.0 Gew.-% bis 30.0 Gew.-%, vorzugsweise zwischen 18.0 Gew.-% bis 30.0 Gew.-% und besonders bevorzugt zwischen 20.0 Gew.-% bis 30.0 Gew.-% und die eine DSC T-onset-Temperatur zwischen 63.0 °C bis 70.0 °C, vorzugsweise zwischen 64.0 °C bis 69.0 °C, besonders bevorzugt zwischen 65.0 °C bis 68.0 °C aufweist.

Die vorliegende Erfindung betrifft in einer weiteren Ausführungsform eine Weizenstärke, die einen Amylosegehalt zwischen 15.0 Gew.-% bis 30.0 Gew.-%, vorzugsweise zwischen 18.0 Gew.-% bis 30.0 Gew.-% und besonders bevorzugt zwischen 20.0 Gew.-% bis 30.0 Gew.-% und die eine DSC T-peak-Temperatur zwischen 70.0 °C bis 78.0 °C, vorzugsweise zwischen 71.0 °C bis 75.0 °C aufweist.

In einer weiteren Ausführungsform weist die erfindungsgemäße Weizenstärke eine DSC T-peak-Temperatur zwischen 71.0 °C bis 77.0 °C, vorzugsweise zwischen 72.0 °C bis 75.0 °C auf.

In einer weiteren Ausführungsform betrifft die vorliegende Erfindung eine Weizenstärke, die neben einem Amylosegehalt zwischen 15.0 Gew.-% bis 30.0 Gew.-%, vorzugsweise zwischen 18.0 Gew.-% bis 29.5 Gew.-% und besonders bevorzugt zwischen 20.0 Gew.-% bis 29.5 Gew.-% zusätzlich wahlweise

a) einen RS-Gehalt zwischen 5.0 Gew.-% bis 30.0 Gew.-%, vorzugsweise zwischen 6.0 Gew.-% bis 29.5Gew.-%, besonders bevorzugt zwischen 7 Gew.-% bis 29.5 Gew.-% ; und/oder

b) einen Gehalt an schnell verdaulicher Stärke (englisch: rapidly digestible starch = RDS) bezogen auf die Stärkemenge (Trockengewicht) zwischen 10.0 Gew.-% bis 38 Gew.-%, vorzugsweise zwischen 15.0 Gew.-% bis 35.0 Gew.-%, besonders bevorzugt zwischen 20.0 Gew.-% bis 33 Gew.-%; und/oder

c) eine DSC T-onset-Temperatur zwischen 63.0 °C bis 70.0 °C, vorzugsweise zwischen 64.0 °C bis 69.0 °C, besonders bevorzugt zwischen 65.0 °C bis 68.0 °C; und/oder

d) eine DSC T-peak-Temperatur zwischen 70.0 °C bis 78.0 °C, vorzugsweise zwischen 71.0 °C bis 75.0 °C aufweist.

In einer weiteren Ausführungsform weist die erfindungsgemäße Weizenstärke eine veränderte Seitenkettenverteilung der Seitenketten des Amylopektins auf gegenüber der Seitenkettenverteilung des Amylopektins von Weizen-Wildtypstärke.

In einer weiteren Ausführungsform weist die erfindungsgemäße Weizenstärke eine Erhöhung des Anteils der Seitenketten des Amylopektins mit einem Polymerisationsgrad (DP) von DP 17-20 um 2%-20%, vorzugsweise um 5%-15% im Vergleich zum Anteil der entsprechenden Seitenketten des Amylopektins von entsprechenden Weizen-Wildtyppflanzen auf.

In einer weiteren Ausführungsform weist die erfindungsgemäße Weizenstärke eine Verringerung des Anteils der Seitenketten des Amylopektins mit einem Polymerisationsgrad (DP) von DP 6-11 um 5%-50%, vorzugsweise um 10%-15% auf im Vergleich zum Anteil der entsprechenden Seitenketten des Amylopektins von entsprechenden Weizen-Wildtyppflanzen.

In einer weiteren Ausführungsform weist die erfindungsgemäße Weizenstärke einen ACR-Wert von weniger als 0.160, vorzugsweise von weniger als 0.155 auf.

Unter dem ACR-Wert wird im Zusammenhang mit der vorliegenden Erfindung das Verhältnis aus der Summe der Anteile der Seitenketten mit einem DP6-10 geteilt durch die Summe der Anteile der Seitenketten mit einem DP6-24 verstanden.

Die Bestimmung der Seitenkettenverteilung erfolgt im Zusammenhang mit der vorliegenden Erfindung nach der weiter unten beschriebenen Methode ("Aufbereitung von Weizenmehl/-stärke zur Untersuchung der Amylopektin-Seitenkettenverteilung mittels Hochdruck-Anionenaustausch-Chromatographie"). Die Bestimmung des Anteils an Seitenketten erfolgt über die Bestimmung des prozentualen Anteils einer bestimmten Seitenkette am Gesamtanteil aller Seitenketten. Der Gesamtanteil aller Seitenketten wird ermittelt über die Bestimmung der Gesamtfläche unter den Peaks, die im HPLC-Chromatogramm die Polymerisationsgrade von DP 6 bis 50 repräsentieren. Der prozentuale Anteil einer bestimmten Seitenkette am Gesamtanteil aller Seitenketten wird über die Bestimmung des Verhältnisses der Fläche unter dem Peak ermittelt, der diese Seitenkette im HPLC-Chromatogramm repräsentiert, zur Gesamtfläche. Zur Bestimmung der Peakflächen kann beispielsweise das Programm Chromelion 6.60 der Firma Dionex, USA, verwendet werden.

In einer besonders bevorzugten Ausführungsform der vorliegenden Erfindung handelt es sich bei den erfindungsgemäßen Weizenstärken um granuläre Weizenstärken.

In einer weiteren besonders bevorzugten Ausführungsform der vorliegenden Erfindung handelt es sich bei den erfindungsgemäßen Weizenmehlen um Weizenmehle, deren Stärkekomponente, d.h. die erfindungsgemäße Weizenstärke, granulär ist.

Unter einer "granulären Weizenstärke" soll im Zusammenhang mit der vorliegenden Erfindung eine Weizenstärke verstanden werden, die nicht oder nicht vollständig verkleistert wurde und vorwiegend eine granuläre Struktur aufweist, d.h. mindestens 90%, vorzugsweise mindestens 95%, besonders bevorzugt mindestens 99% der Stärkekörner einer Stärkeprobe weisen eine granuläre Form auf. Vollständig retrogradierte Weizenstärke ist keine granuläre Weizenstärke im Sinne der vorliegenden Erfindung. Die granuläre Struktur eines Weizenstärkekorns führt im Lichtmikroskop unter polarisiertem Licht zu einer charakteristischen Lichtdoppelbrechung und ist hierüber bestimmbar (siehe beispielsweise Seite 126, Figur 4 in Yahl et al., Microscope 32, (1984), 123-132).

In einer weiteren Ausführungsform betrifft die vorliegende Erfindung die Verwendung der erfindungsgemäßen Weizenstärke, die vorzugsweise eine granuläre Weizen-stärke ist, als resistente Stärke.

In einer weiteren Ausführungsform betrifft die vorliegende Erfindung die Verwendung der erfindungsgemäßen Weizenstärke, die vorzugsweise eine granuläre Weizenstärke ist, als Präbiotikum. Denn die erfindungsgemäße Weizenstärke zeigt überraschenderweise einen erhöhten RS-Gehalt im Vergleich zu Weizenstärke von Weizen-Wildtyppflanzen. Bisher konnte ein präbiotischer Effekt nur für Weizenstärken mit einem erhöhten Amylosegehalt gezeigt werden (Regina et al., PNAS Vol. 103 No. 10, (2006), 3546-3551).

Die erfindungsgemäßen Weizenmehle/ Weizenstärken weisen zudem den Vorteil eines verringerten Anteils an schnell verdauliche(m/r ) Mehl bzw. Stärke (RDS) auf, was besonders vorteilhaft ist, da eine schnelle Freisetzung größerer Mengen Glukose und deren Absorption über das Dünndarmepithel zu einer abrupten Zunahme des Blutzuckerspiegels führt. In dessen Folge kommt es zu einer Ausschüttung von Insulin (Insulin Antwort). Der fortwährende Konsum von Lebensmitteln mit einer hohen glykämischen Ladung, und die damit verbundene Insulinausschüttung, stehen in Verdacht ein Risikofaktor bei der Entstehung von Krankheiten wie Bluthochdruck, Übergewicht, Herzkrankheiten und Diabetes Typ II zu sein.

In einer weiteren Ausführungsform betrifft die vorliegende Erfindung daher die Verwendung der erfindungsgemäßen Weizenstärke, die vorzugsweise eine granuläre Weizenstärke ist, oder des unten beschriebenen erfindungsgemäßen Weizenmehls zur Herstellung eines Lebensmittels, vorzugsweise eines für die Ernährung von Diabetikern oder für die Prävention von Bluthochdruck, Übergewicht, Herzkrankheiten oder Diabetes Typ II geeigneten Lebensmittels.

Aufgrund des Austausches herkömmlicher Weizenstärke bzw. Weizenmehls, z.B. aus Weizen-Wildtyp-Pflanzen, gegen die erfindungsgemäße Weizenstärke bzw. das erfindungsgemäße Weizenmehl zeigt das Lebensmittel vorzugsweise einen reduzierten glykämischen Index, der darauf zurückzuführen ist, dass die erfindungsgemäße Weizenstärke/das erfindungsgemäße Weizenmehl gegenüber Stärke/Mehl aus Weizen-Wildtyp-Pflanzen einen deutlich verringerten Gehalt an schnell verdaulicher Stärke (=RDS Stärke) bzw. schnell verdaulichem Mehl (=RDS Mehl) enthält.

In einer weiteren Ausführungsform betrifft die vorliegende Erfindung die Verwendung der erfindungsgemäßen - vorzugsweise granulären - Weizenstärke bzw. des erfindungsgemäßen Weizenmehls als Bestandteil von Diabetikernahrung oder zur Prävention von Bluthochdruck, Übergewicht, Herzkrankheiten oder Diabetes Typ II.

In einer weiteren Ausführungsform betrifft die vorliegende Erfindung auch die Verwendung der erfindungsgemäßen - vorzugsweise granulären - Weizenstärke bzw. des erfindungsgemäßen Weizenmehls zur Herstellung von Lebensmitteln, die einen verringerten glykämischen Index aufweisen im Vergleich zum glykämischen Index von Lebensmitteln, die Stärke bzw. Mehl aus Weizen-Wildtyp-Pflanzen enthalten.

In einer weiteren Ausführungsform betrifft die vorliegende Erfindung die Verwendung der erfindungsgemäßen - vorzugsweise granulären - Weizenstärke bzw. des erfindungsgemäßen Weizenmehls zur Verringerung des glykämischen Indexes von Lebensmitteln im Vergleich zum glykämischen Index von Lebensmitteln, die Stärke bzw. Mehl aus entsprechenden Weizen-Wildtyp-Pflanzen enthalten.

Der Glykämische Index (=GI) ist ein Maß zur Bestimmung der Wirkung eines kohlenhydrathaltigen Lebensmittels auf den Blutzuckerspiegel. Der Glykämische Index gibt in Zahlen die blutzuckersteigernde Wirkung der Kohlenhydrate bzw. der Lebensmittel an. Die blutzuckersteigernde Wirkung von Traubenzucker (=Glukose) oder Weißbrot dient hierbei in der Regel als Referenzwert (100).

Um den GI eines Lebensmittels zu ermitteln, misst man bei Versuchspersonen nach einer Mahlzeit den Blutzuckerverlauf, in der Regel über einen Zeitraum von 2 Stunden. Hierzu erhalten die Probanden das Lebensmittel, dessen GI festgestellt werden soll, in einer Menge, die genau 50 Gramm verwertbare Kohlenhydrate enthält. Nach der Test-"Mahlzeit" wird der Blutzucker regelmäßig gemessen und so dessen Verlauf beobachtet. Man nimmt die Messung an mehreren Versuchspersonen vor und errechnet einen Mittelwert, um die von Mensch zu Mensch unterschiedlich ausfallenden Blutzuckerkurven zu berücksichtigen. Die Flächen unter den Blutzuckerkurven werden integriert. Die Fläche, die sich nach der Aufnahme von Glukose (normalerweise 50 Gramm) (=Referenz-Lebensmittel) ergibt, wird als Standard gleich 100 gesetzt. Der GI für ein Lebensmittel beschreibt somit die relative Fläche unter der Blutzuckerkurve im Vergleich zur Kurve nach dem Referenz-Lebensmittel (Glukose) als Prozentwert.

Detaillierte Methodenbeschreibungen zur Bestimmung des glykämischen Indexes sind dem Fachmann bekannt und beispielsweise beschrieben von Wolever et al. (Am. J. Clin. Nutr. 54, (1991), 846-854) oder in FAO Food and Nutrition Paper 66, "Carbohydrates in human nutrition", Chapter 4-The Role of the Glycemic Index in Food Choice, pp. 25-30, Report from Apr. 14-18, (1997).

Ein hoher GI bedeutet, dass die Kohlenhydrate des Lebensmittels schnell zu Glukose abgebaut werden und ins Blut gelangen, so dass der Blutzuckerspiegel rasch ansteigt und eine starke, regulative Insulinausschüttung erfolgt. Lebensmittel mit einem mittleren oder niedrigen GI bewirken dagegen nur einen langsameren und insgesamt geringeren Anstieg der Blutzuckerkurve.

In einer weiteren Ausführungsform betrifft die vorliegende Erfindung die Verwendung der erfindungsgemäßen - vorzugsweise granulären - Weizenstärke bzw. des erfindungsgemäßen Weizenmehls zur Herstellung von Lebensmitteln, die nach Aufnahme durch den menschlichen Körper zu einem langsameren Anstieg des Blutzuckerspiegels führen als dies nach Aufnahme entsprechender Lebensmittel der Fall ist, die Stärke/Mehl aus (entsprechenden) Weizen-Wildtyp-Pflanzen enthalten.

Gegenüber den bisher bekannten Weizenstärken/Weizenmehlen weisen die erfindungsgemäßen Weizenstärken/Weizenmehle weiterhin den Vorteil auf, dass sie neben einem verringerten Anteil an RDS gegenüber Weizen-Wildtypstärken/- Wildtypmehlen gleichzeitig einen erhöhten Anteil an RS zeigen. Die mit dieser Erhöhung des RS-Anteils der Stärke/des Mehles einhergehenden Vorteile, wie z.B. die präbiotische Wirkung, werden somit gepaart mit den Vorteilen, die auf den verringerten RDS-Anteil der Stärke/des Mehles (z.B. verringerte glykämische Antwort) zurückzuführen sind.

Typische Lebensmittel, denen die erfindungsgemäße Stärke/das erfindungsgemäße Mehl zugesetzt werden kann oder die aus den erfindungsgemäßen Weizenstärken/Weizenmehlen hergestellt werden können, umfassen Tortillas, Tortillachips, Backwaren (z. B. Brot, Weizenbrot, Brötchen, Kekse, Kuchen, Waffeln, Muffins, Weizenfladen, Bagels), Pfannkuchen, Pizza, Teigwaren (z.B. Nudeln), Eintopfgerichte, Saucen, Weizenmehlpudding, Milchprodukte (z.B. Joghurt, Quark), Puddings, Aufstriche (z.B. Butter, Margarine), Getränke, Getränkepulver, Fertiggerichte, Soßen, (Frühstücks)-Cerealien und andere.

In einer weiteren Ausführungsform betrifft die vorliegende Erfindung ein Verfahren zur Herstellung einer erfindungsgemäßen - vorzugsweise granulären - Weizenstärke umfassend den Schritt der Extraktion der erfindungsgemäßen Stärke aus einer Weizen-Pflanze, die eine heterologe Stärkesynthase II exprimiert.

In einer weiteren Ausführungsform betrifft die vorliegende Erfindung ferner ein Verfahren zur Herstellung einer Stärke, umfassend den Schritt der Extraktion der Stärke aus einer Weizen-Pflanzenzelle, die eine heterologe Stärkesynthase II exprimiert.

In einer bevorzugten Ausführungsform der vorliegenden Erfindung wird die erfindungsgemäße Weizen-Stärke aus einer erfindungsgemäßen Weizen-Pflanze enthaltend erfindungsgemäße Weizen-Pflanzenzellen, aus erfindungsgemäßen Vermehrungsmaterial einer erfindungsgemäßen Weizen-Pflanze und/oder aus stärkespeichernden Teilen einer erfindungsgemäßen Weizen-Pflanze extrahiert.

Vorzugsweise umfasst das erfindungsgemäße Verfahren auch den Schritt des Erntens der kultivierten erfindungsgemäßen Weizen-Pflanzen oder der stärkespeichernden Pflanzenteile und/oder des erfindungsgemäßen Vermehrungsmaterials der erfindungsgemäßen Weizen-Pflanzen vor der Extraktion der Stärke. In einer weiteren Ausführungsform umfasst das erfindungsgemäße Verfahren auch den Schritt der Kultivierung der erfindungsgemäßen Weizen-Pflanzen vor dem Ernten.

Verfahren zur Extraktion der Stärke aus Pflanzen oder von stärkespeichernden Teilen von Weizen-Pflanzen sind dem Fachmann bekannt. Weiterhin sind Verfahren zur Extraktion der Stärke aus Weizen- Pflanzen beschrieben, z. B. in Starch: Chemistry and Technology (Hrsg.: Whistler, BeMiller und Paschall (1994), 2. Ausgabe, Academic Press Inc. London Ltd; ISBN 0-12-746270-8; siehe z.B. Kapitel XV, Seite 491 bis 506: Weizenstärke: Herstellung, Modifizierung und Verwendungen; oder in Eckhoff et al., Cereal Chem. 73 (1996), 54-57). Vorrichtungen, die gewöhnlich zur Extraktion von Stärke aus Pflanzen material verwendet werden, sind Separatoren, Dekanter, Hydrocyclone, Sprühtrockner und Wirbelschichttrockner.

Unter dem Begriff "stärkespeichernde Teile" sollen im Zusammenhang mit der vorliegenden Erfindung solche Teile einer Pflanze verstanden werden, in welchen Stärke im Gegensatz zu transitorischer Blattstärke zur Überdauerung von längeren Zeiträumen als Depot gespeichert wird. Bevorzugte stärkespeichemde Pflanzenteile sind Weizen-Körner, besonders bevorzugt sind Weizen-Körner enthaltend ein Endosperm.

Ferner betrifft die vorliegende Erfindung auch Weizen-Pflanzenzellen oder Weizen-Pflanzen, die eine heterologe Stärkesynthase II exprimieren.

In einer bevorzugten Ausführungsform synthetisieren die erfindungsgemäßen Weizen-Pflanzenzellen oder Weizen-Pflanzen die erfindungsgemäße Weizen-Stärke.

Im Zusammenhang mit der vorliegenden Erfindung soll unter dem Begriff ,,Stärkesynthase II" ein Protein verstanden werden, dass eine Glucosylierungsreaktion katalysiert, bei der Glucosereste des Substrates ADP-Glucose unter Bildung einer alpha-1,4-Verknüpfung auf alpha-1,4-verknüpfte Glucanketten übertragen werden (ADP-Glukose + {(1,4)-alpha-D-glucosyl}(N) <=> ADP + {(1,4)- alpha-D-glucosyl}(N+1)). Die Aminosäuresequenz der Stärkesynthase II weist eine Identität von mindestens 86%, bevorzugt mindestens 93%, besonders bevorzugt mindestens 95% mit den Aminosäuren 333 bis 362 (Domäne 1) der unter SEQ ID NO 4 dargestellten Aminosäuresequenz auf und/oder eine Identität von mindestens 83%, bevorzugt mindestens 86%, besonders bevorzugt mindestens 95% mit den Aminosäuren 434 bis 473 (Domäne 2) der unter SEQ ID NO 4 dargestellten Aminosäuresequenz und/oder eine Identität von mindestens 70%, vorzugsweise mindestens 82%, bevorzugt 86%, besonders bevorzugt 98%, insbesondere bevorzugt von mindestens 95% mit den Aminosäuren 652 bis 716 (Domäne 3) der unter SEQ ID NO 4 dargestellten Aminosäuresequenz auf.

In einer bevorzugten Ausführungsform der vorliegenden Erfindung weist die Stärkesynthase II mindestens eines, vorzugsweise zwei der folgenden Peptidmotive auf:

PVVHAVGGLRDTV (SEQ ID No. 7) und/oder zusätzlich entweder das Motiv SWXXI (SEQ ID No. 8) oder SWXXL (SEQ ID No. 9).

In einer bevorzugten Ausführungsform der vorliegenden Erfindung weist die Stärkesynthase II zusätzlich eines oder mehrere der folgenden Peptidmotive auf: MNVIW (SEQ ID No. 10), GGNRQ (SEQ ID No 11), MADRW (SEQ ID No.12), ELKTT (SEQ ID No.13), RAEPHL (SEQ ID No.14), LDSSK (SEQ ID No. 15).

Nucleinsäuresequenzen und die dazu korrespondierenden Aminosäuresequenzen, die die geforderte Identität mit den Domänen 1, 2 und 3 aufweisen und die eine Stärkesynthase II codieren, sind dem Fachmann bekannt und z.B. veröffentlicht von Gao und Chibbar, (Genome 43 (5), (2000), 768-775: Stärkesynthase II aus Weizen NCBI Acc No. AJ269502.1, AJ269503.1, AJ269504.1) oder unter Accession No. AF155217.2 (*Triticum aestivum*), AY133249 (*Hordeum vulgare*), Accession No AY133248 (*Aegilops tauschii*), Accession Nos XP467757, AAK64284 (*Oryza sativa*), Accession No AAK81729 (*Oryza sativa*) Accession Nos AAD13341, AAS77569, Accession No AAF13168 (*Manihut esculenta*), Accession No AAP41030 (*Colocasia esculenta*), Accession No AAS88880 (*Ostraeococcus tauri*), oder Accession No AAC17970 (*Chlamydomonas reinhardii*). Die genannten Nucleinsäuresequenzen und Aminosäuresequenzen codierend ein Protein mit der Aktivität einer Stärkesynthase II sind zugänglich über NCBI (http://www.ncbi.nlm.nih.gov/entrez/) und sind durch Nennung der Referenzen ausdrücklich in den Offenbarungsgehalt der vorliegenden Anmeldung aufgenommen.

Unter einer "heterologen" Stärkesynthase II ist im Zusammenhang mit der vorliegenden Erfindung eine Stärkesynthase II zu verstehen, deren codierende Nukleotidsequenz natürlicherweise nicht in der Weizen-Pflanze(nzelle) vorkommt oder die nicht unter der Kontrolle ihres eigenen Promotors steht und/oder deren codierende DNA-Sequenz beispielsweise mittels gentechnischer Methoden, wie z.B. Transformation der Zelle, in die Weizen-Zelle eingeführt wird. Vorzugsweise stammt die heterologe Stärkesynthase aus einer anderen Pflanzenart als die transformierte Weizen-Pflanzenzelle bzw. Weizen-Pflanze. Besonders bevorzugt stammt die codierende DNA-Sequenz der heterologen Stärkesynthase II aus einer anderen Pflanzengattung als die transformierte Weizen-Pflanzenzelle bzw. -Pflanze.

Unter dem Begriff "Pflanzengattung" ist im Zusammenhang mit der vorliegenden Erfindung eine hierarchische Stufe der biologischen Systematik zu verstehen. Eine Gattung enthält eine oder mehrere Arten. Ein Beispiel einer Gattung ist *Triticum* L. (Weizen). Alle Arten innerhalb einer Gattung haben immer einen zweiteiligen (binominalen) Namen, der neben der Gattungsbezeichnung noch ein Art-Epipheton enthält. *Triticum aestivum* L. (der Weichweizen) ist danach eine Art der Gattung *Triticum.*

In einer besonders bevorzugten Ausführungsform wird im Zusammenhang mit der vorliegenden Erfindung eine heterologe Stärkesynthase II der Gattung *Oryza,* vorzugsweise der Art *Oryza sativa* verwendet. Weiterhin bevorzugt ist die Stärkesynthase II, die von Jiang et al. als OsSSII-3 bezeichnet wird (Jiang H., Dian W., Liu F., Wu P. (2004). Molecular cloning and expression analysis of three genes encoding starch synthase II in rice. Planta, 218, 1062-1070; GenBank Acc. No. AF419099.1 = SEQ ID No. 1). Besonders bevorzugt ist eine Stärkesynthase II, die gegenüber der OsSSII-3 (SEQ ID No. 2) an Position 737 der unter SEQ ID No.2 angegebenen Aminosäuresequenz einen Aminosäureaustausch aufweist (Valin an Stelle eines Methionins) und deren vollständige Aminosäuresequenz unter SEQ ID No. 4 angegeben ist.

Vorzugsweise weist die Stärkesynthase II die unter SEQ ID No.1 angegebene Nukleotidsequenz auf. Besonders bevorzugt weist die Stärkesynthase II die unter SEQ ID No.3 angegebene Nukleotidsequenz auf. SEQ ID No.3 unterscheidet sich von SEQ ID No. 1 durch einen Nukleotidaustausch an Position 2209 der unter SEQ ID No. 1 angegebenen Nukleotidsequenz (Adenin ausgetauscht gegen Guanin). Besonders bevorzugt ist die Stärkesynthase II mit der unter SEQ ID No.3 angegebenen Nukleotidsequenz.

In einer weiteren bevorzugten Ausführungsform wird im Zusammenhang mit der vorliegenden Erfindung eine synthetische Stärkesynthase II verwendet. Besonders bevorzugt ist eine synthetische Variante der Stärkesynthase II, die die unter SEQ ID No. 5 angegebene Nukleotidsequenz aufweist. Im Unterschied zur natürlichen Stärkesynthase II aus Weizen, die unter SEQ ID No. 6 angegeben ist und die das gleiche Protein codiert wie SEQ ID No. 5, wurde die synthetische Sequenz der SEQ ID No. 5 durch Nukleotidaustausche optimiert, um eine möglichst starke Erhöhung der SSII-Aktivität gegenüber Weizen-Wildtyp-Pflanzen zu erzielen.

Die vorliegende Erfindung betrifft somit auch ein Nucleinsäuremolekül codierend ein Protein mit der enzymatischen Aktivität einer Stärkesynthase II, wobei das Nucleinsäuremolekül ausgewählt ist aus der Gruppe bestehend aus

(a) einem Nucleinsäuremolekül, das ein Protein codiert, das die unter Seq ID NO. 4 angegebene Aminosäuresequenz umfasst;

(b) einem Nucleinsäuremolekül, das die unter Seq ID No. 3 dargestellte Nucleotidsequenz oder eine hierzu korrespondierende Ribonucleotidsequenz;

(c) einem Nucleinsäuremolekül, das die unter Seq ID No.5 dargestellte Nucleotidsequenz oder eine hierzu korrespondierende Ribonucleotidsequenz;

(d) einem Nucleinsäuremolekül, und dessen Nucleotidsequenz aufgrund der Degeneration des genetischen Codes von der Sequenz eines unter (a) oder (b) genannten Nucleinsäuremoleküls abweicht.

In einer weiteren Ausführungsform betrifft die vorliegende Erfindung Vektoren, insbesondere Plasmide, Cosmide, Viren, Bacteriophagen und andere in der Gentechnik gängige Vektoren, die die erfindungsgemäßen Nucleinsäuremoleküle enthalten.

In einer weiteren Ausführungsform sind die in den Vektoren enthaltenen erfindungsgemäßen Nucleinsäuremoleküle verknüpft mit regulatorischen Sequenzen, die die Expression in prokaryontischen oder eukaryontischen Zellen initiieren. Der Begriff "Expression" kann dabei Transkription als auch Transkription und Translation bedeuten. Vorzugsweise liegen die erfindungsgemäßen Nucleinsäuremoleküle dabei zu den regulatorischen Sequenzen in "sense"-Orientierung vor.

Ein weiterer Gegenstand der vorliegenden Erfindung ist eine Wirtszelle, insbesondere eine prokaryontische oder eukaryontische Zelle (wobei menschliche Zellen von diesem Begriff nicht umfasst sein sollen), die genetisch modifiziert ist mit einem erfindungsgemäßen Nucleinsäuremolekül und/oder mit einem erfindungsgemäßen Vektor, sowie Zellen, die von derartigen Wirtszellen abstammen und die die erfindungsgemäße genetische Modifikation enthalten.

In einer weiteren Ausführungsform betrifft die Erfindung Wirtszellen, insbesondere prokaryontische oder eukaryontische Zellen (wobei menschliche Zellen von diesem Begriff nicht umfasst sein sollen), die mit einem erfindungsgemäßen Nucleinsäuremolekül oder einem erfindungsgemäßen Vektor transformiert wurden, sowie Wirtszellen, die von derartigen Wirtszellen abstammen und die beschriebenen erfindungsgemäßen Nucleinsäuremoleküle oder Vektoren enthalten.

Die Wirtszellen können Bakterien- (z.B. *E. coli*, Bakterien der Gattung *Agrobacterium* insbesondere *Agrobac-*

*terium tumefaciens* oder *Agrobacterium rhizogenes*) oder Pilzzellen (z.B. Hefe, insbesondere *S. cerevisiae, Agaricus*, insbesondere *Agaricus bisporus, Aspergillus, Trichoderma*), sowie pflanzliche oder tierische Zellen sein. Der Begriff "transformiert" bedeutet dabei, dass die erfindungsgemäßen Zellen mit einem erfindungsgemäßen Nucleinsäuremolekül genetisch modifiziert sind insofern, als sie zusätzlich zu ihrem natürlichen Genom mindestens ein erfindungsgemäßes Nucleinsäuremolekül enthalten. Dieses kann in der Zelle frei, gegebenenfalls als selbstreplizierendes Molekül, vorliegen oder es kann stabil in das Genom der Wirtszelle integriert vorliegen.

Vorzugsweise sind die Wirtszellen Mikroorganismen. Darunter werden im Rahmen der vorliegenden Anmeldung alle Bakterien und alle Protisten (z. B. Pilze, insbesondere Hefen und Algen) verstanden, so wie sie z. B. in Schlegel "Allgemeine Mikrobiologie" (Georg Thieme Verlag (1985), 1-2) definiert sind.

Bevorzugt sind die erfindungsgemäßen Wirtszellen Pflanzenzellen. Dabei kann es sich prinzipiell um Pflanzenzellen aus jeder beliebigen Pflanzenspezies handeln, d.h. sowohl monokotyle als auch dikotyle Pflanzen. Bevorzugt handelt es sich um Pflanzenzellen aus landwirtschaftlichen Nutzpflanzen, d.h. aus Pflanzen, die vom Menschen kultiviert werden für Zwecke der Ernährung oder für technische, insbesondere industrielle Zwecke. Vorzugsweise betrifft die Erfindung Pflanzenzellen und Pflanzen aus Stärke speichernden Pflanzen (Mais, Reis, Weizen, Roggen, Hafer, Gerste, Maniok, Kartoffel, Sago, Mungbohne, Erbse oder Sorghum), bevorzugt Pflanzenzellen aus Pflanzen der (systematischen) Familie *Poacea.* Besonders bevorzugt sind Pflanzenzellen aus Weizenpflanzen.

Unter dem Begriff "Identität" soll im Zusammenhang mit der vorliegenden Erfindung die Anzahl der übereinstimmenden Aminosäuren/Nucleotide (Identität) mit anderen Proteinen/Nucleinsäuren, ausgedrückt in Prozent verstanden werden. Bevorzugt wird die Identität eines Protein mit der Aktivität einer Stärkesynthase II durch Vergleich mit der unter SEQ ID NO 4 angegebenen Aminosäuresequenz bzw. die Identität eines Nucleinsäuremoleküls codierend ein Protein mit der Aktivität einer Stärkesynthase II durch Vergleich mit der unter SEQ ID NO 3 angegebenen Nucleinsäuresequenz mit anderen Proteinen/Nucleinsäuren durch Computerprogramme ermittelt. Weisen Sequenzen, die miteinander verglichen werden, unterschiedliche Längen auf, ist die Identität so zu ermitteln, dass die Anzahl an Aminosäuren/Nucleotiden, welche die kürzere Sequenz mit der längeren Sequenz gemeinsam hat, den prozentualen Anteil der Identität bestimmt. Vorzugsweise wird die Identität mittels des bekannten und der Öffentlichkeit zur Verfügung stehenden Computerprogramms ClustalW (Thompson et al., Nucleic Acids Research 22 (1994), 4673-4680) ermittelt. ClustalW wird öffentlich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Internetseiten, u.a. beim IGBMC (Institut de Génétique et de Biologie Moléculaire et Cellulaire, B.P.163, 67404 IIlkirch Cedex, France; ftp://ftp-igbmc.u-strasbg.fr/pub/) und beim EBI (ftp://ftp.ebi.ac.uk/pub/software/) sowie bei allen gespiegelten Internetseiten des EBI (European Bioinformatics Institute, Wellcome Trust Genome Campus, Hinxton, Cambridge CB10 1SD, UK) heruntergeladen werden.

Vorzugsweise wird das ClustalW Computerprogramm der Version 1.8 benutzt, um die Identität zwischen im Rahmen der vorliegenden Erfindung beschriebenen Proteinen und anderen Proteinen zu bestimmen. Dabei sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP.

Vorzugsweise wird das ClustalW Computerprogramm der Version 1.8 benutzt, um die Identität zwischen z.B. der Nucleotidsequenz der im Rahmen der vorliegenden Erfindung beschriebenen Nucleinsäuremoleküle und der Nucleotidsequenz von anderen Nucleinsäuremolekülen zu bestimmen. Dabei sind folgende Parameter einzustellen: KTUPLE=2, TOPDIAGS=4, PAIRGAP=5, DNAMATRIX:IUB, GAPOPEN=10, GAPEXT=5, MAXDIV=40, TRANSITIONS: unweighted.

In einer weiteren Ausführungsform der vorliegenden Erfindung ist die erfindungsgemäße Weizen- Pflanze (nzelle), die die erfindungsgemäße Weizenstärke synthetisiert, genetisch modifiziert, wobei die genetische Modifikation zu einer Erhöhung der Aktivität einer Stärkesynthase II führt im Vergleich zu entsprechenden nicht genetisch modifizierten Weizen-Wildtyp-Pflanzenzellen bzw. Weizen-Wildtyp-Pflanzen.

Die genetische Modifikation kann dabei jede genetische Modifikation sein, die zu einer Erhöhung der Aktivität einer Stärkesynthase II führt im Vergleich zu entsprechenden nicht genetisch modifizierten Weizen-Wildtyp-Pflanzenzellen oder Weizen-Wildtyp-Pflanzen.

Der Begriff "Weizen-Wildtyp-Pflanzenzelle" bedeutet im Zusammenhang mit der vorliegenden Erfindung, dass es sich um Weizen-Pflanzenzellen handelt, die als Ausgangsmaterial für die Herstellung der erfindungsgemäßen Weizen-Pflanzenzellen dienten, die die erfindungsgemäße Stärke synthetisieren.

Der Begriff "Weizen-Wildtyp-Pflanze" bedeutet im Zusammenhang mit der vorliegenden Erfindung, dass es sich um Weizen-Pflanzen handelt, die als Ausgangsmaterial für die Herstellung der erfindungsgemäßen Weizen-Pflanzen dienten, die die erfindungsgemäße Stärke synthetisieren.

Vorzugsweise bezieht sich der Begriff "Weizen-Wildtyp-Pflanze" auf die Varietät Fielder, die öffentlich verfügbar ist, beispielsweise beim Alberta Stock Seed Distribution Committee, Alberta Agriculture and Rural Development, J. G. O'Donoghue Building 203, 7000 - 113 St. Edmonton, AB T6H 5T6, Kanada.

Der Begriff "entsprechend" bedeutet im Zusammenhang mit der vorliegenden Erfindung, dass beim Vergleich von mehreren Gegenständen die betreffenden Gegenstände, die miteinander verglichen werden, unter gleichen Bedingungen gehalten werden. Im Zusammenhang mit der vorliegenden Erfindung bedeutet der Begriff "entsprechend" im Zusammenhang mit Weizen-Wildtyp-Pflanzenzellen oder Weizen-Wildtyp-Pflanzen, dass die Pflanzenzellen oder Pflanzen, die miteinander verglichen werden, unter gleichen Kulturbedingungen aufgezogen wurden und dass sie ein gleiches (Kultur)-Alter aufweisen.

Der Begriff "Erhöhung der Aktivität einer Stärkesynthase II" bedeutet im Rahmen der vorliegenden Erfindung eine Erhöhung der Expression endogener Gene, die Proteine mit der Aktivität einer Stärkesynthase II codieren und/oder eine Erhöhung der Menge an Proteinen mit der Aktivität einer Stärkesynthase II in den Weizen-Pflanzen(zellen) und/oder vorzugsweise eine Erhöhung der enzymatischen Aktivität von Proteinen mit der Aktivität einer Stärkesynthase II in den erfindungsgemäßen Weizen-Pflanzen(zellen).

Die Erhöhung der Expression kann beispielsweise bestimmt werden durch Messung der Menge an Transkripten, die Proteine mit der Aktivität einer Stärkesynthase II codieren. Die Bestimmung kann z.B. durch Northern-Blot-Analyse oder RT-PCR erfolgen.

Die Menge der Aktivität eines Proteins mit der Aktivität einer Stärkesynthase II kann z.B. wie in der Literatur beschrieben (Nishi et al., 2001, Plant Physiology 127, 459-472) bestimmt werden. Eine im Zusammenhang mit der vorliegenden Erfindung bevorzugte Methode zur Bestimmung der Menge an Aktivität eines Proteins mit der Aktivität einer Stärkesynthase II ist weiter unten beschrieben ("Bestimmung der SSII-Aktivität mittels Aktivitätsgel").

Vorzugsweise weisen die Weizen-Pflanzen(zellen), die die erfindungsgemäße Stärke synthetisieren, eine enzymatische Aktivität der Stärkesynthase II auf, die mindestens 2 fach, bevorzugt 3 - 10 fach erhöht ist im Vergleich zu entsprechenden genetisch nicht modifizierten Weizen-Wildtyp-Pflanzenzellen bzw. Weizen-Wildtyp-Pflanzen.

In einer weiteren Ausführungsform der vorliegenden Erfindung besteht die genetische Modifikation der erfindungsgemäßen Weizen-Pflanze(nzelle) in der Einführung mindestens eines fremden Nucleinsäuremoleküls in das Genom der Pflanzenzelle bzw. in das Genom der Pflanze.

In diesem Zusammenhang bedeutet der Begriff "genetische Modifikation" das Einführen mindestens eines fremden Nucleinsäuremoleküls in das Genom einer Weizen-Pflanze(nzelle), wobei besagtes Einführen dieses Moleküls zur Erhöhung der Aktivität eines Proteins mit der Aktivität einer Stärkesynthase II führt.

Durch Einführung eines fremden Nucleinsäuremoleküls sind die erfindungsgemäßen Weizen-Pflanzen(zellen) in ihrer genetischen Information verändert. Das Vorhandensein oder die Expression mindestens eines fremden Nucleinsäuremoleküls führt zu einer phänotypischen Veränderung. "Phänotypische Veränderung" bedeutet dabei vorzugsweise eine messbare Veränderung einer oder mehrerer Funktionen der Zellen. Beispielsweise zeigen die genetisch modifizierten Weizen-Pflanzen(zellen) aufgrund des Vorhandenseins oder bei Expression eingeführter fremder Nucleinsäuremoleküle eine Erhöhung der Aktivität eines Proteins mit der Aktivität einer Stärkesynthase II und/oder synthetisieren eine erfindungsgemäße Weizen-Stärke.

Unter dem Begriff "fremdes Nukleinsäuremolekül" versteht man im Zusammenhang mit der vorliegenden Erfindung ein solches Molekül, das entweder natürlicherweise in entsprechenden Weizen-Wildtyp-Pflanzen(zellen) nicht vorkommt oder das in der konkreten räumlichen Anordnung nicht natürlicherweise in Wildtyp-Pflanzen(zellen) vorkommt oder das an einem Ort im Genom der Wildtyp-Pflanzenzelle lokalisiert ist, an dem es natürlicherweise nicht vorkommt. Prinzipiell kann ein fremdes Nucleinsäuremolekül jedes beliebige Nucleinsäuremolekül sein, das in der Pflanzenzelle oder Pflanze eine Erhöhung der Aktivität eines Proteins mit der Aktivität einer Stärkesynthase II bewirkt.

Bevorzugt ist das fremde Nukleinsäuremolekül ein rekombinantes Nukleinsäuremolekül, das aus verschiedenen Elementen besteht, deren Kombination oder spezifische räumliche Anordnung natürlicherweise in pflanzlichen Zellen nicht auftritt.

Unter dem Begriff "rekombinantes Nukleinsäuremolekül" soll im Zusammenhang mit der vorliegenden Erfindung ein Nukleinsäuremolekül verstanden werden, welches unterschiedliche Nucleinsäuremoleküle aufweist, die natürlicherweise nicht in einer Kombination vorliegen, wie sie in einem rekombinanten Nucleinsäuremolekül vorliegen. So weisen die rekombinanten Nucleinsäuremoleküle beispielsweise neben Nucleinsäuremolekülen, die ein Protein mit der Aktivität einer Stärkesynthase II codieren (z.B. genomische Nukleinsäuremoleküle oder cDNAs), zusätzliche Nucleinsäuresequenzen auf, welche natürlicherweise nicht in Kombination mit diesen Nucleinsäuremolekülen vorliegen. Das rekombinante Nukleinsäuremolekül weist beispielsweise regulatorische Sequenzen auf (z.B. Promotoren, Terminationssignale, Enhancer), vorzugsweise regulatorische Sequenzen, die heterolog sind in Bezug auf das Nukleinsäuremolekül, das die Stärkesynthase II codiert. Heterolog bedeutet in diesem Zusammenhang, dass es sich bei der regulatorischen Sequenz nicht um die eigene endogene regulatorische Sequenz des eingesetzten Stärkesynthase II Gens handelt. Weiterhin bevorzugt sind regulatorische Sequenzen, die in pflanzlichem Gewebe aktiv sind.

Geeignete Promotoren sind konstitutive Promotoren, wie z.B. der Promotor der 35S RNA des Cauliflower Mosaic Virus (Odell et al., 1985, Nature, 313, 810-812), der Ubiquitin-Promotor aus Mais (Christensen et al., Plant Mol. Biol. 18, (1992), 675-689), der Ubiquitin-Promotor aus Reis (Liu et al., Plant Science 165, (2003), der Reis Actin Promoter (Zhang, et al., Plant Cell 3:1150-1160, 1991), der Cassava Vein Mosaic Virus (CVMV) Promotor (Verdaguer et. al., Plant Mol. Biol. 31: 1129-1139), der Mais Histon H3C4 Promotor (US 6,750,378) oder der *Cestrum* YLCV Promotor (Yellow Leaf Curling Virus; WO 01 73087; Stavolone et al., 2003, Plant Mol. Biol. 53, 703-713).
Besonders bevorzugt handelt es sich um gewebespezifische regulatorische Sequenzen, die in Weizengewebe, vorzugsweise im Endosperm von Weizenpflanzen aktiv sind. Weitere endosperm-spezifische Promotoren sind der Promotor des10 kD Zein-Gens aus Mais (Kirihara et al. (1988) Gene 71: 359-370), des 15 kD Zein-Gens aus Mais (Hoffmann et al. (1987) EMBO J. 6: 3213-3221; Schernthaner et al. (1988) EMBO J. 7: 1249-1253; Williamson et al. (1988) Plant Physiol. 88: 1002-1007), des 27 kd Zein-Gens aus Mais (Prat et al. (1987) Gene 52: 51-49; Gallardo et al. (1988) Plant Sci. 54: 211-281), des 19 kD Zein-Gens aus Mais (Marks et al. (1985) J. Biol. Chem. 260: 16451-16459). Die relativen transkriptionalen Aktivitäten dieser Promotoren in Mais sind bei Kodrzyck et al., (1989), Plant Cell 1, 105-114) beschrieben.

Andere, in Verbindung mit der vorliegenden Erfindung denkbare Promotoren sind der Promotor des Sucrose Synthase Gens (Yang, N.-S. and Russel, D. (1990) Proc. Natl. Acad Sci 87: 4144-4148), des waxy-Gens (Unger et al. (1991) Plant Physiol. 96: 124), des sh 2-Gens (Bhave et al. (1990) Plant Cell 2: 581-588, des bt 2-Gens (Bae et al. (1990) Maydica 35: 317-322). Ferner der HMG-Promotor (auch als Weizen Glutenin HMWG-Promotor bezeichnet) aus Weizen (Colot et al., EMBO J. 6, (1987, 3559-3564; Clarke and Appels, Genome 41, (1998), 865-871), der USP-Promotor, der Phaseolinpromotor, Promotoren von Zein-Genen aus Mais (Pedersen et al., Cell 29 (1982), 1015-1026; Quatroccio et al., Plant Mol. Biol. 15 (1990), 81-93), der Glutelin-Promotor (Leisy et al., Plant Mol. Biol. 14 (1990), 41-50; Zheng et al., Plant J. 4 (1993), 357-366; Yoshihara et al., FEBS Lett. 383 (1996), 213-218), der Globulin Promotor (Nakase et al., 1996, Gene 170(2), 223-226) oder der Prolamin Promotor (Qu und Takaiwa, 2004, Plant Biotechnology Journal 2(2), 113-125).

Vorzugsweise werden Promotoren verwendet, die spezifisch sind für stärkespeichernde Organe, wie z.B. endosperm-spezifische Promotoren, wie beispielsweise der Glutelin-Promotor (Leisy et al., Plant Mol. Biol. 14, (1990), 41-50; Zheng et al., Plant J. 4, (1993), 357-366; Yoshihara et al., FEBS Lett. 383, (1996), 213-218), der HMW-Promotor aus Weizen (Anderson, Theoretical and Applied Genetics 96, (1998), 568-576, Thomas, Plant Cell 2 (12), (1990), 1171-1180), der USP-Promotor, der Phaseolinpromotor (Sengupta-Gopalan, Proc. Natl. Acad. Sci. USA 82 (1985), 3320-3324, Bustos, Plant Cell 1 (9) (1989), 839-853) oder die karyopsenspezifischen Promotoren der GBSSI (granule bound starch synthase I) (DE10041861.9) und der SSII (soluble starch synthase II) aus Weizen (DE10032379.0).

Es können auch Intronsequenzen zwischen dem Promotor und der codierenden Region vorhanden sein. Solche Intronsequenzen können zur Stabilität der Expression und zu einer erhöhten Expression in Pflanzen führen (Callis et al., 1987, Genes Devel. 1, 1183-1200; Luehrsen, and Walbot, 1991, Mol. Gen. Genet. 225, 81-93; Rethmeier, et al., 1997; Plant Journal. 12(4):895-899; Rose and Beliakoff, 2000, Plant Physiol. 122 (2), 535-542; Vasil et al., 1989, Plant Physiol. 91, 1575-1579; XU et al., 2003, Science in China Series C Vol.46 No.6, 561-569). Geeignete Intronsequenzen sind beispielsweise das erste Intron des sh1-Gens aus Mais (Maas et al. (1991) Plant. Mol. Biol. 16: 199-207, das erste Intron des Poly-Ubiquitin Gens 1 aus Mais, das erste Intron des EPSPS Gens aus Reis oder eines der beiden ersten Introns des PAT1 Gens aus *Arabidopsis*, ferner Introns des Adh-1 oder Bz-1 Gens aus Mais (Callis et al. (1987) Genes Dev. 1: 1183-1200), das Intron 3 des Mais Actin Gens (Luehrsen, K. R. and Walbot, V. (1991) Mol. Gen. Genet. 225: 81-93) oder des Adh1 Introns 6 (Oard et al. (1989) Plant Cell Rep 8: 156-160).

Methoden zur Erzeugung rekombinanter Nucleinsäuremoleküle sind dem Fachmann bekannt und umfassen gentechnische Methoden, wie z.B. die Verbindung von Nucleinsäuremolekülen durch Ligation, genetische Rekombination oder die Neusynthese von Nucleinsäuremolekülen (siehe z.B. Sambrook et al., Molecular Cloning, A Laboratory Manual, 3rd edition (2001) Cold Spring Harbour Laboratory Press, Cold Spring Harbour, NY. ISBN: 0879695773, Ausubel et al., Short Protocols in Molecular Biology, John Wiley & Sons; 5th edition ( 2002), ISBN: 0471250929).

Unter dem Begriff "Genom" soll im Zusammenhang mit der vorliegenden Erfindung die Gesamtheit des in einer pflanzlichen Zelle vorliegenden Erbmaterials verstanden werden. Dem Fachmann ist bekannt, dass neben dem Zellkern auch andere Kompartimente (z.B. Plastiden, Mitochondrien) Erbmaterial enthalten.

Ferner sind Gegenstand der Erfindung genetisch modifizierte Weizen-Pflanzen, die die erfindungsgemäßen Weizen-Pflanzenzellen enthalten. Derartige Weizen-Pflanzen können durch Regeneration aus erfindungsgemäßen Pflanzenzellen erzeugt werden.

Die vorliegende Erfindung betrifft auch Vermehrungsmaterial erfindungsgemäßer Weizen-Pflanzen. Der Begriff "Vermehrungsmaterial" umfasst dabei jene Bestandteile der Pflanze, die geeignet sind zur Erzeugung von Nachkommen auf vegetativem oder sexuellem Weg. Für die vegetative Vermehrung eignen sich beispielsweise Calluskulturen. Besonders bevorzugt handelt es sich bei dem Vermehrungsmaterial um endospermhaltige Weizen-Samen (Körner).

Weiterhin betrifft die vorliegende Erfindung auch ein Verfahren zur Herstellung einer erfindungsgemäßen Weizen- Pflanze, worin

a) eine Weizen-Pflanzenzelle genetisch modifiziert wird, wobei die genetische Modifikation zu einer Erhöhung der Aktivität einer Stärkesynthase II führt im Vergleich zu entsprechenden nicht genetisch modifizierten Weizen-Wildtyp-Pflanzenzellen;
b) aus Weizen-Pflanzenzellen von Schritt a) eine Weizen-Pflanze regeneriert wird; und

c) gegebenenfalls weitere Weizen-Pflanzen mit Hilfe der Weizen-Pflanzen nach Schritt b) erzeugt werden.

Für die laut Schritt a) in die Weizen-Pflanzenzelle eingeführte genetische Modifikation gilt, was bereits im Zusammenhang mit der Beschreibung der erfindungsgemäßen Weizen-Pflanzen(zellen) erläutert wurde.

Die Regeneration der Weizen-Pflanzen gemäß Schritt b) kann nach dem Fachmann bekannten Methoden erfolgen (z.B. beschrieben in "Plant Cell Culture Protocols", 1999, edt. by R.D. Hall, Humana Press, ISBN 0-89603-549-2).

Die Erzeugung weiterer Pflanzen gemäß Schritt c) des erfindungsgemäßen Verfahrens kann z.B. erfolgen durch vegetative Vermehrung (beispielsweise über Calluskulturen) oder durch sexuelle Vermehrung. Die sexuelle Vermehrung findet dabei vorzugsweise kontrolliert statt, d.h. es werden ausgewählte Weizen-Pflanzen mit bestimmten Eigenschaften miteinander gekreuzt und vermehrt. Die Auswahl erfolgt dabei bevorzugt in der Weise, dass die Weizen-Pflanzen, die nach Schritt c) erhalten werden, die genetische Modifikation, die in Schritt a) eingeführt wurde, aufweisen.

In einer weiteren Ausführungsform betrifft die vorliegende Erfindung Weizenmehl enthaltend die erfindungsgemäße - vorzugsweise granuläre - Weizenstärke.

Stärkespeichernde Teile von Pflanzen können zu Mehlen verarbeitet werden. Zur Herstellung von Weizenmehlen werden die endospermhaltigen Weizenkörner gemahlen und gesiebt. Stärke ist ein Hauptbestandteil des Endosperms. Die erfindungsgemäße Weizenstärke ist neben Proteinen und Lipiden der wesentliche Bestandteil des erfindungsgemäßen Weizenmehls. Die Eigenschaften der erfindungsgemäßen Weizenmehle werden daher stark durch die in dem Weizenmehl enthaltene erfindungsgemäße Weizenstärke beeinflusst.

Unter dem Begriff "Weizenmehl" soll im Zusammenhang mit der vorliegenden Erfindung ein durch Mahlen von Weizenkörnern erhaltenes Pulver verstanden werden, wobei die Weizenkörner aus Weizen-Pflanzenzellen bestehen, die eine heterologe Stärkesynthase II exprimieren. Gegebenenfalls werden die Weizenkörner vor dem Mahlen getrocknet und nach dem Mahlen zerkleinert und/oder gesiebt.

In einer weiteren Ausführungsform betrifft die vorliegende Erfindung Weizenmehl, dessen Stärkekomponente einen Amylosegehalt zwischen 15.0 Gew.-% bis 30.0 Gew.-%, vorzugsweise zwischen 18.0 Gew.-% bis 30.0 Gew.-% und besonders bevorzugt zwischen 20.0 Gew.-% bis 30.0 Gew.-% und das Weizenmehl einen Gehalt an resistenter Stärke des Mehles (RS Mehl) von mehr als 5.0 Gew.-%, vorzugsweise zwischen 5.0 Gew.-% bis 30.0 Gew.-%, besonders bevorzugt zwischen 6.0 Gew.-% bis 20 Gew.-% aufweist.

Im Zusammenhang mit der vorliegenden Erfindung wird der Amylosegehalt der Stärkekomponente des erfindungsgemäßen Weizenmehles mit Hilfe der weiter unten beschriebenen DSC-Methode "Bestimmung des Gehaltes an apparenter Amlyose" ermittelt. Die DSC-Methode kann dabei wahlweise an einer Weizenstärkeprobe (eine Methode zur Isolierung von Weizenstärke aus Weizenmehl ist weiter unten beschrieben unter "Herstellung von Weizenmehlen und nachfolgende Extraktion von Weizenstärke") oder an einer Weizenmehlprobe durchgeführt werden.
Vorzugsweise wird die DSC-Methode an einer Weizenmehlprobe durchgeführt. Der Amylosegehalt der Stärkekomponente des Weizenmehles ergibt sich dann unter Annahme eines x-%-igen Gewichtsanteils der Stärke am Weizenmehl (Hung et al., Trends in Food Science & Technology 17, (2006), 448-456) rechnerisch gemäß folgender Formel:

$$\text{Amylosegehalt der Stärke} = \frac{\text{Amylosegehalt (Mehl)} \times 100}{X}$$

Der Gewichtsanteil X der Stärke an der Mehlprobe wird vorzugsweise wie in Methode 8 (Bestimmung des Anteiles an schnell verdaubarer und resistenter Stärke in Weizenmehlen/-stärken) beschrieben bestimmt.

Die Bestimmung des RS-Gehaltes des erfindungsgemäßen Weizenmehles (RS Mehl) erfolgt im Zusammenhang mit der vorliegenden Erfindung vorzugsweise über die bereits oben erwähnte Methode von Englyst et al. (Europ. J. of Clinical Nutrition 46 (Suppl. 2), (1992), S 33-50, siehe insbesondere folgende Abschnitte aus Englyst et al., Seite S35-S36: "Reagents, Apparatus, Spectrophotometer"; Seite S36-S37, Absatz ,,Measurement of free glucose (FG)"; Seite S38, Absatz ,,Measurement of RDS and SDS"). Als "RS-Gehalt des Weizenmehles" bezeichnet man im Zusammenhang mit der vorliegenden Erfindung den Anteil der eingewogenen Mehlprobe (Frischgewicht), der in der Methode von Englyst et al. nach 2 Stunden nicht als Glukose freigesetzt wird. Er ergibt sich demnach gemäß folgender Formel:

$$RS\ Mehl\ in\ \% = Gesamtglucose\ in\ \%\ Frischgewicht - freigesetzte\ Glukose\ nach\ 2\ h\ in\ \%\ Frischgewicht$$

In einer weiteren bevorzugten Ausführungsform der vorliegenden Erfindung bezieht sich die Angabe des Amylosegehaltes nicht auf die Stärkekomponente des Weizenmehles, sondern auf den Amylosegehalt des erfindungsgemäßen Weizenmehles (Amylosegehalt Mehl). Der Amylosegehalt der erfindungsgemäßen Weizenmehle (Amylosegehalt Mehl) beträgt zwischen 10.0 Gew.-% bis 22 Gew.-%, vorzugsweise zwischen 11 Gew.-% bis 21 Gew.-% und besonders bevorzugt zwischen 12 Gew.-% bis 20.0 Gew.-%.

Im Zusammenhang mit der vorliegenden Erfindung wird der Amylosegehalt des erfindungsgemäßen Weizenmehles (Amylosegehalt Mehl) mit Hilfe der weiter unten beschriebenen DSC-Methode "Bestimmung des Gehaltes an apparenter Amlyose" an einer Weizenmehlprobe ermittelt.

In einer weiteren Ausführungsform weisen die erfindungsgemäßen Weizenmehle einen Gehalt an schnell verdaulichem Mehl (RDS Mehl) bezogen auf die Mehlmenge (Frischgewicht) zwischen 10 Gew.-% bis 22 Gew.-%, vorzugsweise zwischen 11 Gew.-% bis 21 Gew.-%, besonders bevorzugt zwischen 12 Gew.-% bis 20 Gew.-% auf.

Im Zusammenhang mit der vorliegenden Erfindung soll unter "dem Gehalt an schnell verdaulichem Mehl (=RDS Mehl)" der Anteil eines Weizenmehls verstanden werden, der in der oben genannten Methode von Englyst et al. zur Bestimmung des RS-Gehaltes nach 20 Minuten als Glukose freigesetzt wird. Die Angabe in Gewichtsprozent bezieht sich hierbei auf das Frischgewicht der Mehlprobe. Demnach gilt im Zusammenhang mit der vorliegenden Erfindung:

$$RDS\ Mehl\ in\ \% = freigesetzte\ Glukose\ nach\ 20\ Minuten\ in\ \%\ Frischgewicht$$

In einer weiteren Ausführungsform betrifft die vorliegende Erfindung Weizenmehl, dessen Stärkekomponente einen Amylosegehalt zwischen 15.0 Gew.-% bis 30.0 Gew.-%, vorzugsweise zwischen 18.0 Gew.-% bis 29.5 Gew.-% und besonders bevorzugt zwischen 20.0 Gew.-% bis 29.5 Gew.-% und das Weizenmehl

a) einen Gehalt an resistenter Stärke des Mehles (RS Mehl) von mehr als 5.0 Gew.-%, vorzugsweise zwischen 6.0 Gew.-% bis 30.0 Gew.-%; und

b) einen Gehalt an schnell verdaulichem Mehl (RDS Mehl) bezogen auf die Mehlmenge (Trockengewicht) zwischen 10 Gew.-% bis 22 Gew.-%, vorzugsweise zwischen 11 Gew.-% bis 21 Gew.-%, besonders bevorzugt zwischen 12 Gew.-% bis 20 Gew.-% aufweist.

In einer weiteren Ausführungsform betrifft die vorliegende Erfindung Weizenmehl, dessen Stärkekomponente einen Amylosegehalt zwischen 15.0 Gew.-% bis 30.0 Gew.-%, vorzugsweise zwischen 18.0 Gew.-% bis 30.0 Gew.-% und besonders bevorzugt zwischen 20.0 Gew.-% bis 30.0 Gew.-% und dessen Stärkekomponente einen Gehalt an resistenter Stärke (RS Stärke) von mehr als 5.0 Gew.-%, vorzugsweise zwischen 6 Gew.-% bis 30.0 Gew.-% aufweist.

Die Bestimmung des RS-Gehaltes der Stärkekomponente (RS Stärke) des erfindungsgemäßen Weizenmehles erfolgt im Zusammenhang mit der vorliegenden Erfindung wie oben für die erfindungsgemäße Weizenstärke beschrieben.

In einer weiteren Ausführungsform weist die Stärkekomponente des erfindungsgemäßen Weizenmehls einen Gehalt an schnell verdaulicher Stärke (RDS Stärke) zwischen 10.0 Gew.-% bis 38 Gew.-%, vorzugsweise zwischen 15.0 Gew.-% bis 35.0 Gew.-%, besonders bevorzugt zwischen 20.0 Gew.-% bis 33 Gew.-% auf.

Die Bestimmung des RDS-Gehaltes der Stärkekomponente (RDS Stärke) des erfindungsgemäßen Weizenmehles erfolgt im Zusammenhang mit der vorliegenden Erfindung wie oben für die erfindungsgemäße Weizenstärke beschrieben.

Die erfindungsgemäßen Weizenstärken/Weizenmehle weisen den Vorteil eines verringerten Anteils an schnell verdauliche(m/r) Mehl/Stärke auf, was besonders vorteilhaft ist, da eine schnelle Freisetzung größerer Mengen Glukose und dessen Absorption über das Dünndarmepithel zu einer abrupten Zunahme des Blutzuckerspiegels führt. In dessen Folge kommt es zu einer Ausschüttung von Insulin (Insulin Antwort). Der fortwährende Konsum von Lebensmitteln mit einer hohen glykämischen Ladung, und die damit verbundene Insulinausschüttung, steht in Verdacht ein Risikofaktor bei der Entstehung von Krankheiten wie Bluthochdruck, Übergewicht, Herzkrankheiten und Diabetes Typ II zu sein.

In einer weiteren Ausführungsform betrifft die vorliegende Erfindung Weizenmehl, dessen Stärkekomponente einen Amylosegehalt zwischen 15.0 Gew.-% bis 30.0 Gew.-%, vorzugsweise zwischen 18.0 Gew.-% bis 29.5 Gew.-% und besonders bevorzugt zwischen 20.0 Gew.-% bis 29.5 Gew.-% und dessen Stärkekomponente

a) einen Gehalt an resistenter Stärke (RS Stärke) von mehr als 5.0 Gew.-%, vorzugsweise zwischen 6.0 Gew.-% bis 30.0 Gew.-%; und

b) einen Gehalt an schnell verdaulicher Stärke (RDS Stärke) zwischen 10.0 Gew.-% bis 38 Gew.-%, vorzugsweise zwischen 15.0 Gew.-% bis 35.0 Gew.-%, besonders bevorzugt zwischen 20.0 Gew.-% bis 33 Gew.-% aufweist.

In einer weiteren Ausführungsform betrifft die vorliegende Erfindung auch Weizenmehle, deren Stärkekomponente einen Amylosegehalt zwischen 15.0 Gew.-% bis 30.0 Gew.-%, vorzugsweise zwischen 18.0 Gew.-% bis 30.0 Gew.-% und besonders bevorzugt zwischen 20.0 Gew.-% bis 30.0 Gew.-% bezogen auf die Stärke, und das Mehl eine DSC T-onset-Temperatur zwischen 63.0 °C bis 70.0 °C, vorzugsweise zwischen 64.0 °C bis 69.0 °C, besonders bevorzugt zwischen 65.0 °C bis 68.0 °C aufweist.

In einer weiteren Ausführungsform weisen die erfindungsgemäßen Weizenmehle eine Erhöhung der DSC T-onset-Temperatur im Vergleich zur DSC T-onset-Temperatur von entsprechenden Weizenmehlen aus Weizen-Wildtyppflanzen um 2 °C bis 9 °C, vorzugsweise um 3 °C bis 7 °C auf.

In einer weiteren Ausführungsform weist das erfindungsgemäße Weizenmehl eine DSC T-peak-Temperatur zwischen 70.0 °C bis 78.0 °C, vorzugsweise zwischen 71.0 °C bis 75.0 °C auf.

In einer weiteren Ausführungsform weisen die erfindungsgemäßen Weizenmehle eine Erhöhung der DSC T-peak-Temperatur im Vergleich zur DSC T-peak-Temperatur von entsprechenden Weizenmehlen aus Weizen-Wildtyppflanzen um 2 °C bis 8 °C, vorzugsweise um 3 °C bis 7 °C auf.

Es war für den Fachmann überraschend, dass die DSC-T-onset- und die DSC T-peak-Temperatur der erfindungsgemäßen Weizenstärke bzw. - mehle deutlich erhöht war gegenüber entsprechenden Stärken bzw. Mehlen von Weizen-Wildtyppflanzen. Insbesondere deshalb, weil eine Erhöhung der thermischen Stabilität normalerweise bei Stärken/Mehlen mit erhöhtem Amylosegehalt gegenüber Wildtyp-Stärken/-Mehlen zu beobachten ist, die erfindungsgemäßen Weizenstärken und -mehle aber einen Amylosegehalt aufweisen, der im Vergleich zu Stärken/Mehlen aus Weizen-Wildtyppflanzen nicht erhöht ist.

Bei vielen thermischen Prozessierungen und Applikationen ist der Einsatz von (granulären) Weizenstärken oder von Weizenmehlen enthaltend solche (granulären) Weizenstärken wünschenswert. Die hohe DSC T-onset bzw. T-peak Temperatur der erfindungsgemäßen Weizenstärken bzw. der erfindungsgemäßen Weizenmehle ist daher von besonderem Vorteil, da der Strukturerhalt der Stärkegranuli aufgrund dieser Eigenschaft auch bei erhöhten Prozesstemperaturen gewährleistet wird.

Die erfindungsgemäßen Weizenmehle zeichnen sich gegenüber bisher bekannten Weizenmehlen mit erhöhtem RS-Gehalt oder verringertem Gehalt an RDS, der in diesen auf einen gegenüber Weizen-Wildtypmehlen deutlich erhöhten Amylosegehalt zurückzuführen ist, durch eine wesentlich verbesserte Verarbeitbarkeit aus.

Die im Stand der Technik beschriebenen Weizenstärken mit einem Amylosegehalt von >70 Gew.-% (Regina et al., PNAS 103 (10), (2006), 3546-3551) weisen den Nachteil schlechter Verarbeitungseigenschaften auf, weil diese Stärken kaum verkleistern, ein erhöhte Neigung zur Retrogradation, ein geringes Quellvermögen aufweisen und in Wasser schlecht löslich sind. Für Anwendungen, in denen nur verkleisterte Stärken einsetzbar sind oder die Retrogradationsneigung vermindert werden soll (z.B. zur Vermeidung von Alterungsprozessen bei Backwaren) oder ein höheres Quellvermögen oder eine höhere Löslichkeit erforderlich sind, sind diese Weizenstärken mit einem Amylosegehalt von >70 Gew.-% daher entweder überhaupt nicht geeignet, oder sie müssen zusätzlich chemisch modifiziert werden, um die gewünschten Eigenschaften einzustellen. Gegenüber diesen Weizenstärken und - Mehlen mit einem Amylosegehalt >70 Gew.-% weisen die erfindungsgemäßen Weizenstärken und -mehle den Vorteil auf, dass sich vorteilhafte Verdauungseigenschaften (erhöhter RS-Gehalt, verringerter Gehalt an RDS) paaren mit vorteilhaften Verarbeitungseigenschaften (z.B. thermische Stabilität, Quellvermögen, Löslichkeit, Verkleisterung). Dadurch eignen sich die erfindungsgemäßen Weizenstärken und - mehle besser für solche Anwendungen, in denen entweder nur verkleisterte Stärken einsetzbar sind und/oder in denen ein höheres Quellvermögen und/oder eine höhere Löslichkeit und/oder eine erhöhte thermische Stabilität erforderlich sind.

Insbesondere weisen die erfindungsgemäßen Weizen-Mehle gegenüber den im Stand der Technik beschriebenen Weizen-Mehlen mit erhöhtem RS-Gehalt den Vorteil einer erhöhten Teigstabilität der Teige auf, die aus dem erfindungsgemäßen Weizen-Mehl hergestellt werden können. Denn der Teig, der aus dem Weizenmehl einer SSIIa inhibierten Weizenpflanze hergestellt wird, zeigt eine verringerte Teigstabilität (Morita et al., Cereal Chemistry 79, (2002), 491-495; Hung et al., Cereal Chemistry 82, (2005), 690-694; Hung et al., Trends in Food Science & Technology 17, (2006), 448-456). Ferner weisen die erfindungsgemäßen Weizen-Mehle den Vorteil auf, dass sie Im Vergleich zum Weizenmehl SSIIa inhibierter Weizenpflanzen beim Backen nicht die unerwünschte Verringerung des Brotvolumens (Morita et al., Cereal Chemistry 79, (2002), 491-495) zeigen. Darüber hinaus weisen die erfindungsgemäßen Weizen-Mehle gegenüber hochamylosigen Weizenmehlen den Vorteil auf, dass sie aufgrund ihrer guten Verarbeitungseigenschaften alleine eingesetzt werden können, wohingegen hochamylosehaltige Weizen-Mehle aufgrund ihrer schlechten Verarbeitungsqualität nur in Mischungen mit herkömmlichen Weizen-Mehlen eingesetzt werden können Hung et al., Trends in Food Science & Technology 17, (2006), 448-456). Durch die vorliegende Erfindung werden daher erstmals Weizenmehle mit gegenüber Weizenmehl aus Weizen-Wildtyppflanzen erhöhtem RS-Gehalt und/oder verringertem RDS-Gehalt zur Verfügung gestellt, die darüber hinaus wesentlich verbesserte Verarbeitungseigenschaften aufweisen im Vergleich zu hochamylosehaltigen RS-Weizen-Mehlen aus Weizen-Pflanzen mit inhibierter Genexpression der SSIIa (Yamamori et al., Australian Journal of Agricultural Research 57, (2006), 531-53) oder des Verzweigungsenzyms BEIIa (Regina et al., PNAS Vol. 103 No.10, (2006), 3546-3551).

In einer weiteren Ausführungsform betrifft die vorliegende Erfindung ein Weizenmehl, dessen Stärkekomponente einen Amylosegehalt zwischen 15.0 Gew.-% bis 30.0 Gew.-%, vorzugsweise zwischen 18.0 Gew.-% bis 29.5 Gew.-% und besonders bevorzugt zwischen 20.0 Gew.-% bis 29.5 Gew.-% aufweist und das Mehl zusätzlich wahlweise

a) einen Gehalt an resistenter Stärke des Mehles (RS Mehl) von mehr als 5.0 Gew.-%, vorzugsweise zwischen 6.0 Gew.-% bis 30.0 Gew.-%; und/oder
b) einen Gehalt an schnell verdaulichem Mehl (RDS Mehl) bezogen auf die Mehlmenge (Trockengewicht) zwischen 10 Gew.-% bis 22 Gew.-%, vorzugsweise zwischen 11 Gew.-% bis 21 Gew.-%, besonders bevorzugt zwischen 12 Gew.-% bis 20 Gew.-%; und/oder
c) eine DSC T-onset-Temperatur zwischen 63.0 °C bis 70.0 °C, vorzugsweise zwischen 64.0 °C bis 69.0 °C, besonders bevorzugt zwischen 65.0 °C bis 68.0 °C; und/oder
d) DSC T-peak-Temperatur zwischen 70.0 °C bis 78.0 °C, vorzugsweise zwischen 71.0 °C bis 75.0 °C aufweist.

Ein weiterer Gegenstand der vorliegenden Erfindung ist ein Verfahren zur Herstellung der erfindungsgemäßen Weizen-Mehle umfassend den Schritt des Mahlens mindestens einer Weizen-Pflanze, die eine heterologe Stärkesynthase II exprimiert.

In einer weiteren Ausführungsform des erfindungsgemäßen Verfahrens zur Herstellung der erfindungsgemäßen Mehle werden Weizen-Körner gemahlen, die aus (erfindungsgemäßen) Weizen-Pflanzenzellen bestehen, die eine heterologe Stärkesynthase II exprimieren.

Vorzugsweise umfasst das erfindungsgemäße Verfahren zur Herstellung von Weizen-Mehlen auch den Schritt des Erntens der (erfindungsgemäßen) Weizenpflanzen bzw. der Weizenkörner dieser Weizenpflanzen vor dem Mahlen, vorzugsweise des Waschens der (erfindungsgemäßen) Weizenpflanzen bzw. der Weizenkörner vor dem Mahlen und ferner den Schritt der Kultivierung der (erfindungsgemäßen) Weizenpflanzen vor dem Ernten.

In einer weiteren Ausführungsform der vorliegenden Erfindung umfasst das erfindungsgemäße Verfahren zur Herstellung von Mehlen eine Prozessierung der (erfindungsgemäßen) Weizenpflanzen bzw. Weizenkörnern, deren Pflanzenzellen eine heterologe Stärkesynthase II exprimieren, vor dem Mahlen.

Die Prozessierung kann dabei z.B. eine Hitzebehandlung und/oder eine Trocknung sein. Die Zerkleinerung von Weizenpflanzen, von stärkespeichernden Teilen oder Weizenkörnern solcher (erfindungsgemäßer) Weizenpflanzen vor dem Mahlen kann ebenfalls eine Prozessierung im Sinne der vorliegenden Erfindung darstellen. Das Entfernen von pflanzlichem Gewebe, wie z.B. von Spelzen der Körner, vor dem Mahlen stellt auch eine Prozessierung vor dem Mahlen in Sinne der vorliegenden Erfindung dar.

In einer weiteren Ausführungsform der vorliegenden Erfindung umfasst das Verfahren zur Herstellung von Mehlen nach dem Mahlen eine Prozessierung des Mahlgutes. Das Mahlgut kann dabei z.B. nach dem Mahlen gesiebt werden, um z.B. verschiedene Typenmehle herzustellen.

In einer weiteren Ausführungsform betrifft die vorliegende Erfindung die Verwendung von erfindungsgemäßem Weizenmehl zur Herstellung eines Lebensmittels.

In einer weiteren Ausführungsform betrifft die vorliegende Erfindung die Verwendung von erfindungsgemäßem Weizenmehl als Präbiotikum.

In einer weiteren Ausführungsform betrifft die vorliegende Erfindung eine Zusammensetzung enthaltend die erfindungsgemäße Weizenstärke und mindestens einen Nahrungsmittelzusatzstoff.

In einer weiteren Ausführungsform betrifft die vorliegende Erfindung eine Zusammensetzung enthaltend das erfindungsgemäße Weizenmehl und mindestens einen Nahrungsmittelzusatzstoff.

Als Nahrungsmittelzusatzstoff sollen im Zusammenhang mit der vorliegenden Erfindung beispielsweise Vitamine (z.B. Vitamin A, B1, B2, B3, B5, B6, B9, B12, C, D, E, F, K), Provitamine, Antioxidantien, Spurenelemente (z. B. Chrom, Eisen, Fluor, Jod, Kobalt, Kupfer, Mangan, Molybdän, Selen, Vanadium, Zink), Mengenelemente (z.B. Calcium, Chlor, Kalium, Magnesium, Phosphor, Schwefel, Natrium), Aromen, Farbstoffe, Öle, Fette, Fettsäuren, insbesondere (mehrfach) ungesättigte Fettsäuren, essentielle Fettsäuren, Kohlenhydrate (z.B. Stärken, Galaktooligosaccharide, Gentiobiose, Tagatose), Ballaststoffe (z.B. Zellulose, Hemizellulose, Pektin, Ligin), Präbiotika (z.B. Oligofruktose, Oligosaccharide, Chitosan, beta Glukane, Arabinogalactan), Probiotika (z.B. Bifidobakterien, Milchsäurebakterien wie z.B. der Gattung Lactobacillus), d.h. nicht-pathogene Mikroorganismen, die dem Lebensmittel lebend oder in Sporenform zugesetzt werden und die Darmflora positiv beeinflussen können.

Die Herstellung der erfindungsgemäßen Zusammensetzungen kann beispielsweise durch einfaches Mischen erfolgen.

In einer weiteren Ausführungsform betrifft die vorliegende Erfindung ein Lebensmittel enthaltend die erfindungsgemäße Weizenstärke.

In einer weiteren Ausführungsform betrifft die vorliegende Erfindung ein Lebensmittel enthaltend das erfindungsgemäße Weizenmehl.

In einer weiteren Ausführungsform betrifft die vorliegende Erfindung ein Lebensmittel enthaltend die erfindungsgemäße Zusammensetzung.

Typische Lebensmittel, die unter Verwendung der erfindungsgemäßen Weizenstärke, des erfindungsgemäßen Weizenmehls oder der erfindungsgemäßen Zusammensetzung hergestellt werden können, sind beispielsweise Tortillas, Tortillachips, Backwaren (z.B. Brot, Weizenbrot, Brötchen, Kekse, Kuchen, Waffeln, Muffins, Weizenfladen, Bagels), Pfannkuchen, Pizza, Teigwaren (z.B. Nudeln), Eintopfgerichte, Saucen, Weizenmehlpudding, Milchprodukte (z.B. Joghurt, Quark, Eis), Puddings, Aufstriche (z.B. Butter, Margarine), Getränke, Getränkepulver, Fertiggerichte, (Frühstücks)-Cerealien, Wurstwaren, Fleischwaren, Kindernahrung, Ketchup, Mayonnaise, Barbecue-Saucen und andere.

**Material und Methoden**

In den Beispielen wurden die folgenden Methoden verwendet. Diese Methoden können zur Durchführung der erfindungsgemäßen Verfahren verwendet werden, sie stellen konkrete Ausführungsformen der vorliegenden Erfindung dar, beschränken die vorliegende Erfindung jedoch nicht auf diese Methoden.

**1) Pflanzenmaterial und Kultivierung**

Weizenpflanzen: Triticum aestivum, Varietät Fielder

Die Kulturführung der Weizenpflanzen im Gewächshaus erfolgte unter nachfolgenden Bedingungen:

| | |
|---|---|
| **Substrat:** | Sondermischung für Aussaat<br>80% Weißtorf<br>20% Schwarztorf<br>100 kg/m$^3$ Sand<br>40 kg/m$^3$ Feuchtton<br>Struktur: fein<br>pH-Wert: 5,3 - 6,1<br>Grunddüngung: 2 kg/m$^3$ 12-12-17 (+2) und 100g/m$^3$ Radigen (Theraflor GmbH; Iserlohn; Germany) |
| **Töpfe:** | 12 cm Vierecktopf |
| **Standweite:** | Max. 64 Pflanzen/m$^2$ |
| **Düngung:** | Blattdüngung mit 1% Vitanica Si (5-3-7) + 10% Silikat (Fa.Compo) Hakaphos blau (15-10-15+2) 0,2g/Pflanze |
| **Temperatur:** | Tag 22-25°C/Nacht 16°C |
| **Licht:** | 16 Stunden, 350-400$\mu$Einstein/s/m |
| **Luftfeuchte:** | 50% rel. |
| **Pflanzenschutzmaßnahmen:** | nach Bedarf (Insektizid z.B.:Karate Zeon, Fungizid z.B. Stratego) |

**2) Herkunft der zur Transformation verwendeten Sequenzen und Konstrukte**

Zur Transformation von Weizen wurde eine mutagenisierte Form der OsSSII-3 aus Reis verwendet. Isolierung und Klonierung erfolgte wie in Beispiel 1 beschrieben. Der verwendete Transformationsvektor pBA71 ist in Beispiel 1 beschrieben.

**3) Transformation und Regeneration von Weizenpflanzen**

Weizenpflanzen wurden nach der von Wu et al. (2003; Wu H, Sparks C, Amoah B, Jones HD (2003) Factors influencing successful Agrobacterium-mediated genetic transformation of wheat. Plant Cell Reports 21:659-6686) beschriebenen Methode transformiert und regeneriert.

**4) Produktion von T1-Körner**

Zur Produktion von T1-Körnern wurden die in der Gewebekultur regenerierten Pflanzen nach Erreichen einer hinreichenden Pflanzengröße in Töpfe mit Erde überführt. Die Kulturführung sowie die Zusammensetzung der Erde sind unter 1) beschrieben. Die Pflanzen wurden zunächst mit einer Plastikhaube abgedeckt, um einen zu starken Feuchtigkeitsverlust zu vermeiden. Die Befruchtung der Blüten erfolgte mittels Selbstbestäubung. Die Körner wurden bis zur Reife an der Pflanze belassen und nach der Ernte für 3-5 Tage bei 37°C getrocknet.

**5) Produktion von T2-Körner unter Verwendung von Embryo rescue**

Um eine zeitnahe Produktion von T2-Körner zu bewerkstelligen wurden T1-Körner vor Eintreten der Samenruhe geerntet und von den Deckspelzen befreit. Zur Sterilisation der Samenoberfläche wurden die Samen in einem Eppendorfgefäß für eine Minute mit 70% Ethanol inkubiert, bevor das Ethanol durch eine 1%ige Natriumhypochlorid (NaOCl) -Lösung ersetzt wurde, in der die Samen für 20 Minuten verblieben. Zur vollständigen Entfernung des NaOCls wurden die Samen dreimal aufeinanderfolgend mit Wasser gewaschen. Die Embryonen wurden unter sterilen Bedingungen vom Endosperm getrennt und zur Keimung auf Petrischalen mit MS-Medium (Murashige und Skoog (1962); Physiol. Plant. 15:473-497) mit 3% Saccharose gelegt. Nach dreitägiger Inkubation im Dunkeln wurden die Petrischalen mit den Embryonen ins Licht überführt. Die gekeimten Pflanzen wurden in Gläser mit MS-Medium mit 2% Saccharose überführt und bis zum Erreichen einer für den Transfer in Erdkultur ausreichenden Größe dort belassen. Die weitere Produktion von T2-Körner erfolgte entsprechend dem unter 4) beschriebenen Ablauf.

**6) Herstellung von Weizenmehlen und nachfolgende Extraktion von Weizenstärke**

Für Analysen im kleinen Maßstab wurden Weizenkörner in 2ml Eppendorfgefässe gefüllt und zusammen mit einer Wolfram-Carbit-Kugel für 30 Sekunden bei 30 Hertz in einer Kugelmühle der Firma Retsch GmbH (Haan) zerkleinert. Das resultierende Mehl diente als Ausgangsmaterial für alle weiteren Analysen.
Für die Produktion von größeren Mengen an Mehl als Ausgangsmaterial für die Extraktion von Weizenstärke wurden Weizenkörner in einer Brabender Mühle (Typ Quadrumat Junior; Brabender GmbH, Duisburg) zu einem Typenmehl 550 vermahlen. Jeweils 10 g Weizenmehl (Typ 550) wurden in die Gluten-Auswaschmaschine Glutomatic (Perten GmbH; Hamburg) eingefüllt und die Maschien nach Angaben des Herstellers bedient. Die Stärke wird als wässrige Suspension aufgefangen und nachfolgend mittels Zentrifugation (3000 rpm, 10 min, RT) sedimentiert. Das Pellet wird erneut in Wasser resuspendiert und die entstandene Suspension wie oben beschreiben zentrifugiert. Die obere gelblich/braune Schicht des Sediment wurde manuel entfernt und das verbleibende Sediment in Aceton resuspendiert. Nach erneuter Zentrifugation wurde der Überstand verworfen und das Stärkepellet unter dem Abzug getrockent.
Vor der weiteren Verwendung wurde die Stärke mittels Mörsern zu einem feinen Pulver verarbeitet.

**7) Thermische Analyse von Weizenmehl mittels Wärmefluss-Kalorimetrieverfahren (Differential Scanning Calorimetry (DSC))**

Jeweils 10mg Weizenmehl oder Weizenstärke wurden in einem Edelstahl-Pfännchen (Perkin Elmer, "Large Volume Stainless Steel Pans" [03190218], Volumen 60 μl) mit 30μl bi-destilliertem Wasser versetzt und dieses nachfolgend hermetisch verschlossen. Mit einer Heizgeschwindigkeit von 10°C/min wurde die Probe von 20°C bis 150°C in einem Diamond DSC-Gerät (Perkin Elmer) erhitzt. Dabei dient ein leeres verschlossenes Edelstahlpfännchen als Referenz. Das System wurde mit definierten Mengen Indium kalibriert.
Die Datenanalyse wurde mittels Softwareprogramm von Pyris (Perkin Elmer, Version 7.0) durchgeführt. Die Weiterverarbeitung auswertbarer Rohdaten erfolgte durch Analyse der einzelnen Peaks der Phasenübergänge 1. Ordnung auf T-onset (°C), T-peak (°C), T-end (°C) und dH (J/g) (Standard ist dabei die gerade Basislinie).
DSC T-onset ist dabei charakterisiert als der Schnittpunkt zwischen der Verlängerung der Basislinie und der an die ansteigende Flanke des Peaks angelegten Tangente durch den Wendepunkt. Sie charakterisiert den Beginn der Phasenumwandlung.
Als Maximaltemperatur DSC T-peak wird die Maximaltemperatur bezeichnet, bei der die DSC-Kurve ein Maximum erreicht hat (d.h., diejenige Temperatur, bei der die erste Ableitung der Kurve Null ist).
Bei der in Pyris verwendeten Funktion (calc-peak Area) wird von Hand eine Start-und eine Endtemperatur für den Baselinefit eingegeben.

**8) Bestimmung des Anteiles an schnell verdaubarer und resistenter Stärke in Weizenmehlen/-stärken**

Die Bestimmung des Anteils an resistenter Stärke erfolgt in Anlehnung an die nach Englyst et al. (Europ. J. of Clinical Nutrition 46 (Suppl. 2), (1992), S 33-50)) beschriebene Methode (siehe insbesondere folgende Abschnitte aus Englyst et al., Seite S35-S36: "Reagents, Apparatus, Spectrophotometer"; Seite S36-S37, Absatz ,,Measurement of free glucose (FG)"; Seite S38, Absatz ,,Measurement of RDS and SDS").

Zur Herstellung der Enzymlösung werden 1,2 g Pankreatin (Merck) in 8 ml Wasser für 10 Minuten bei 37 °C extrahiert. Nach Zentrifugation (10', 3000 U/min; RT) werden 7,02 ml des Überstandes mit 1,227 ml demineralisiertem Wasser und 0,136 ml Amyloglukosidase (3260 U/ml) (Sigma-Aldrich, Taufkirchen) versetzt.

Parallel werden 10 mg (Trockengewicht) Weizenmehl oder -stärke pro Probe in einem 2 ml Reaktionsgefäß mit 0,75 ml Natrium-Acetat-Puffer (0,1 M Natrium-Acetat pH 5,2; 4 mM $CaCl_2$) versetzt und zur Erwärmung des Ansatzes für 5 Minuten bei 37°C inkubiert.

Der Verdau der Stärke wird durch Zugabe von jeweils 0,25 ml Enzymlösung pro Ansatz gestartet. Als Kontrolle dient ein Ansatz ohne Stärke. Nach jeweils 20 und 120 Minuten werden Aliquots von 50 µl entnommen und mit je 12,5 µl 25 %iger TCA für 10 min auf Eis gestellt, wodurch die Enzyme inaktiviert werden. Nach Zentrifugation (2', 13 000 U/min; RT) werden 25 µl des Überstandes mit Wasser auf 1 ml verdünnt.

Zur Bestimmung der Gesamtstärke werden nach der 120 min-Probenahme je 150 µl hitzestabile α-Amylase (53 U) in MOPS-Puffer (50 mM; pH 7,0) zu den Ansätzen gegeben. Anschließend werden die Proben für 6 min bei 95°C geschüttelt. Dann werden die Proben bei RT 5 min abgekühlt, bevor je 200 µl Amyloglucosidase (4,5 U) in NaOAc-Puffer (200 mM, pH 4,5) dazugegeben werden. Nach 60 min Inkubation bei 50 °C werden Aliquots von 50 µl entnommen und wie oben inaktiviert und verdünnt.

Die verdünnten Proben werden zur Messung des Gehaltes an freigesetzter Glucose nach 20 min, 120 min und nach Gesamtstärkeaufschluss verwendet. Hierzu werden 20µl verdünnte Probe mit 180 µl Messpuffer (100 mM Imidazol/HCl pH 6,9; 5 mM $MgCl_2$; 1 mM ATP; 2 mM NADP) vermischt und die Absorption der Probe bei 340 nm erfasst. Die Umsetzung der Glukose wird durch Zugabe von 2 µl Enzym-Mix (10 µl Hexokinase, 10 µl Glukose-6-Phosphat-Dehydrogenase, 80 µl Messpuffer) gestartet und der äquimolare Umsatz von NADP zu NADPH bei 340 nm bis zum Erreichen eines Plateaus verfolgt. Die ermittelten Glukose-Mengen werden in Relation zur Einwaage gesetzt und ergeben den Anteil der Probe, der nach dem entsprechenden Zeitraum als Glukose freigesetzt wurde. Insbesondere bei Weizenmehlen können die Werte an freigesetzter Glucose nach 20 min und 120 min auch auf die Menge an Gesamtglucose bezogen werden, da der Anteil an Stärke am Frischgewicht variieren kann.

**9) Aufbereitung von Weizenmehl/-stärke zur Untersuchung der Amylopektin-Seitenkettenverteilung mittels Hochdruck-Anionenaustausch-Chromatographie**

Pro Probe wurden 10 mg Weizenmehl oder -stärke in ein 2 ml Eppendorfcup eingewogen und mit 250µl 90% (v/v) DMSO versetzt. Nach einstündigem Lösen der Probe unter Schütteln bei 95°C wurden 375µl Wasser zugesetzt und der Ansatz für eine Stunde bei 95°C inkubiert. Zu 200µl des Ansatzes wurden 300µl 16,7 mM Natriumacetat pH 3,5 sowie 0,5 U Isoamylase aus Pseudomonas sp. (Megazyme; Bray, Irland) gegeben und 24 Stunden bei 37°C inkubiert. Für die Chromatographie wurden 50 µl des Ansatzes 1:10 mit Wasser verdünnt und nachfolgend durch 0,22µm Nylon-Filter filtriert. Etwa 50µl des Filtrats wurden injiziert.

Chromatographie- Methode:

| | | |
|---|---|---|
| HPLC- Anlage: | GP 50 Dionex | Gradient Pump |
| | ED 50 | Dionex Electrochem. Detector/ PAD |
| | AS 50 | Autosampler |
| | Säulenofen | |
| Säule: | Dionex CarboPac PA 100 4 x 250 mm (P/N 046110) | |
| | mit Guard Column PA 100 4 x 50 mm (P/N 046115) | |

Gerätekonfiguration:

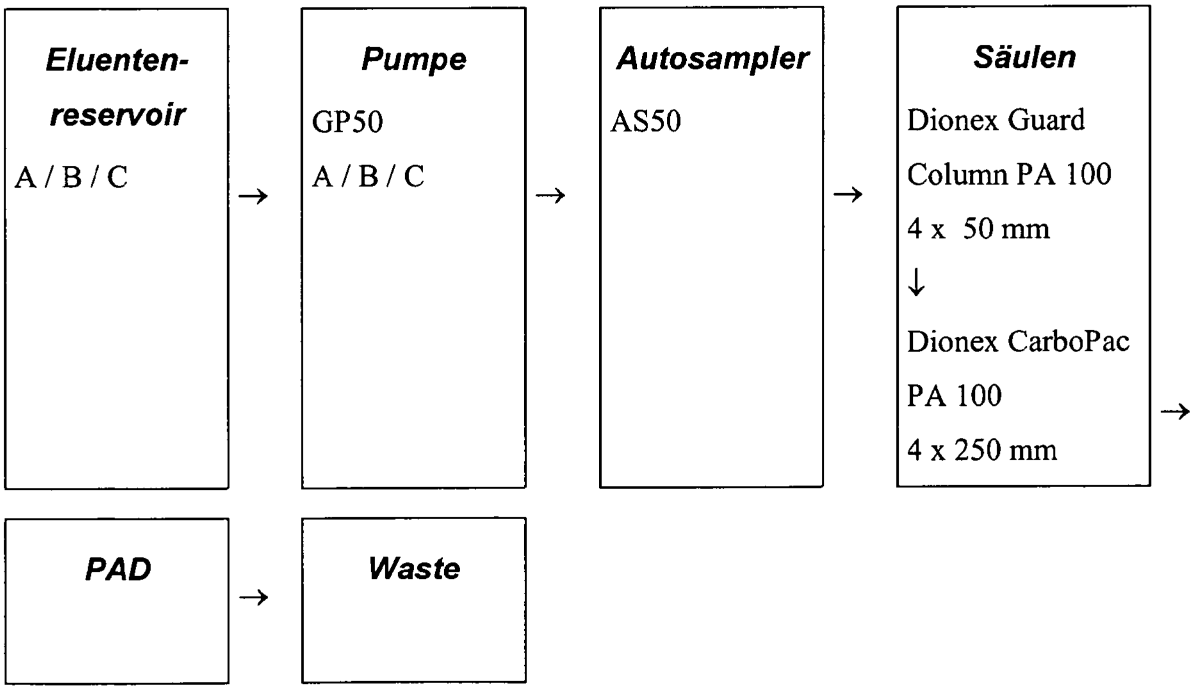


**HPAEC Programm:**

```
        Pressure.LowerLimit =                        50
        Pressure.UpperLimit =                        3500
        %A.Equate =                                  "Wasser"
        %B.Equate =                                  "NaOAc 1,0 M in NaOH
0,25 M"
        %C.Equate =                                  "NaOH 0,25 M"
        ECD.Data_Collection_Rate =                   1.0
        Waveform Time = 0.00, Potential =            0.05
        Waveform Time = 0.20, Potential =            0.05, Integration =
Begin
        Waveform Time = 0.40, Potential =            0.05, Integration = End
        Waveform Time = 0.41, Potential =            0.75
        Waveform Time = 0.60, Potential =            0.75
        Waveform Time = 0.61, Potential =            -0.15
        Waveform Time = 1.00, Potential =            -0.15
        Cell =                                       On

        Pump_Pressure.Formula                        Formula=Pump.Pressure
        Pump_Pressure.Type =                         Analog
        Pump_Pressure.Step =                         Auto
        Pump_Pressure.Average =                      On

        Flush                                        Volume = 500
        Wait                                         FlushState
        NeedleHeight =                               2
        CutSegmentVolume =                           1
        SyringeSpeed =                               4
        Cycle =                                      0
        WaitForTemperature =                         False
        Wait                                         SampleReady
```

```
0.000 Flow =                                                   1.00
          %B =                                                 0.0
          %C =                                                 100.0
          %D =                                                 0.0
          Curve =                                              5
          Load
          Inject
          Wait                                                 InjectState
          ECD.Autozero
          ECD_1.AcqOn
          Pump_Pressure.AcqOn
          Flow =                                               1.00
          %B =                                                 0.0
          %C =                                                 100.0
          %D =                                                 0.0
          Curve =                                              5

4.000 Flow =                                                   1.00
          %B =                                                 11.0
          %C =                                                 89.0
          %D =                                                 0.0
          Curve =                                              5
          Flow =                                               1.00
          %B =                                                 11.0
          %C =                                                 89.0
          %D =                                                 0.0
          Curve =                                              4

95.000 Flow =                                                  1.00
          %B =                                                 35.0
          %C =                                                 65.0
          %D =                                                 0.0
          Curve =                                              4

97.000 Flow =                                                  1.00
          %B =                                                 100.0
          %C =                                                 0.0
          %D =                                                 0.0
          Curve =                                              5

98.000 Flow =                                                  1.00
          %B =                                                 100.0
          %C =                                                 0.0
          %D =                                                 0.0
          Curve =                                              5

105.000 Flow =                                                 1.00
          %B =                                                 0.0
          %C =                                                 100.0
          %D =                                                 0.0
          Curve =                                              5

106.000 Flow =                                                 1.00
          %B =                                                 0.0
          %C =                                                 5.0
          %D =                                                 95.0
          Curve =                                              5

112.000 Flow =                                                 1.00
          %B =                                                 0.0
          %C =                                                 5.0
          %D =                                                 95.0
          Curve =                                              5
```

```
ECD_1.AcqOff
Pump_Pressure.AcqOff
          End
```

Die Auswertung der Daten erfolgt mit Dionex Chromeleon v6.70 (Dionex Corporation, Sunnyvale, Kalifornien, USA). Das Handbuch "Tutorial and User Manual" der Version 6.60, März 2004, ist über Dionex zu beziehen bzw. kann über die Homepage (http://www.dionex.com) herunter geladen werden.
Zum Vergleich der Chromatogramme gegeneinander wurden für jedes Chromatogramm die identifizierten Peaks der unterschiedlichen Maltooligasaccharide Mittelwert-normalisiert (Summe aller Peakflächen = 1). Die Auswertung erfolgte aufgrund der ,,force common baseline", wie im Dionex Chromeleon v.6.60 zu "log baseline" beschrieben. Dabei wird die log baseline gesetzt kurz vor dem ersten Seitenkettenpeak und bis zum letzten auswertbaren Peak des kürzesten Chromatogramms eines Messdurchgangs, daraus wird der letzte auswertbare Peak für alle Chromatogramme berechnet.

**10) Bestimmung des Gehaltes an apparenter Amlyose mittels DSC**

Der Amylose-Gehalt von Weizenmehl/-stärke wurde mittels DSC in Anlehnung an die Methode von Polaske et. Al (2005) (Starch 57:118-123) ermittelt.
Das Messprinzip dieser Methode beruht darauf, dass Stärke - bestehend aus Amylose und Amylopektin - zunächst in Gegenwart einer Lipidlösung vollständig aufgeschmolzen wird und nachfolgend wieder abgekühlt wird. Im Verlauf des Abkühlungsprozess kommt es zur Ausbildung von Amlyose-Lipid-Komplexen, deren Ausbildung zu einer Freisetzung von Energie führt, welche als Peak im Thermogramm sichtbar wird und entsprechend ausgewertet werden kann. Hierbei ist die Peakfläche entpsrechend der Menge an freigesetzter Energie direkt proportional zur Menge an Amlyose in der Probe.
Für die Messung wurden 10 mg Weizenmehl/-stärke in ein Edelstahlpfännchen (Perkin Elmer, "Large Volume Stainless Steel Pans" [03190218], Volumen 60 μl) eingewogen, mit 50μl einer 2%igen wässrigen L-a-Lysophosphatidylcholine (LPC)-Lösung versetzt. Nachfolgend wurden die Pfännchen hermetisch verschlossen und in dem DSC-Gerät Diamond (Perkin-Elmer Inc, USA) zunächst bei einer Heizrate von 10°C/min von 25°C auf 125°C erhitzt, bei dieser Temperatur für 2 Minuten belassen und anschließend bei identischer Kühlrate von 125°C auf 60°C abgekühlt. Dabei diente ein leeres verschlossenes Edelstahlpfännchen als Referenz. Das System wurde mit definierten Mengen Indium kalibriert.
Die Datenanalyse wurde mittels Softwareprogramm von Pyris (Perkin Elmer, Version 7.0) durchgeführt. Zur Bestimmung des Amlyosegehaltes einer Probe wurde die Fläche des Peak (deltaH) in der Abkühlphase in dem Temperaturbereich zwischen 65°C und 105°C ermittelt und auf die Einwaage bezogen. Zur Normierung des Systems werden parallel Proben mit bekanntem Amylose-Gehalt analysiert und aus diesen Daten eine Eichkurve erstellt, die zur Berechnung des Amlyosegehalte (ausgedrückt in %FG) in der Probe herangezogen wird.

**11) Analyse von Weizenstärke mittels Rapid Visco Analyser (RVA)**

Das Prinzip dieser Analyse beruht darauf, dass eine Suspension aus Wasser und Weizenstärke einem definierten Temperatur- und Scher-Protokol unterzogen wird und dabei kontinuierlich die Viskosität der Suspension aufgezeichnet wird. Als Messgerät dient ein RVA Super3 der Firma Newport Scientific (Macclesfield, UK) mit der entsprechenden Software "Thermocline for Windows", Version 2.3.
Für die Analyse wurden 2,5 g Weizenstärke (Einwaage als reines Trockengewicht des Probenmaterials, korrigiert auf 0% Feuchte) in ein Messgefäß eingewogen, mit 25 ml Wasser versetzt und das Messgerät nach Einsetzen eines Rührers in das Gerät eingespannt.
Das folgende Temperatur- und Scher-Profil wurde appliziert:
(entspricht der "RVA-method for wheat and rye flour" von Newport Scientific; Australien)

| Zeit | Art | Wert |
|---|---|---|
| 00:00:00 | Temp | 50°C |
| 00:00:00 | Speed | 960 rpm |
| 00:00:10 | Speed | 160 rpm |
| 00:01:00 | Temp | 50°C |
| 00:04:42 | Temp | 95°C |
| 00:07:12 | Temp | 95°C |

(fortgesetzt)

| Zeit | Art | Wert |
|---|---|---|
| 00:11:00 | Temp | 50°C |
| 00:13:00 | End of test | |

Nach Beendigung der Messung wurden die folgenden Parameter ermittelt:
Peak Viskosität (höchste Viskosität zwischen 2 und 7 Minuten Messzeit)
Trough Viskosität (geringste Viskosität zwischen 7 und 12 Minuten Messzeit)
Final Viskosität (Viskosität am Ende der Messung)
Breakdown = Peak - Trough
Setback = Final - Trough
Pasting Temperatur (Temperatur, bei der sich in einem Zeitintervall von 0,5 Minuten die Viskosität um mehr als 50cP ändert)
Peak time (Zeit, bei der die Peak Viskosität erreicht ist)

**12) Bestimmung des Phosphatgehaltes in C6-Position (C6-P-Gehalt)**

In der Stärke können die Positionen C3 und C6 der Glucoseeinheiten phosphoryliert sein. Zur Bestimmung des C6-P-Gehaltes der Stärke (modifiziert nach Nielsen et al., 1994, Plant Physiol. 105: 111-117) wurden 50 mg Weizenmehl/-stärke in 500 μl 0,7 M HCl 4 h bei 95°C unter ständigem Schütteln hydrolysiert. Anschließend wurden die Ansätze für 10 min bei 15.500 g zentrifugiert und die Überstände mittels einer Filtermembran (0,45 μM) von Schwebstoffen und Trübungen gereinigt. Von dem klaren Hydrolysat wurden 20 μl mit 180 μl Imidazol-Puffer (300 mM Imidazol, pH 7,4; 7,5 mM $MgCl_2$, 1 mM EDTA und 0,4 mM NADP) gemischt. Die Messung wurde im Photometer bei 340 nm durchgeführt. Nach Erfassung der Basisabsorption wurde die Enzymreaktion durch die Zugabe von 2 Einheiten (units) Glucose-6-Phosphat Dehydrogenase (von *Leuconostoc mesenteroides*, Boehringer Mannheim) gestartet. Die Absorptionsänderung beruht auf einer äquimolaren Umsetzung von Glucose-6-Phosphat und NADP zu 6-Phosphoglukonat und NADPH, wobei die Bildung des NADPH bei der o. g. Wellenlänge erfasst wird. Die Reaktion wurde bis zum Erreichen eines Plateaus verfolgt. Das Ergebnis dieser Messung liefert den Gehalt an Glucose-6-Phosphat im Hydrolysat. Aus dem identischen Hydrolysat wurde anhand des Gehaltes an freigesetzter Glucose der Grad der Hydrolyse bestimmt. Dieser wird verwendet, um den Gehalt an Glucose-6-Phosphat auf den Anteil hydrolysierter Stärke aus der Menge Frischgewicht zu beziehen. Hierzu wurden 10μl Hydrolysat mit 10μl 0,7 M NaOH neutralisiert und nachfolgend 1:100 mit Wasser verdünnt. 4 μl dieser Verdünnung wurden mit 196μl Messpuffer (100mM Imidazol pH 6,9; 5 mM $MgCl_2$, 1 mM ATP, 0,4 mM NADP) versetzt und zur Bestimmung der Basisabsorption verwendet. Die Reaktion wurde durch Zugabe von 2 μl Enzym-Mix (Hexokinase 1:10; Glucose-6-Phosphat Dehydrogenase aus Hefe 1:10 in Messpuffer) und bei 340nm bis zum Plateau verfolgt. Das Messprinzip entspricht dem der ersten Reaktion.

Das Ergebnis dieser Messung liefert die Menge an Glucose (in mg), die im Verlauf der Hydrolyse aus der im Ausgangsmaterial vorhandenen Stärke freigesetzt wurde.

Nachfolgend wird das Ergebnis beider Messung in Relation gesetzt, um so den Gehalt an Glucose-6-Phosphat pro mg hydrolysierter Stärke auszudrücken. Anders als bei einem Bezug der Menge an Glucose-6-Phosphat auf das Frischgewicht der Probe wird durch diese Berechnung die Menge an Glucose-6-Phosphat lediglich auf den Teil der der Stärke bezogen, welcher vollständig zu Glucose hydrolysiert wurde und somit auch als Quelle für das Glucose-6-Phosphat anzusehen ist.

**13) Bestimmung der SSII-Aktivität mittels Aktivitätsgel**

Der Nachweis der verschiedenen Stärke-Synthase-Aktivitäten in unreifen Weizenkörnern erfolgte mittels Aktivitätsgelen (Zymogrammen), bei denen Proteinextrakte unter nativen Bedingungen in einem Polyacrylamidgel aufgetrennt und nachfolgend mit entsprechenden Substraten inkubiert werden. Das entstandene Reaktionsprodukt (Stärke) wurde mittels Lugol'scher Lösung (2% (w/v) KI; 0,2% (w/v) $I_2$) im Gel angefärbt.

Einzelne unreife Weizenkörner (ca. 15 Tage nach der Blüte - gemessen ab dem Tag des Blühbeginns) wurden in flüssigem Stickstoff schock gefroren und in 150-200 μl kaltem Extraktionspuffer (50 mM Tris/HCl pH 7,6, 2,5 mM EDTA, 2 mM DTT, 4 mM PMSF, 0,1% (w/v) Glykogen, 10% (v/v) Glyzerin) homogenisiert. Nach Zentrifugation (15 min, 13000 g, 4°C) wurde der klare Überstand in ein frisches Reaktionsgefäß überführt und ein Aliquot des Extraktes zur Bestimmung des Proteingehaltes nach Bradford (1976, Anal Biochem 72: 248-254) verwendet.
Die Auftrennung der Proteinextrakte erfolgte mittels eines kontinuierlichen 7,5% Polyacrylamid-Geles (7,5% AA/BAA 37,5:1; 25 mM Tris/HCl pH 7,6, 192 mM Glycin, 0,1% (w/v) APS, 0,05% (v/v) TEMED) unter Verwendung von einfach konzentriertem Laufpuffer (25 mM Tris/HCl, 192 mM Glycin). Vor Beladung der Gele erfolgt ein Vorlauf zur Entfernung von Radikalen für 30 Minuten bei 8mA und 4°C. Für jede Probe wurden 30 μg Protein aufgetragen und die Elektrophorese für 2 - 2,5 Stunden bei 4°C durchgeführt.
Nachfolgend wurden die Gele in 15 ml Inkubationspuffer ( 0,5M Natriumcitrat pH 7,0, 25mM Kaliumacetat, 2mM EDTA, 2 mM DTT, 0,1% (w/v) Amylopektin, 50mM Tricine/NaOH pH 8,5, 1 mM ADP-Glucose) über Nacht bei Raumtemperatur unter ständigem Schütteln inkubiert. Die Anfärbung der gebildeten Stärke erfolgte mittels Lugol'scher Lösung.

Um zu ermitteln, um das wie viel Fache die Aktivität eines Proteins mit der Aktivität einer Stärkesynthase II im Vergleich zu entsprechenden nicht genetisch modifizierten Wildtyp-Pflanzen erhöht ist, wurden Proteinextrakte der genetisch modifizierten Linien jeweils sequentiell verdünnt und entsprechend der oben beschriebenen Methode elektrophoretisch aufgetrennt. Die Durchführung der weiteren Schritte erfolgte wie oben bereits beschrieben. Nach Anfärbung der Zymogrammme mit Lugol'scher Lösung wurde ein optischer Vergleich der Intensität der angefärbten Produkte, produziert durch ein Protein mit der Aktivität einer Stärkesynthase II für die verschiedenen Verdünnungen der Proteinextrakte von genetisch modifizierten Pflanzen mit den betreffenden Produkten des unverdünnten Wildtyp-Proteinextraktes durchgeführt. Da die Intensität der Färbung der Produkte direkt korreliert mit der Aktivität eines Proteins mit der Aktivität einer Stärkesynthase II, weisen Banden der Produkte mit gleichen Intensitäten die gleiche Aktivität auf. Weist die Bande des Produktes eines Proteins mit der Aktivität einer Stärkesynthase II im verdünnten Proteinextrakt die gleiche Intensität auf, wie die betreffende Bande des Produktes von entsprechendem, unverdünntem Proteinextrakt aus Wildtyp-Pflanzen, so entspricht der Verdünnungsfaktor dem Grad der Erhöhung der Aktivität in der betreffenden genetisch modifizierten Pflanze.

Die folgenden Beispiele erläutern die vorstehend beschriebene Erfindung.

### Beispiel 1

### Herstellung des Vektors pBA71 zur Expression einer synthetischen, mutagenisierten Form der Stärkesynthase II-3 aus Reis (=synthSS2 Os mut) in Weizen

Funktionelle Elemente des Transformationsvektors

Der Vektor pBA71 ist ein Derivat des Plasmids pGREEN (Hellens *et al.,* 2000). Der "backbone" des Plasmids enthält folgende funktionelle Elemente:

[1] Den "origin of replication" des Plasmids pBR322 (Bolivar *et al*., 1977) zur Replikation in *Escherichia coli* (ORI ColE1)
[2] Einen Selektionsmarker vermittelnd Resistenz gegen Kanamyzin (*nptI*; Grindley and Joyce, 1980) zur Vermehrung in *Escherichia coli* and *Agrobacterium tumefaciens.*
[3] Den "origin of replication" des Plasmids pSA (Tait *et al.,* 1982) zur Replikation in *Agrobacterium* (ORI pSA)

Die in das Pflanzengenom übertragenen genetischen Elemente sind in der Vektor Karte dargestellt (siehe Figur 1) und detailliert beschrieben in Tabelle 1.

Tabelle 1:

| Beschreibung der genetischen Elemente welche in das Pflanzengenom inseriert werden | | |
|---|---|---|
| **Nt Positions** | **Orientation** | **Origin** |
| 540-563 | | **LB:** "left border repeat" der T-DNA of *Agrobacterium tumefaciens* (Zambryski, 1988) |
| 615-2623 | Clockwise | **PUbiZm + intron:** Promotor und Intron des Ubiquitin-1 Gens (*ubi1*) aus *Zea mays* (Christensen *et al.,* 1992). |
| 2624-3204 | Clockwise | ***bar*.** Kodierende Region des Phosphinothricin Acetyltransferase Gens aus *Streptomyces hygroscopicus* Thompson *et al.* (1987) |
| 3205-3461 | Clockwise | **3'nos:** Sequenz umfassend die 3' nicht translatierte Region des Nopalin Synthase Gens der T-DNA von pTiT37 (Depicker *et al.,* 1982) |

(fortgesetzt)

| Beschreibung der genetischen Elemente welche in das Pflanzengenom inseriert werden | | |
|---|---|---|
| **Nt Positions** | **Orientation** | **Origin** |
| 3543-3743 | Counter clockwise | **3'35S:** Fragment des 3' nicht translatierten Bereichs des 35S Transkripts des Blumenkohl Mosaik Virus (Sanfaçon et al (1991) |
| 3759-6191 | Counter clockwise | ***synthSS2* Os mut:** mutagenisierte kodierende Region der Stärkesynthase II-3 aus *Oryza sativa* (Jiang et al., 2004 oder Acc. AF419099); durch DNA-Synthese synthetisch hergestellte Version mit einem Nukleotidaustausch an Position 2209 (Adenin ausgetauscht gegen ein Guanin); DNA-Sequenz der ***synthSS2* Os mut** siehe SEQ ID NO. 3* |
| 6302-6821 | Counter clockwise | **intron1 *ubi1* Zm:** Erstes Intron des Ubiquitin-1 Gens (*ubi1*) aus *Zea mays* (Christensen *et al.,* 1992). |
| 6844-7561 | Counter clockwise | **Phmw4 Ta:** Promotor Region der hochmolekularen Untereinheit des Glutenin 1 D Gens aus *Triticum aestivum* (Jiang et al., GenBank Acc. No. DQ208971) |
| 8052-8076 | | **RB:** "right border repeat" der T-DNA of *Agrobacterium tumefaciens* (Zambryski, 1988) |
| *Ergänzender Hinweis zur ***synthSS2* Os mut:** | | |

Im Unterschied zur Stärkesynthase II-3 aus *Oryza sativa* wurde unter anderem eine Restriktionsschnittstelle zur Klonierung EcoRV (N-Terminus), eine ,,Kozak" Consensus Sequenz für Monokotyledone Pos -10 bis -1 relativ zum Startcodon (Joshi et al., 1997 PMB 35: 993-1001, ,,Contex sequences of translation initiation codon in plants") sowie C-terminal eine HindIII site zur Klonierung eingeführt. Die interne HindIII site (nt 1037-1042) wurde zu diesem Zwecke über Codon-Switch deletiert (nt1038 A->G). Ebenso wurde zu Klonierungszwecken die Not I sites (nt 406-413 und 470-477) über Codon Switch deletiert (nt 408 G->C), (nt 470 G->C, nt 471 C->A, nt 475 C->A, nt 477 C->G). Ebenso deletiert wurde die interne XhoI site (nt 2302-2307) durch einen Nukleotidaustausch an Position 2304 (C->G). Im Sinne einer Optimierung der intrinsischen Aktivität der Stärkesynthase SS2a wurde auf der kodierenden Region der synthetischen Sequenz eine spezifische Mutagenese mit Hilfe der Primer Os_SS2-3-Mutag.F1 (CTg Agg gAC ACC gTg TCg gCg TTC gA = SEQ ID No. 16) und Os_SS2-3-Mutag.R1 (TCg AAC gCC gAC ACg gTg TCC CTC Ag = SEQ ID No. 17) und dem Site Directed Mutagenesis Kit von Stratagene nach Herstellerangaben durchgeführt, um das Adenin an Position 2209 des offenen Leserasters zu Guanin zu verändern. Damit wurde in der resultierenden Kodierregion ein Kodon geschaffen, welches an der AS Position 737 für Valin anstelle von Methionin kodiert.

Referenzen:

| |
|---|
| Bolivar F., Rodriguez R.L.., Greene P.J., Betlach M.C., Heyneker H.L., Boyer H.W. (1977). Construction and characterization of new cloning vehicles. II. A multipurpose cloning system, Gene, 2, 95-113. |
| Christensen A. H., Sharrock R.A., Quail P.H. (1992). Maize polyubiquitin genes: structure, thermal pertubation of expression and transcript splicing, and promoter activity following transfer to protoplasts by electroporation. Plant Molecular Biology, 18, 675-689. |
| Depicker A., Stachel S., Dhaese P., Zambryski P., Goodman H.M. (1982). Nopaline synthase: transcript mapping and DNA sequence. Journal of Molecular and Applied Genetics, 1, 561-573. |
| Grindley N.D.F., Joyce C.M. (1980) Genetic and DNA sequence analysis of the kanamycin resistance transposon Tn903. Proc. Natl. Acad. Sci. U.S.A. 77(12):7176-7180 |
| Hellens RP, Edwards EA, Leyland NR, Bean S, Mullineaux PM. (2000). pGreen: a versatile and flexible binary Ti vector for Agrobacterium-mediated plant transformation. Plant Mol Biol 42(6): 819-832. |

(fortgesetzt)


| |
|---|
| Jiang H., Dian W., Liu F., Wu P. (2004). Molecular cloning and expression analysis of three genes encoding starch synthase II in rice. Planta, 218, 1062-1070. |
| Jiang,Q.-T., Wang,X.-R., Wei,Y.-M., Zheng,Y.-L.. Triticum aestivum high molecular weight glutenin subunit 1 Dx2 gene, promoter region and 5' UTR. ACCESSION DQ208971 |
| Sanfaçon H, Brodmann P, Hohn T. (1991) A dissection of the cauliflower mosaic virus polyadenylation signal. Genes Dev. 5(1): 141-149. |
| Tait RC, Close TJ, Rodriguez RL, Kado CI. (1982) Isolation of the origin of replication of the IncW-group plasmid pSa. Gene. 20(1): 39-49. |
| Thompson C.J., Rao Movva N., Tizard R., Crameri R., Davies J., Lauwereys M., Botterman J. (1987). Characterization of the herbicide resistance gene bar from Streptomyces hygroscopicus. The EMBO Journal, 6, 2519-2523. |
| Zambryski P. (1988). Basic processes underlying Agrobacterium-mediated DNA transfer to plant cells. Annual Review of Genetics, 22, 1-30. |


**Beispiel 2**

**Herstellung und Identifizierung genetisch modifizierter Weizenpflanzen, die eine erhöhte SSII-Aktivität aufweisen**

Zur Herstellung genetisch veränderter Pflanzen mit einer erhöhten Stärke Synthase II (SSII) Aktivität, wurde die T-DNA des Plasmids pBA71 mit Hilfe der bei Wu et al. (2003; Wu H, Sparks C, Amoah B, Jones HD (2003) Factors influencing successful Agrobacterium-mediated genetic transformation of wheat. Plant Cell Reports 21:659-6686) beschriebenen Methode mit Hilfe von Agrobakterien in Weizenpflanzen der Varietät Fielder transformiert und anschließend regeneriert.
Der Anstieg der SS2 Aktivität der transgenen Weizen-Pflanzen im Vergleich zu den Wildtyp-Weizen-Pflanzen wird in Zymogrammen nachgewiesen.

Beispiel 3

Analyse der Stärken und Mehle von genetisch modifizierten Weizenpflanzen, die eine erhöhte **SSII-Aktivität** aufweisen

Die Analyse von T1-Körnern erfolgte auf Basis von Pools aus einer geringen Zahl von Einzelkörnern. Das Kornmaterial wurde wie unter Material und Methoden beschrieben zu Weizenmehlen verarbeitet und nachfolgend für die Analyse des Amlyosegehaltes, der DSC-Eigenschaften, der Verdaubarkeit sowie der Amylopektin-Seitenkettenverteilung verwendet.
Mehle der Weizenkörner, welche die T-DNA für die Expression der mutagenisierten Form der Stärkesynthase II-3 aus Reis (=synthSS2 Os mut; siehe SEQ ID No. 3) enthalten, weisen einen nahezu unveränderten oder leicht reduzierten Amlyosegehalt im Vergleich zu Mehlen genetisch unveränderter Weizenkörner von Wildtyp-Pflanzen auf. Die DSC-Temperaturen der Mehle der transgenen Weizenkörner sind bis zu 5°C höher als die der entsprechenden Kontrollen. Zudem sind die Mehle der transgenen Weizenkörner schlechter für einen Abbau durch Stärke-hydrolysierende Enzyme zugänglich, was an einem reduzierten Gehalt an schnell verdaubarer Stärke (RDS) sowie einem erhöhten Gehalt an resistenter Stärke (RS) zu erkennen ist. Die Struktur der Stärke der transgenen Weizenkörner ist insofern verändert, als dass sie über weniger kurze Seitenketten (DP6-10) und mehr mittlere Seitenketten (DP11-24) verfügt. Deutlich erkennbar wird dies auch in dem als ACR bezeichneten Verhältnis von kurzen Seitenketten zu kurzen und mittleren Seitenketten, welches für die Stärken der transgenen Weizen-Pflanzen deutlich geringer ist.
a. Amylosegehalt des Mehles und der Stärke (Amylosegehalt der Stärke wurde rechnerisch ermittelt, ausgehend von einem gemäß Methode 8 experimentell ermittelten Gewichtsanteil der Stärke von 60%):

| Probe | Amylosegehalt Mehl in Gew.-% | Amylosegehalt Stärke in Gew.-% |
|---|---|---|
| **Wildtyp-Pflanze Varietät Fielder** | 17.7 | 29.5 |
| **GKTA0001-2201** | 17.1 | 28.5 |
| **GKTA0001-1304** | 13.8 | 23.0 |

b. DSC-Daten der Mehle (DSC-Analyse bei 3-fachem Wasserüberschuss)

| Probe | Tonset in °C | Tpeak in °C |
|---|---|---|
| **Wildtyp-Pflanze Varietät Fielder** | 62.0 | 68.2 |
| **GKTA0001-2201** | 65.5 | 71.9 |
| **GKTA0001-1304** | 66.9 | 73.0 |

c. RS-und RDS-Gehalt des Mehles (= RS Mehl und RDS Mehl)

| Probe | RS Mehl (in Gew.-%) | RDS Mehl (in Gew.-%) |
|---|---|---|
| **Wildtyp-Pflanze Varietät Fielder** | 2.4 | 24.0 |
| **GKTA0001-2201** | 15.0 | 18.6 |
| **GKTA0001-1304** | 13.0 | 18.6 |

d. RS-und RDS-Gehalt der Stärke (= RS Stärke bzw. RDS Stärke) (bestimmt an einer Mehlprobe, die einen gemäß Methode 8 experimentell ermittelten Gewichtsanteil der Stärke von 60 Gew.- % an der Mehlprobe aufwies. Der RS Stärke- bzw. der RDS Stärke-Wert ergibt sich dann rechnerisch aus dem RS Mehl- bzw. dem RDS Mehl-Wert, in dem man den RS Mehl bzw. RDS-Mehl-Wert jeweils mit 100 multipliziert und anschließend jeweils durch 60 dividiert):

| Probe | RS Stärke (in Gew.-%) | RDS Stärke (in Gew.-%) |
|---|---|---|
| **Wildtyp-Pflanze Varietät Fielder** | 4.0 | 40.0 |
| **GKTA0001-2201** | 25.0 | 31.0 |
| **GKTA0001-1304** | 22.0 | 31.0 |

e. Seitenkettenverteilung des Amylopektins

| Probe | DP6-11 (Summe der Flächen im Chromatogramm) | Verhältnis zum Wildtyp in % | Unterschied zum Wildtyp (in %) |
|---|---|---|---|
| **Wildtyp-Pflanze Varietät Fielder** | 21.95 | 100% | 0% |
| **GKTA0001-2201** | 19.03 | 86.7% | -13.3 % |
| **GKTA0001-1304** | 18.59 | 84.7% | -15.3% |
| **Probe** | **DP17-20 (Summe der Flächen im Chromatogramm)** | **Verhältnis zum Wildtyp in %** | **Unterschied zum Wildtyp (in %)** |
| **Wildtyp-Pflanze Varietät Fielder** | 15.29 | 100% | 0% |
| **GKTA0001-2201** | 16.60 | 108.6% | 8.6% |
| **GKTA0001-1304** | 17.03 | 111.4% | 11.4% |

f. ACR-Werte

| Probe | ACR-Wert |
|---|---|
| Wildtyp-Pflanze Varietät Fielder | 0.177 |
| GKTA0001-2201 | 0.148 |

(fortgesetzt)

| Probe | ACR-Wert |
|---|---|
| GKTA0001-1304 | 0.143 |

**Beispiel 4**

**Herstellung des Vektors pBA74 zur Expression einer synthetischen Form der Stärkesynthase II aus Weizen (=synthSS2 Ta) in Weizen**

Funktionelle Elemente des Transformationsvektors
Der Vektor pBA71 ist ein Derivat des Plasmids pGREEN (Hellens *et al.,* 2000). Der "backbone" des Plasmids enthält folgende funktionelle Elemente:

[1] Den "origin of replication" des Plasmids pBR322 (Bolivar *et al.,* 1977) zur Replikation in *Escherichia coli* (ORI ColE1)
[2] Einen Selektionsmarker vermittelnd Resistenz gegen Kanamyzin (*nptl*; Grindley and Joyce, 1980) zur Vermehrung in *Escherichia coli* and *Agrobacterium tumefaciens*.
[3] Den "origin of replication" des Plasmids pSA (Tait *et al.,* 1982) zur Replikation in *Agrobacterium* (ORI pSA)

Die in das Pflanzengenom übertragenen genetischen Elemente sind in der Vektor Karte dargestellt (siehe Figur 2) und detailliert beschrieben in Tabelle 2.

Tabelle2:

| Beschreibung der genetischen Elemente welche in das Pflanzengenom inseriert werden | | |
|---|---|---|
| **Nt Positions** | **Orientation** | **Origin** |
| 540-563 | | **LB:** "left border repeat" der T-DNA of *Agrobacterium tumefaciens* (Zambryski, 1988) |
| 615-2623 | Clockwise | **PUbiZm + intron:** Promotor und Intron des Ubiquitin-1 Gens (*ubi1*) aus *Zea mays* (Christensen *et al.,* 1992). |
| 2624-3204 | Clockwise | ***bar.*** Kodierende Region des Phosphinothricin Acetyltransferase Gens aus *Streptomyces hygroscopicus* Thompson *et al*. (1987) |
| 3205-3461 | Clockwise | **3'nos:** Sequenz umfassend die 3' nicht translatierte Region des Nopalin Synthase Gens der T-DNA von pTiT37 (Depicker *et al.,* 1982) |
| 3562-3587 | Clockwise | **attB1:** Sequenz umfassend modifizierte Erkennungssequenz von attB aus E.coli (Hartley et al., 2000) |
| 3543-3743 | Counter clockwise | **3'ocs:** Sequenz beinhaltend die 3' untranslatierte Region des Octopin Synthase Gens von *Agrobacterium tumefaciens* wie beschrieben bei De Greve *et al.* (1982) |
| 3828-6227 | Counter clockwise | ***synthSS2*** Ta.: Kodierende Region der Stärkesynthase II aus Weizen als synthetische Version.** Aminosäure-Sequenz identisch zur Stärkesynthase aus Weizen (SEQ ID No. 6 oder GenBank Acc. Nummer CAB69544.1) |
| 6268-6729 | Counter clockwise | **intron1 *act1* Os:** Erstes Intron des Actin Gens aus *Oryza sativa* (Reis) (Mc Elroy *et al.,* 1990). |
| 6769-8408 | Counter clockwise | **Pact1Os:** Sequenz umfassens die Promotor Region des Actin 1 Gens aus *Oryza sativa* (Reis) (Mc Elroy *et al.*, 1990). |
| 8549-8573 | Clockwise | **attB2:** Sequenz umfassend modifizierte Erkennungssequenz von attB aus E.coli (Hartley et al., 2000) |

(fortgesetzt)

| Beschreibung der genetischen Elemente welche in das Pflanzengenom inseriert werden | | |
|---|---|---|
| **Nt Positions** | **Orientation** | **Origin** |
| 8581-8605 | | **RB:** "right border repeat" der T-DNA aus *Agrobacterium tumefaciens* (Zambryski, 1988) |
| ** Ausgehend von der kodierenden Region der SS2 aus Weizen (siehe SEQ ID No. 18) wurde zur Vermeidung von Cosuppressionseffekten die DNA-Sequenz in einer Weise synthetisiert, dass keine identischen Nukleotidabschnitte mit einer Länge von mehr als 11 Basenpaaren zwischen der natürlichen SS2 aus Weizen (SEQ ID No. 18) und der synthetischen Sequenz (synthSS2 Ta, siehe SEQ ID No. 5) vorhanden sind. | | |

Referenzen:

| |
|---|
| Bolivar F., Rodriguez R.L.., Greene P.J., Betlach M.C., Heyneker H.L., Boyer H.W. (1977). Construction and characterization of new cloning vehicles. II. A multipurpose cloning system, Gene, 2, 95-113. |
| Christensen A. H., Sharrock R.A., Quail P.H. (1992). Maize polyubiquitin genes: structure, thermal pertubation of expression and transcript splicing, and promoter activity following transfer to protoplasts by electroporation. Plant Molecular Biology, 18, 675-689. |
| De Greve H., Dhaese P., Seurinck J., Lemmers M., Van Montagu M., Schell J. (1982). Nucleotide sequence and transcript map of the Agrobacterium tumefaciens Ti plasmid-encoded octopine synthase gene. Journal of Molecular and Applied Genetics, 1, 499-511. |
| Depicker A., Stachel S., Dhaese P., Zambryski P., Goodman H.M. (1982). Nopaline synthase: transcript mapping and DNA sequence. Journal of Molecular and Applied Genetics, 1, 561-573. |
| Grindley N.D.F., Joyce C.M. (1980) Genetic and DNA sequence analysis of the kanamycin resistance transposon Tn903. Proc. Natl. Acad. Sci. U.S.A. 77(12):7176-7180 |
| Hartley J.L., Temple G.F., Brasch M.A. (2000) DNA cloning using in vitro site-specific recombination. Genome Research 10: 1788-1795 |
| Hellens RP, Edwards EA, Leyland NR, Bean S, Mullineaux PM. (2000). pGreen: a versatile and flexible binary Ti vector for Agrobacterium-mediated plant transformation. Plant Mol Biol 42(6): 819-832. |
| Mc Elroy D., Zhang W., Cao J., Wu R. (1990). Isolation of an efficient actin promoter for use in rice transformation. The Plant Cell, 2, 163-171. |
| Tait RC, Close TJ, Rodriguez RL, Kado CI. (1982) Isolation of the origin of replication of the IncW-group plasmid pSa. Gene. 20(1): 39-49. |
| Thompson C.J., Rao Movva N., Tizard R., Crameri R., Davies J., Lauwereys M., Botterman J. (1987). Characterization of the herbicide resistance gene bar from Streptomyces hygroscopicus. The EMBO Journal, 6, 2519-2523. |
| Zambryski P. (1988). Basic processes underlying Agrobacterium-mediated DNA transfer to plant cells. Annual Review of Genetics, 22, 1-30. |

**Patentansprüche**

1. Weizenmehl, dessen Stärkekomponente einen Amylosegehalt zwischen 15.0 Gew.-% bis 30.0 Gew.-% und dessen Stärkekomponente einen Gehalt an resistenter Stärke (RS Stärke) von mehr als 5.0 Gew.-% aufweist.

2. Weizenmehl nach Anspruch 1, dessen Stärkekomponente einen Amylose-gehalt zwischen 18.0 Gew.-% bis 30.0 Gew.-% aufweist.

3. Weizenmehl nach Anspruch 1, dessen Stärkekomponente einen Amylosegehalt zwischen 20.0 Gew.-% bis 30.0 Gew.-% aufweist.

4. Weizenmehl nach einem der Ansprüche 1-3, das einen Gehalt an resistenter Stärke (RS) zwischen 5.0 Gew.-% bis 30.0 Gew.-% aufweist.

5. Weizenmehl nach einem der Ansprüche 1-4, das aus einer Weizen-Pflanze gewonnen wurde, die eine heterologe Stärkesynthase II exprimiert.

6. Weizenmehl nach einem der Ansprüche 1 bis 5, dessen Stärkekomponente granulär ist.

7. Weizenmehl nach Anspruch 5 oder 6, wobei die Aminosäuresequenz der heterologen Stärkesynthase II **dadurch gekennzeichnet ist, dass** sie eine Identität von mindestens 86% mit den Aminosäuren 333 bis 362 der unter SEQ ID NO 4 dargestellten Aminosäuresequenz und eine Identität von mindestens 83% mit den Aminosäuren 434 bis 473 der unter SEQ ID NO 4 dargestellten Aminosäuresequenz und eine Identität von mindestens 70% mit den Aminosäuren 652 bis 716 der unter SEQ ID NO 4 dargestellten Aminosäuresequenz aufweist.

8. Zusammensetzung enthaltend Weizenmehl nach einem der Ansprüche 1 bis 7 und mindestens einen Nahrungsmittelzusatzstoff.

9. Lebensmittel enthaltend Weizenmehl nach einem der Ansprüche 1 bis 7.

10. Lebensmittel enthaltend eine Zusammensetzung nach Anspruch 8.

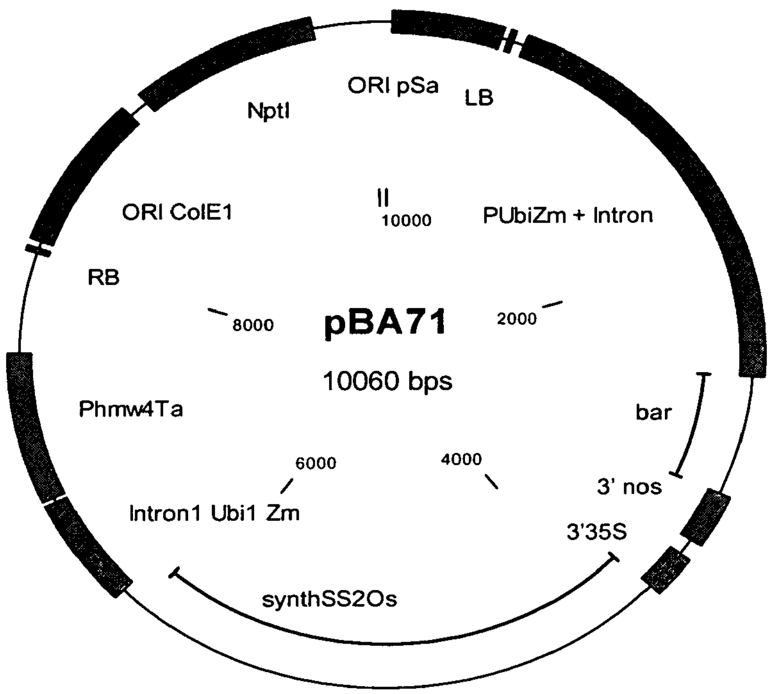
ORI pSa
LB
Nptl
ORI ColE1
10000
PUbiZm + Intron
RB
8000
pBA71
2000
10060 bps
Phmw4Ta
bar
6000
4000
3' nos
Intron1 Ubi1 Zm
3'35S
synthSS2Os

**Figur 1**

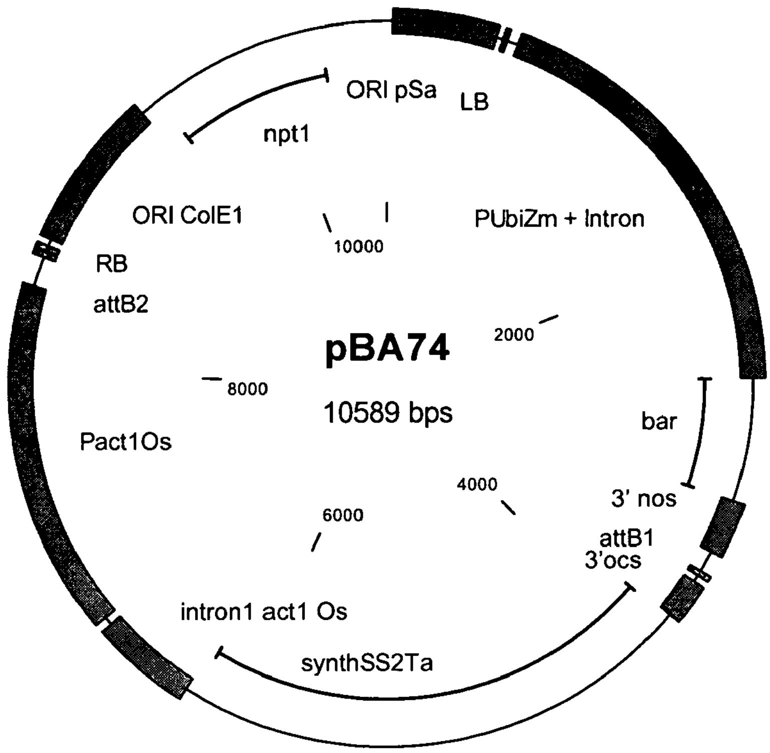
ORI pSa
LB
npt1
ORI ColE1
PUbiZm + Intron
10000
RB
attB2
pBA74
10589 bps
2000
8000
bar
Pact1Os
4000
3' nos
6000
attB1
3'ocs
intron1 act1 Os
synthSS2Ta

**Figur 2**

Europäisches Patentamt
European Patent Office
Office européen des brevets

# EUROPÄISCHER RECHERCHENBERICHT

Nummer der Anmeldung
EP 08 07 5631

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | SHIN MALSHICK ET AL: "Hot-water solubilities and water sorptions of resistant starches at 25degreeC" CEREAL CHEMISTRY, AMERICAN ASSOCIATION OF CEREAL CHEMISTS. MINNEAPOLIS, US, Bd. 80, Nr. 5, 1. September 2003 (2003-09-01), Seiten 564-566, XP008098848 ISSN: 0009-0352 * Tabelle I * ----- | 1-10 | INV. C12N15/82 A01H5/00 |
| X | SHIN MALSHICK ET AL: "In vitro digestibility of cross-linked starches - RS4" STARCH, Bd. 56, Nr. 10, Oktober 2004 (2004-10), Seiten 478-483, XP002504472 ISSN: 0038-9056 * Tabelle 2 * ----- | 1-10 | |
| X | WOO K S ET AL: "CROSS-LINKED RESISTANT STARCH: PREPARATION AND PROPERTIES" CEREAL CHEMISTRY, AMERICAN ASSOCIATION OF CEREAL CHEMISTS. MINNEAPOLIS, US, Bd. 79, Nr. 6, 1. November 2002 (2002-11-01), Seiten 819-825, XP001132662 ISSN: 0009-0352 * Tabellen I,III * ----- | 1-10 | RECHERCHIERTE SACHGEBIETE (IPC) C12N A01H A21D |
| | -/-- | | |

Der vorliegende Recherchenbericht wurde für alle Patentansprüche erstellt

| Recherchenort | Abschlußdatum der Recherche | Prüfer |
|---|---|---|
| Den Haag | 26. November 2008 | Bucka, Alexander |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 08 07 5631

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | VAN HUNG PHAM ET AL: "Formation of enzyme-resistant starch in bread as affected by high-amylose wheat flour substitutions" CEREAL CHEMISTRY, Bd. 82, Nr. 6, November 2005 (2005-11), Seiten 690-694, XP008098914 ISSN: 0009-0352 * Abbildung 2 * ----- | 1-10 | |
| X | MIKULIKOVA D ET AL.: "The Potential of Common Cereals to form Retrograded Resistant Starch" CZECH J. GENET. PLANT BREED., Bd. 42, 2006, Seiten 95-102, XP002504475 * Tabelle 3 * ----- | 1,4-10 | |
| X | YAMAMORI M ET AL: "Resistant starch and starch pasting properties of a starch synthase IIa-deficient wheat with apparent high amylose" AUSTRALIAN JOURNAL OF AGRICULTURAL RESEARCH, Bd. 57, Nr. 5, 2006, Seiten 531-535, XP008098696 ISSN: 0004-9409 * Tabelle 1 * ----- | 1,4-10 | RECHERCHIERTE SACHGEBIETE (IPC) |
| X | WO 2007/009823 A1 (BAYER CROPSCIENCE GMBH [DE]; FROHBERG CLAUS [DE]; SCHMIDT RALF CHRISTI) 25. Januar 2007 (2007-01-25) * Beispiele 1-4,9; Tabellen 3,8 * ----- | 1-10 | |
| A | WO 2006/069422 A1 (COMMW SCIENT IND RES ORG [AU]; BIOGEMMA S A S [FR]; BIRD ANTHONY RICHA) 6. Juli 2006 (2006-07-06) * das ganze Dokument * ----- | 1-10 | |
| | -/-- | | |

Der vorliegende Recherchenbericht wurde für alle Patentansprüche erstellt

| Recherchenort | Abschlußdatum der Recherche | Prüfer |
|---|---|---|
| Den Haag | 26. November 2008 | Bucka, Alexander |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

## EUROPÄISCHER RECHERCHENBERICHT

Nummer der Anmeldung
EP 08 07 5631

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| A | SKRABANJA VIDA ET AL: "Nutritional properties of starch in buckwheat products: Studies in vitro and in vivo" JOURNAL OF AGRICULTURAL AND FOOD CHEMISTRY, Bd. 49, Nr. 1, Januar 2001 (2001-01), Seiten 490-496, XP002504474 ISSN: 0021-8561 * Tabelle 2 * ----- | 1-10 | |
| A | CHANVRIER HELENE ET AL: "Processing of novel elevated amylose wheats: Functional properties and starch digestibility of extruded products" JOURNAL OF AGRICULTURAL AND FOOD CHEMISTRY, Bd. 55, Nr. 25, Dezember 2007 (2007-12), Seiten 10248-10257, XP002504473 ISSN: 0021-8561 * Tabelle 4 * ----- | 1-10 | |
| | | | RECHERCHIERTE SACHGEBIETE (IPC) |
| A | WASSERMAN L A ET AL.: "PREPARATION OF WHEAT RESISTANT STARCH. Treatment of gels and DSC characterization" JOURNAL OF THERMAL ANALYSIS AND CALORIMETRY, Bd. 87, 2007, Seiten 153-157, XP019482422 * das ganze Dokument * ----- | 1-10 | |
| A | SESTILI F ET AL.: "PRODUCTION OF DURUM AND BREAD WHEAT LINES WITH HIGH AMYLOSE STARCH" PROCEEDINGS OF THE 51ST ITALIAN SOCIETY OF AGRICULTURAL GENETICS ANNUAL CONGRESS, RIVA DEL GARDA, ITALY, 23/26 SEPTEMBER 2007, 2007, XP002504478 ISBN: 978-88-900622-7-8 * das ganze Dokument * ----- | 1-10 | |
| | -/-- | | |

Der vorliegende Recherchenbericht wurde für alle Patentansprüche erstellt

| Recherchenort | Abschlußdatum der Recherche | Prüfer |
|---|---|---|
| Den Haag | 26. November 2008 | Bucka, Alexander |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 08 07 5631

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | SAJILATA M G ET AL.: "Resistant Starch - A Review"<br>COMPREHENSIVE REVIEWS IN FOOD SCIENCE AND FOOD SAFETY,<br>Bd. 5, 2006, Seiten 1-17, XP002504477<br>* das ganze Dokument *<br>----- | 1-10 | |
| | | | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| Den Haag | 26. November 2008 | Bucka, Alexander |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

5

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 08 07 5631

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-11-2008

| Im Recherchenbericht angeführtes Patentdokument | Datum der Veröffentlichung | Mitglied(er) der Patentfamilie | Datum der Veröffentlichung |
|---|---|---|---|
| WO 2007009823 A1 | 25-01-2007 | AU 2006271887 A1 | 25-01-2007 |
| | | KR 20080028950 A | 02-04-2008 |
| WO 2006069422 A1 | 06-07-2006 | CA 2594155 A1 | 06-07-2006 |
| | | EP 1833291 A1 | 19-09-2007 |
| | | JP 2008526690 T | 24-07-2008 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 564893 A1
- EP 688872 A1
- EP 846704 A1
- US 5051271 A
- US 6750378 B
- WO 0173087 A
- DE 10041861
- DE 10032379


### In der Beschreibung aufgeführte Nicht-Patentliteratur


- **Faisant et al.** *Sciences des Aliments,* 1995, vol. 15, 83-89
- **Evans ; Thompson.** *Cereal Chemistry,* 2004, vol. 81 (1), 31-37
- **von Englyst et al.** *Europ. J. of Clinical Nutrition,* 1992, vol. 46 (2), 33-50
- **McCleary ; Monaghan.** *J. AOAC Int.,* 2002, vol. 85, 665-675
- **Senti ; Russell.** *Tappi,* April 1960, vol. 43 (4), 343-349
- **Z. Luo et al.** *Starch/Stärke,* 2006, vol. 58, 468-474
- **Regina et al.** *PNAS,* 2006, vol. 103 (10), 3546-3551
- **Yamamori et al.** *Australian Journal of Agricultural Research,* 2006, vol. 57, 531-535
- **Konik-Rose et al.** *Theor. Appl. Genet.,* 2007, vol. 115, 1053-1065
- **Morita et al.** *Cereal Chemistry,* 2002, vol. 79, 491-495
- **Hung et al.** *Cereal Chemsitry,* 2005, vol. 82, 690-694
- **Hung et al.** *Trends in Food Science & Technology,* 2006, vol. 17, 448-456
- **Morell et al.** *Journal of AOAC International,* 2004, vol. 87 (3), 740-748
- **Englyst et al.** *British Journal of Nutrition,* vol. 75, 327-337
- **W. Banks ; C.T. Greenwood.** Starch and its components. Edinburgh University Press, 51-66
- **Larson et al.** *Analytical Chemistry,* 1953, vol. 25 (5), 802-804
- **Morrison ; Laignelet.** *J. Cereal Sc.,* 1983, vol. 1, 9-20
- **Kugimiya ; Donovan.** *Journal of Food Science,* 1981, vol. 46, 765-770
- **Sievert ; Holm.** *Starch/Stärke,* 1993, vol. 45 (4), 136-139
- **Englyst et al.** *Reagents, Apparatus, Spectrophotometer,* S35-S36S36-S37
- **Yahl et al.** *Microscope,* 1984, vol. 32, 123-132
- **von Wolever et al.** *Am. J. Clin. Nutr.,* 1991, vol. 54, 846-854
- Carbohydrates in human nutrition. The Role of the Glycemic Index in Food Choice. 14. April 1997, 25-30
- Starch: Chemistry and Technology. Academic Press Inc. London Ltd, 1994, 491-506
- **Eckhoff et al.** *Cereal Chem.,* 1996, vol. 73, 54-57
- **von Gao ; Chibbar.** *Genome,* 2000, vol. 43 (5), 768-775
- **Jiang H. ; Dian W. ; Liu F. ; Wu P.** Molecular cloning and expression analysis of three genes encoding starch synthase II in rice. *Planta,* 2004, vol. 218, 1062-1070
- GenBank. AF419099.1
- **Schlegel.** Allgemeine Mikrobiologie. Georg Thieme Verlag, 1985, 1-2
- **Thompson et al.** *Nucleic Acids Research,* 1994, vol. 22, 4673-4680
- **Nishi et al.** *Plant Physiology,* 2001, vol. 127, 459-472
- **Odell et al.** *Nature,* 1985, vol. 313, 810-812
- **Christensen et al.** *Plant Mol. Biol.,* 1992, vol. 18, 675-689
- **Liu et al.** *Plant Science,* 2003, 165
- **Zhang et al.** *Plant Cell,* 1991, vol. 3, 1150-1160
- **Verdaguer.** *Plant Mol. Biol.,* vol. 31, 1129-1139
- **Stavolone et al.** *Plant Mol. Biol.,* 2003, vol. 53, 703-713
- **Kirihara et al.** *Gene,* 1988, vol. 71, 359-370
- **Hoffmann et al.** *EMBO J.,* 1987, vol. 6, 3213-3221
- **Schernthaner et al.** *EMBO J.,* 1988, vol. 7, 1249-1253
- **Williamson et al.** *Plant Physiol.,* 1988, vol. 88, 1002-1007
- **Prat et al.** *Gene,* 1987, vol. 52, 51-49

- **Gallardo et al.** *Plant Sci.,* 1988, vol. 54, 211-281
- **Marks et al.** *J. Biol. Chem.,* 1985, vol. 260, 16451-16459
- **Kodrzyck et al.** *Plant Cell,* 1989, vol. 1, 105-114
- **Yang, N.-S. ; Russel, D.** *Proc. Natl. Acad Sci,* 1990, vol. 87, 4144-4148
- **Unger et al.** *Plant Physiol.,* 1991, vol. 96, 124
- **Bhave et al.** *Plant Cell,* 1990, vol. 2, 581-588
- **Bae et al.** *Maydica,* 1990, vol. 35, 317-322
- **Colot et al.** *EMBO J.,* 1987, vol. 6, 3559-3564
- **Clarke ; Appels.** *Genome,* 1998, vol. 41, 865-871
- **Pedersen et al.** *Cell,* 1982, vol. 29, 1015-1026
- **Quatroccio et al.** *Plant Mol. Biol.,* 1990, vol. 15, 81-93
- **Leisy et al.** *Plant Mol. Biol.,* 1990, vol. 14, 41-50
- **Zheng et al.** *Plant J.,* 1993, vol. 4, 357-366
- **Yoshihara et al.** *FEBS Lett.,* 1996, vol. 383, 213-218
- **Nakase et al.** *Gene,* 1996, vol. 170 (2), 223-226
- **Qu ; Takaiwa.** *Plant Biotechnology Journal,* 2004, vol. 2 (2), 113-125
- **Anderson.** *Theoretical and Applied Genetics,* 1998, vol. 96, 568-576
- **Thomas.** *Plant Cell,* 1990, vol. 2 (12), 1171-1180
- **Sengupta-Gopalan.** *Proc. Natl. Acad. Sci. USA,* 1985, vol. 82, 3320-3324
- **Bustos.** *Plant Cell,* 1989, vol. 1 (9), 839-853
- **Callis et al.** *Genes Devel.,* 1987, vol. 1, 1183-1200
- **Luehrsen ; Walbot.** *Mol. Gen. Genet.,* 1991, vol. 225, 81-93
- **Rethmeier et al.** *Plant Journal.,* 1997, vol. 12 (4), 895-899
- **Rose ; Beliakoff.** *Plant Physiol.,* 2000, vol. 122 (2), 535-542
- **Vasil et al.** *Plant Physiol.,* 1989, vol. 91, 1575-1579
- **XU et al.** *Science in China Series C,* 2003, vol. 46 (6), 561-569
- **Maas et al.** *Plant. Mol. Biol.,* 1991, vol. 16, 199-207
- **Callis et al.** *Genes Dev.,* 1987, vol. 1, 1183-1200
- **Luehrsen, K. R. ; Walbot, V.** *Mol. Gen. Genet.,* 1991, vol. 225, 81-93
- **Oard et al.** *Plant Cell Rep,* 1989, vol. 8, 156-160
- **Sambrook et al.** Molecular Cloning, A Laboratory Manual. Cold Spring Harbour Laboratory Press, 2001
- **Ausubel et al.** Short Protocols in Molecular Biology. John Wiley & Sons, 2002
- Plant Cell Culture Protocols. Humana Press, 1999
- **Englyst et al.** *Reagents, Apparatus, Spectrophotometer,* S35-S36
- **Hung et al.** *Cereal Chemistry,* 2005, vol. 82, 690-694
- **Yamamori et al.** *Australian Journal of Agricultural Research,* 2006, vol. 57, 531-53
- **Wu H ; Sparks C ; Amoah B ; Jones HD.** Factors influencing successful Agrobacterium-mediated genetic transformation of wheat. *Plant Cell Reports,* 2003, vol. 21, 659-6686
- **Murashige ; Skoog.** *Physiol. Plant.,* 1962, vol. 15, 473-497
- **Englyst et al.** *Europ. J. of Clinical Nutrition,* 1992, vol. 46 (2), 33-50
- **von Polaske.** *Starch,* 2005, vol. 57, 118-123
- **Nielsen et al.** *Plant Physiol.,* 1994, vol. 105, 111-117
- **Bradford.** *Anal Biochem,* 1976, vol. 72, 248-254
- **Joshi et al.** Contex sequences of translation initiation codon in plants. *PMB,* 1997, vol. 35, 993-1001
- **Bolivar F. ; Rodriguez R.L. ; Greene P.J. ; Betlach M.C. ; Heyneker H.L. ; Boyer H.W.** Construction and characterization of new cloning vehicles. II. A multipurpose cloning system. *Gene,* 1977, vol. 2, 95-113
- **Christensen A. H. ; Sharrock R.A. ; Quail P.H.** Maize polyubiquitin genes: structure, thermal pertubation of expression and transcript splicing, and promoter activity following transfer to protoplasts by electroporation. *Plant Molecular Biology,* 1992, vol. 18, 675-689
- **Depicker A. ; Stachel S. ; Dhaese P. ; Zambryski P. ; Goodman H.M.** Nopaline synthase: transcript mapping and DNA sequence. *Journal of Molecular and Applied Genetics,* 1982, vol. 1, 561-573
- **Grindley N.D.F. ; Joyce C.M.** Genetic and DNA sequence analysis of the kanamycin resistance transposon Tn903. *Proc. Natl. Acad. Sci. U.S.A.,* 1980, vol. 77 (12), 7176-7180
- **Hellens RP ; Edwards EA ; Leyland NR ; Bean S ; Mullineaux PM.** pGreen: a versatile and flexible binary Ti vector for Agrobacterium-mediated plant transformation. *Plant Mol Biol,* 2000, vol. 42 (6), 819-832
- **Sanfaçon H ; Brodmann P ; Hohn T.** A dissection of the cauliflower mosaic virus polyadenylation signal. *Genes Dev.,* 1991, vol. 5 (1), 141-149
- **Tait RC ; Close TJ ; Rodriguez RL ; Kado CI.** Isolation of the origin of replication of the IncW-group plasmid pSa. *Gene,* 1982, vol. 20 (1), 39-49

- **Thompson C.J. ; Rao Movva N. ; Tizard R. ; Crameri R. ; Davies J. ; Lauwereys M. ; Botterman J.** Characterization of the herbicide resistance gene bar from Streptomyces hygroscopicus. *The EMBO Journal,* 1987, vol. 6, 2519-2523
- **Zambryski P.** Basic processes underlying Agrobacterium-mediated DNA transfer to plant cells. *Annual Review of Genetics,* 1988, vol. 22, 1-30
- **De Greve H. ; Dhaese P. ; Seurinck J. ; Lemmers M. ; Van Montagu M. ; Schell J.** Nucleotide sequence and transcript map of the Agrobacterium tumefaciens Ti plasmid-encoded octopine synthase gene. *Journal of Molecular and Applied Genetics,* 1982, vol. 1, 499-511
- **Hartley J.L. ; Temple G.F. ; Brasch M.A.** DNA cloning using in vitro site-specific recombination. *Genome Research,* 2000, vol. 10, 1788-1795
- **Mc Elroy D. ; Zhang W. ; Cao J. ; Wu R.** Isolation of an efficient actin promoter for use in rice transformation. *The Plant Cell,* 1990, vol. 2, 163-171
- **Tait RC ; Close TJ ; Rodriguez RL ; Kado CI.** Isolation of the origin of replication of the IncW-group plasmid pSa. *Gene,* 1982, vol. 20 (1), 39-49